# EUROPEAN PATENT SPECIFICATION

(11) **EP 3 265 568 B1**
(45) Date of publication and mention of the grant of the patent: **06.05.2020**
(21) Application number: 16762307.3
(22) Date of filing: 07.03.2016
(51) Int. Cl.: C12N 15/90, A61K 9/00, A61K 48/00, C12N 15/113

(54) **GENE AUGMENTATION THERAPIES FOR INHERITED RETINAL DEGENERATION CAUSED BY MUTATIONS IN THE PRPF31 GENE**
GENAUGMENTATIONSTHERAPIEN FÜR HEREDITÄRE NETZHAUTDEGENERATION DURCH MUTATIONEN IM PRPF31-GEN
THÉRAPIES D'AUGMENTATION GÉNÉTIQUE DE LA DÉGÉNÉRESCENCE RÉTINIENNE HÉRÉDITAIRE CAUSÉE PAR DES MUTATIONS AU NIVEAU DU GÈNE PRPF31

(30) Priority: 06.03.2015 US 201562129638 P; 14.04.2015 US 201562147307 P
(43) Date of publication of application: 10.01.2018
(73) Proprietor: Massachusetts Eye & Ear Infirmary, Boston, MA 02114 (US)
(72) Inventor: PIERCE, Eric A., Brookline, Massachusetts 02478 (US); FARKAS, Michael H., Getzville, NY 14053-1492 (US); SOUSA, Maria E., Dedham, Massachusetts 02026 (US)
(74) Representative: Donald, Jenny Susan
(86) International application number: PCT/US2016/021226
(87) International publication number: WO 2016/144892

(56) References cited:
- WO-A1-2008/137066
- WO-A1-2014/011210
- WO-A1-2015/189429
- WO-A2-02/082904
- WO-A2-2004/084951
- WO-A2-2009/134681
- WO-A2-2010/005533
- US-A1- 2010 190 841
- Farkas ET AL: "Mutations in Pre-mRNA Processing Factors 3, 8, and 31 Cause Dysfunction of the Retinal Pigment Epithelium", American Journal of Pathology, The, 1 January 2014 (2014-01-01), pages 2641-2652, XP55487560, United States DOI: 10.1016/j.ajpath.2014.06.026 Retrieved from the Internet: URL:https://www.sciencedirect.com/science/ article/pii/S0002944014003836/pdfft?md5=f3 559481d47d35022e9f0a51794cda10&pid=1-s2.0- S0002944014003836-main.pdf [retrieved on 2018-06-25]
- JOHN J. GRAZIOTTO ET AL: "Three Gene-Targeted Mouse Models of RNA Splicing Factor RP Show Late-Onset RPE and Retinal Degeneration", INVESTIGATIVE OPTHALMOLOGY & VISUAL SCIENCE, vol. 52, no. 1, 5 January 2011 (2011-01-05), page 190, XP055487574, US ISSN: 1552-5783, DOI: 10.1167/iovs.10-5194
- A. M. ROSE ET AL: "Expression of PRPF31 and TFPT: regulation in health and retinal disease", HUMAN MOLECULAR GENETICS, vol. 21, no. 18, 20 June 2012 (2012-06-20) , pages 4126-4137, XP055111690, ISSN: 0964-6906, DOI: 10.1093/hmg/dds242
- GIULIA VENTURINI ET AL: "CNOT3 Is a Modifier of PRPF31 Mutations in Retinitis Pigmentosa with Incomplete Penetrance", PLOS GENETICS, vol. 8, no. 11, 10 November 2012 (2012-11-10), page e1003040, XP055487567, US ISSN: 1553-7390, DOI: 10.1371/journal.pgen.1003040
- WANG, L ET AL.: 'Novel deletion in the pre-mRNA splicing gene PRPF31 causes autosomal dominant retinitis pigmentosa in a large Chinese family.' AM J MED GENET A. vol. 121A, no. 3, 01 September 2003, pages 235 - 239, XP055310112

### SEQUENCE LISTING

<110> Massachusetts Eye and Ear Infirmary
<120> GENE AUGMENTATION THERAPIES FOR
   INHERITED RETINAL DEGENERATION CAUSED BY MUTATIONS IN THE
PRPF31 GENE
<130> 00633-0192WO1
<150> US 62/147,307
   <151> 2015-04-14
<150> US 62/129,638
   <151> 2015-03-06
<160> 34
<170> PatentIn version 3.5
<210> 1
   <211> 736
   <212> PRT
   <213> Adeno-associated virus
<220>
   <221> VARIANT
   <222> (168)..(168)
   <223> /replace="Arg"
<220>
   <221> VARIANT
   <222> (204)..(204)
   <223> /replace="Ser"
<220>
   <221> VARIANT
   <222> (266)..(266)
   <223> /replace="Gly"
<220>
   <221> VARIANT
   <222> (311)..(311)
   <223> /replace="Lys"
<220>
   <221> VARIANT
   <222> (411)..(411)
   <223> /replace="Gln"
<220>
   <221> VARIANT
   <222> (460)..(460)
   <223> /replace="Glu"
<220>
   <221> VARIANT
   <222> (493)..(493)
   <223> /replace="Thr"
<220>
   <221> VARIANT
   <222> (562)..(562)
   <223> /replace="Asn"
<220>
   <221> VARIANT
   <222> (576)..(576)
   <223> /replace="Glu"
<220>
   <221> VARIANT
   <222> (587)..(587)
   <223> /replace="Ala"
<220>
   <221> VARIANT
   <222> (609)..(609)
   <223> /replace="Asp"
<220>
   <221> misc_feature
   <222> (1)..(736)
   <223> /note="Variant residues given in the sequence have no preference with respect to those in the annotations for variant positions"
<400> 1
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Lys Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ala Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Ala Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Arg Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
               340                 345                 350
   Tyr Val Leu Gly Ser Ala His Gln Gly Cys Leu Pro Pro Phe Pro Ala
           355                 360                 365
   Asp Val Phe Met Ile Pro Gln Tyr Gly Tyr Leu Thr Leu Asn Asn Gly
       370                 375                 380
   Ser Gln Ala Val Gly Arg Ser Ser Phe Tyr Cys Leu Glu Tyr Phe Pro
   385                 390                 395                 400
   Ser Gln Met Leu Arg Thr Gly Asn Asn Phe Glu Phe Ser Tyr Thr Phe
                   405                 410                 415
   Glu Asp Val Pro Phe His Ser Ser Tyr Ala His Ser Gln Ser Leu Asp
               420                 425                 430
   Arg Leu Met Asn Pro Leu Ile Asp Gln Tyr Leu Tyr Tyr Leu Ser Arg
           435                 440                 445
   Thr Gln Thr Thr Ser Gly Thr Ala Gly Asn Arg Thr Leu Gln Phe Ser
       450                 455                 460
   Gln Ala Gly Pro Ser Ser Met Ala Asn Gln Ala Lys Asn Trp Leu Pro
   465                 470                 475                 480
   Gly Pro Cys Tyr Arg Gln Gln Arg Val Ser Lys Thr Ala Asn Gln Asn
                   485                 490                 495
   Asn Asn Ser Asn Phe Ala Trp Thr Gly Ala Thr Lys Tyr His Leu Asn
               500                 505                 510
   Gly Arg Asp Ser Leu Val Asn Pro Gly Pro Ala Met Ala Thr His Lys
           515                 520                 525
   Asp Asp Glu Asp Lys Phe Phe Pro Met Ser Gly Val Leu Ile Phe Gly
       530                 535                 540
   Lys Gln Gly Ala Gly Asn Ser Asn Val Asp Leu Asp Asn Val Met Ile
   545                 550                 555                 560
   Thr Ser Glu Glu Glu Ile Lys Thr Thr Asn Pro Val Ala Thr Glu Gln
                   565                 570                 575
   Tyr Gly Thr Val Ala Thr Asn Leu Gln Ser Ser Asn Thr Ala Pro Ala
               580                 585                 590
   Thr Gly Thr Val Asn Ser Gln Gly Ala Leu Pro Gly Met Val Trp Gln
           595                 600                 605
   Asn Arg Asp Val Tyr Leu Gln Gly Pro Ile Trp Ala Lys Ile Pro His
       610                 615                 620
   Thr Asp Gly His Phe His Pro Ser Pro Leu Met Gly Gly Phe Gly Leu
   625                 630                 635                 640
   Lys His Pro Pro Pro Gln Ile Leu Ile Lys Asn Thr Pro Val Pro Ala
                   645                 650                 655
   Asn Pro Pro Thr Thr Phe Ser Pro Ala Lys Phe Ala Ser Phe Ile Thr
               660                 665                 670
   Gln Tyr Ser Thr Gly Gln Val Ser Val Glu Ile Glu Trp Glu Leu Gln
           675                 680                 685
   Lys Glu Asn Ser Lys Arg Trp Asn Pro Glu Ile Gln Tyr Thr Ser Asn
       690                 695                 700
   Tyr Asn Lys Ser Thr Asn Val Asp Phe Ala Val Asp Thr Asn Gly Val
   705                 710                 715                 720
   Tyr Ser Glu Pro Arg Pro Ile Gly Thr Arg Tyr Leu Thr Arg Asn Leu
                   725                 730                 735
<210> 2
   <211> 2208
   <212> DNA
   <213> Adeno-associated virus
<220>
   <221> variation
   <222> (502)..(504)
   <223> /replace="aaa"
<220>
   <221> variation
   <222> (610)..(612)
   <223> /replace="agc"
<220>
   <221> variation
   <222> (796)..(798)
   <223> /replace="ggc"
<220>
   <221> variation
   <222> (931)..(933)
   <223> /replace="aag"
<220>
   <221> variation
   <222> (1231)..(1233)
   <223> /replace="cag"
<220>
   <221> variation
   <222> (1378)..(1380)
   <223> /replace="gag"
<220>
   <221> variation
   <222> (1477)..(1479)
   <223> /replace="acc"
<220>
   <221> variation
   <222> (1684)..(1686)
   <223> /replace="aac"
<220>
   <221> variation
   <222> (1726)..(1728)
   <223> /replace="gag"
<220>
   <221> variation
   <222> (1759)..(1761)
   <223> /replace="gcc"
<220>
   <221> variation
   <222> (1825)..(1827)
   <223> /replace="gac"
<220>
   <221> misc_feature
   <222> (1)..(2208)
   <223> /note="Variation nucleotides given in the sequence have no preference with respect to those in the annotations for variation positions"
<400> 2
   atggctgccg atggttatct tccagattgg ctcgaggaca acctctctga gggcattcgc      60
   gagtggtggg acttgaaacc tggagccccg aaacccaaag ccaaccagca aaagcaggac     120
   gacggccggg gtctggtgct tcctggctac aagtacctcg gacccttcaa cggactcgac     180
   aagggggagc ccgtcaacgc ggcggacgca gcggccctcg agcacgacaa ggcctacgac     240
   cagcagctca aagcgggtga caatccgtac ctgcggtata accacgccga cgccgagttt     300
   caggagcgtc tgcaagaaga tacgtctttt gggggcaacc tcgggcgagc agtcttccag     360
   gccaagaagc gggttctcga acctctcggt ctggttgagg aaggcgctaa gacggctcct     420
   ggaaagaaga gaccggtaga gcaatcaccc caggaaccag actcctcttc gggcatcggc     480
   aagaaaggcc agcagcccgc gaagaagaga ctcaactttg ggcagacagg cgactcagag     540
   tcagtgcccg accctcaacc actcggagaa ccccccgcag ccccctctgg tgtgggatct     600
   aatacaatgg cagcaggcgg tggcgctcca atggcagaca ataacgaagg cgccgacgga     660
   gtgggtaacg cctcaggaaa ttggcattgc gattccacat ggctgggcga cagagtcatc     720
   accaccagca cccgaacctg ggccctcccc acctacaaca accacctcta caagcaaatc     780
   tccagccaat cgggagcaag caccaacgac aacacctact tcggctacag caccccctgg     840
   gggtattttg actttaacag attccactgc cacttctcac cacgtgactg gcagcgactc     900
   atcaacaaca actggggatt ccggcccaag agactcaact tcaagctctt caacatccag     960
   gtcaaggagg tcacgacgaa tgatggcacc acgaccatcg ccaataacct taccagcacg    1020
   gttcaggtct ttacggactc ggaataccag ctcccgtacg tcctcggctc tgcgcaccag    1080
   ggctgcctgc ctccgttccc ggcggacgtc ttcatgattc ctcagtacgg gtacctgact    1140
   ctgaacaatg gcagtcaggc cgtgggccgt tcctccttct actgcctgga gtactttcct    1200
   tctcaaatgc tgagaacggg caacaacttt gagttcagct acacgtttga ggacgtgcct    1260
   tttcacagca gctacgcgca cagccaaagc ctggaccggc tgatgaaccc cctcatcgac    1320
   cagtacctgt actacctgtc tcggactcag accacgagtg gtaccgcagg aaatcggacg    1380
   ttgcaatttt ctcaggccgg gcctagtagc atggcgaatc aggccaaaaa ctggctaccc    1440
   gggccctgct accggcagca acgcgtctcc aagacagcga atcaaaataa caacagcaac    1500
   tttgcctgga ccggtgccac caagtatcat ctgaatggca gagactctct ggtaaatccc    1560
   ggtcccgcta tggcaaccca caaggacgac gaagacaaat tttttccgat gagcggagtc    1620
   ttaatatttg ggaaacaggg agctggaaat agcaacgtgg accttgacaa cgttatgata    1680
   accagtgagg aagaaattaa aaccaccaac ccagtggcca cagaacagta cggcacggtg    1740
   gccactaacc tgcaatcgtc aaacaccgct cctgctacag ggaccgtcaa cagtcaagga    1800
   gccttacctg gcatggtctg gcagaaccgg gacgtgtacc tgcagggtcc tatctgggcc    1860
   aagattcctc acacggacgg acactttcat ccctcgccgc tgatgggagg ctttggactg    1920
   aaacacccgc ctcctcagat cctgattaag aatacacctg ttcccgcgaa tcctccaact    1980
   accttcagtc cagctaagtt tgcgtcgttc atcacgcagt acagcaccgg acaggtcagc    2040
   gtggaaattg aatgggagct gcagaaagaa aacagcaaac gctggaaccc agagattcaa    2100
   tacacttcca actacaacaa atctacaaat gtggactttg ctgttgacac aaatggcgtt    2160
   tattctgagc ctcgccccat cggcacccgt tacctcaccc gtaatctg                 2208
<210> 3
   <211> 737
   <212> PRT
   <213> Adeno-associated virus
<220>
   <221> VARIANT
   <222> (157)..(157)
   <223> /replace="Ser"
<220>
   <221> VARIANT
   <222> (168)..(168)
   <223> /replace="Arg"
<220>
   <221> VARIANT
   <222> (262)..(262)
   <223> /replace="Ser"
<220>
   <221> VARIANT
   <222> (263)..(263)
   <223> /replace="His"
<220>
   <221> VARIANT
   <222> (312)..(312)
   <223> /replace="Lys"
<220>
   <221> VARIANT
   <222> (412)..(412)
   <223> /replace="Gln"
<220>
   <221> VARIANT
   <222> (460)..(460)
   <223> /replace="Gln"
<220>
   <221> VARIANT
   <222> (461)..(461)
   <223> /replace="Glu"
<220>
   <221> VARIANT
   <222> (552)..(552)
   <223> /replace="Ser"
<220>
   <221> VARIANT
   <222> (556)..(556)
   <223> /replace="Tyr"
<220>
   <221> VARIANT
   <222> (557)..(557)
   <223> /replace="Ser"
<220>
   <221> VARIANT
   <222> (563)..(563)
   <223> /replace="Asn"
<220>
   <221> VARIANT
   <222> (580)..(580)
   <223> /replace="Ile"
<220>
   <221> VARIANT
   <222> (588)..(588)
   <223> /replace="Ser"
<220>
   <221> VARIANT
   <222> (664)..(664)
   <223> /replace="Thr"
<220>
   <221> misc_feature
   <222> (1)..(737)
   <223> /note="Variant residues given in the sequence have no preference with respect to those in the annotations for variant positions"
<400> 3
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Thr Gly Ile Gly
   145                 150                 155                 160
   Lys Lys Gly Gln Gln Pro Ala Lys Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ala Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Asn Ser Gln Ser Gly Gly Ser Thr Asn Asp Asn
               260                 265                 270
   Thr Tyr Phe Gly Tyr Ser Thr Pro Trp Gly Tyr Phe Asp Phe Asn Arg
           275                 280                 285
   Phe His Cys His Phe Ser Pro Arg Asp Trp Gln Arg Leu Ile Asn Asn
       290                 295                 300
   Asn Trp Gly Phe Arg Pro Lys Arg Leu Asn Phe Lys Leu Phe Asn Ile
   305                 310                 315                 320
   Gln Val Lys Glu Val Thr Thr Asn Asp Gly Thr Thr Thr Ile Ala Asn
                   325                 330                 335
   Asn Leu Thr Ser Thr Val Gln Val Phe Thr Asp Ser Glu Tyr Gln Leu
               340                 345                 350
   Pro Tyr Val Leu Gly Ser Ala His Gln Gly Cys Leu Pro Pro Phe Pro
           355                 360                 365
   Ala Asp Val Phe Met Ile Pro Gln Tyr Gly Tyr Leu Thr Leu Asn Asn
       370                 375                 380
   Gly Ser Gln Ala Val Gly Arg Ser Ser Phe Tyr Cys Leu Glu Tyr Phe
   385                 390                 395                 400
   Pro Ser Gln Met Leu Arg Thr Gly Asn Asn Phe Glu Phe Ser Tyr Thr
                   405                 410                 415
   Phe Glu Asp Val Pro Phe His Ser Ser Tyr Ala His Ser Gln Ser Leu
               420                 425                 430
   Asp Arg Leu Met Asn Pro Leu Ile Asp Gln Tyr Leu Tyr Tyr Leu Ser
           435                 440                 445
   Arg Thr Gln Thr Thr Gly Gly Thr Ala Gly Asn Arg Thr Leu Gln Phe
       450                 455                 460
   Ser Gln Ala Gly Pro Ser Ser Met Ala Asn Gln Ala Lys Asn Trp Leu
   465                 470                 475                 480
   Pro Gly Pro Cys Tyr Arg Gln Gln Arg Val Ser Lys Thr Thr Asn Gln
                   485                 490                 495
   Asn Asn Asn Ser Asn Phe Ala Trp Thr Gly Ala Thr Lys Tyr His Leu
   500 505 510
   Asn Gly Arg Asp Ser Leu Val Asn Pro Gly Val Ala Met Ala Thr His
   515 520 525
   Lys Asp Asp Glu Asp Arg Phe Phe Pro Ser Ser Gly Val Leu Ile Phe
   530 535 540
   Gly Lys Gln Gly Ala Gly Asn Asp Asn Val Asp Leu Asp Asn Val Met
   545 550 555 560
   Ile Thr Ser Glu Glu Glu Ile Lys Thr Thr Asn Pro Val Ala Thr Glu
   565 570 575
   Glu Tyr Gly Val Val Ala Thr Asn Leu Gln Ser Ala Asn Thr Ala Pro
   580 585 590
   Gln Thr Gly Thr Val Asn Ser Gln Gly Ala Leu Pro Gly Met Val Trp
   595 600 605
   Gln Asn Arg Asp Val Tyr Leu Gln Gly Pro Ile Trp Ala Lys Ile Pro
   610 615 620
   His Thr Asp Gly Asn Phe His Pro Ser Pro Leu Met Gly Gly Phe Gly
   625 630 635 640
   Leu Lys His Pro Pro Pro Gln Ile Leu Ile Lys Asn Thr Pro Val Pro
   645 650 655
   Ala Asn Pro Pro Thr Thr Phe Ser Pro Ala Lys Phe Ala Ser Phe Ile
   660 665 670
   Thr Gln Tyr Ser Thr Gly Gln Val Ser Val Glu Ile Glu Trp Glu Leu
   675 680 685
   Gln Lys Glu Asn Ser Lys Arg Trp Asn Pro Glu Ile Gln Tyr Thr Ser
   690 695 700
   Asn Tyr Asn Lys Ser Thr Asn Val Asp Phe Ala Val Asp Thr Glu Gly
   705 710 715 720
   Val Tyr Ser Glu Pro Arg Pro Ile Gly Thr Arg Tyr Leu Thr Arg Asn
   725 730 735
   Leu
<210> 4
   <211> 2211
   <212> DNA
   <213> Adeno-associated virus
<220>
   <221> variation
   <222> (469)..(471)
   <223> /replace="agc"
<220>
   <221> variation
   <222> (502)..(504)
   <223> /replace="aag"
<220>
   <221> variation
   <222> (784)..(786)
   <223> /replace="agt"
<220>
   <221> variation
   <222> (787)..(789)
   <223> /replace="cac"
<220>
   <221> variation
   <222> (934)..(936)
   <223> /replace="aag"
<220>
   <221> variation
   <222> (1234)..(1236)
   <223> /replace="cag"
<220>
   <221> variation
   <222> (1378)..(1380)
   <223> /replace="cag"
<220>
   <221> variation
   <222> (1381)..(1383)
   <223> /replace="gag"
<220>
   <221> variation
   <222> (1654)..(1656)
   <223> /replace="agc"
<220>
   <221> variation
   <222> (1666)..(1668)
   <223> /replace="tac"
<220>
   <221> variation
   <222> (1669)..(1671)
   <223> /replace="agc"
<220>
   <221> variation
   <222> (1687)..(1689)
   <223> /replace="aac"
<220>
   <221> variation
   <222> (1738)..(1740)
   <223> /replace="atc"
<220>
   <221> variation
   <222> (1762)..(1764)
   <223> /replace="agc"
<220>
   <221> variation
   <222> (1990)..(1992)
   <223> /replace="acc"
<220>
   <221> misc_feature
   <222> (1)..(2211)
   <223> /note="Variation nucleotides given in the sequence have no preference with respect to those in the annotations for variation positions"
<400> 4
   atggctgccg atggttatct tccagattgg ctcgaggaca acctctctga gggcattcgc      60
   gagtggtggg acttgaaacc tggagccccg aaacccaaag ccaaccagca aaagcaggac     120
   gacggccggg gtctggtgct tcctggctac aagtacctcg gacccttcaa cggactcgac     180
   aagggggagc ccgtcaacgc ggcggacgca gcggccctcg agcacgacaa ggcctacgac     240
   cagcagctca aagcgggtga caatccgtac ctgcggtata accacgccga cgccgagttt     300
   caggagcgtc tgcaagaaga tacgtctttt gggggcaacc tcgggcgagc agtcttccag     360
   gccaagaagc gggttctcga acctctcggt ctggttgagg aaggcgctaa gacggctcct     420
   ggaaagaaga gaccggtaga gcaatcaccc caggaaccag actcctctac gggcatcggc     480
   aagaaaggcc agcagcccgc gaaaaagaga ctcaactttg ggcagactgg cgactcagag     540
   tcagtgcccg accctcaacc actcggagaa ccccccgcag cccctctggg tgtgggatct     600
   aatacaatgg ctgcaggcgg tggcgctcca atggcagaca ataacgaagg cgccgacgga     660
   gtgggtaatg cctcaggaaa ttggcattgc gattccacat ggctgggcga cagagtcatc     720
   accaccagca cccgaacctg ggccctcccc acctacaaca accacctcta caagcaaatc     780
   tccaacagcc aatcgggagg aagcaccaac gacaacacct acttcggcta cagcaccccc     840
   tgggggtatt ttgactttaa cagattccac tgccacttct caccacgtga ctggcagcga     900
   ctcatcaaca acaactgggg attccggccc aagagactca acttcaagct cttcaacatc     960
   caggtcaagg aggtcacgac gaatgatggc accacgacca tcgccaataa ccttaccagc    1020
   acggttcagg tctttacgga ctcggaatac cagctcccgt acgtcctcgg ctctgcgcac    1080
   cagggctgcc tgcctccgtt cccggcggac gtcttcatga ttcctcagta cgggtacctg      1140
   actctgaaca atggcagtca ggccgtgggc cgttcctcct tctactgcct ggagtacttt      1200
   ccttctcaaa tgctgagaac gggcaacaac tttgagttca gctacacgtt tgaggacgtg      1260
   ccttttcaca gcagctacgc gcacagccaa agcctggacc ggctgatgaa ccccctcatc     1320
   gaccagtacc tgtactacct gtctcggact cagaccacgg gaggtaccgc aggaaatcgg      1380
   acgttgcaat tttctcaggc cgggcctagt agcatggcga atcaggccaa aaactggcta      1440
   cccgggccct gctaccggca gcaacgcgtc tccaagacaa cgaatcaaaa taacaacagc      1500
   aactttgcct ggaccggtgc caccaagtat catctgaatg gcagagactc tctggtaaat      1560
   cccggtgtcg ctatggcaac ccacaaggac gacgaagacc gattttttcc gtccagcgga      1620
   gtcttaatat ttgggaaaca gggagctgga aatgacaacg tggaccttga caacgttatg      1680
   ataaccagtg aggaagaaat taaaaccacc aacccagtgg ccacagaaga gtacggcgtg      1740
   gtggccacta acctgcaatc ggcaaacacc gctcctcaaa cagggaccgt caacagtcaa      1800
   ggagccttac ctggcatggt ctggcagaac cgggacgtgt acctgcaggg tcctatctgg      1860
   gccaagattc ctcacacgga cggaaacttt catccctcgc cgctgatggg aggctttgga      1920
   ctgaaacacc cgcctcctca gatcctgatt aagaatacac ctgttcccgc gaatcctcca      1980
   actaccttca gtccagctaa gtttgcgtcg ttcatcacgc agtacagcac cggacaggtc      2040
   agcgtggaaa ttgaatggga gctgcagaaa gaaaacagca aacgctggaa cccagagatt      2100
   caatacactt ccaactacaa caaatctaca aatgtggact ttgctgttga cacagaaggc      2160
   gtttattctg agcctcgccc catcggcacc cgttacctca cccgtaatct g               2211
<210> 5
   <211> 738
   <212> PRT
   <213> Adeno-associated virus
<220>
   <221> VARIANT
   <222> (158)..(158)
   <223> /replace="Ser"
<220>
   <221> VARIANT
   <222> (169)..(169)
   <223> /replace="Arg"
<220>
   <221> VARIANT
   <222> (564)..(564)
   <223> /replace="Asn"
<220>
   <221> misc_feature
   <222> (1)..(738)
   <223> /note="Variant residues given in the sequence have no preference with respect to those in the annotations for variant positions"
<400> 5
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Arg Glu Pro Asp Ser Ser Thr Gly Ile
   145                 150                 155                 160
   Gly Lys Lys Gly Gln Gln Pro Ala Lys Lys Arg Leu Asn Phe Gly Gln
                   165                 170                 175
   Thr Gly Asp Ser Glu Ser Val Pro Asp Pro Gln Pro Leu Gly Glu Pro
               180                 185                 190
   Pro Ala Ala Pro Ser Gly Val Gly Ser Asn Thr Met Ala Ala Gly Gly
           195                 200                 205
   Gly Ala Pro Met Ala Asp Asn Asn Glu Gly Ala Asp Gly Val Gly Asn
       210                 215                 220
   Ser Ser Gly Asn Trp His Cys Asp Ser Thr Trp Leu Gly Asp Arg Val
   225                 230                 235                 240
   Ile Thr Thr Ser Thr Arg Thr Trp Ala Leu Pro Thr Tyr Asn Asn His
                   245                 250                 255
   Leu Tyr Lys Gln Ile Ser Asn Gly Thr Ser Gly Gly Ser Thr Asn Asp
               260                 265                 270
   Asn Thr Tyr Phe Gly Tyr Ser Thr Pro Trp Gly Tyr Phe Asp Phe Asn
           275                 280                 285
   Arg Phe His Cys His Phe Ser Pro Arg Asp Trp Gln Arg Leu Ile Asn
       290                 295                 300
   Asn Asn Trp Gly Phe Arg Pro Lys Arg Leu Asn Phe Lys Leu Phe Asn
   305                 310                 315                 320
   Ile Gln Val Lys Glu Val Thr Thr Asn Glu Gly Thr Lys Thr Ile Ala
                   325                 330                 335
   Asn Asn Leu Thr Ser Thr Val Gln Val Phe Thr Asp Ser Glu Tyr Gln
               340                 345                 350
   Leu Pro Tyr Val Leu Gly Ser Ala His Gln Gly Cys Leu Pro Pro Phe
           355                 360                 365
   Pro Ala Asp Val Phe Met Ile Pro Gln Tyr Gly Tyr Leu Thr Leu Asn
       370                 375                 380
   Asn Gly Ser Gln Ala Val Gly Arg Ser Ser Phe Tyr Cys Leu Glu Tyr
   385                 390                 395                 400
   Phe Pro Ser Gln Met Leu Arg Thr Gly Asn Asn Phe Gln Phe Ser Tyr
                   405                 410                 415
   Thr Phe Glu Asp Val Pro Phe His Ser Ser Tyr Ala His Ser Gln Ser
               420                 425                 430
   Leu Asp Arg Leu Met Asn Pro Leu Ile Asp Gln Tyr Leu Tyr Tyr Leu
           435                 440                 445
   Ser Arg Thr Gln Thr Thr Gly Gly Thr Ala Gly Thr Gln Thr Leu Gln
       450                 455                 460
   Phe Ser Gln Ala Gly Pro Ser Ser Met Ala Asn Gln Ala Lys Asn Trp
   465                 470                 475                 480
   Leu Pro Gly Pro Cys Tyr Arg Gln Gln Arg Val Ser Thr Thr Thr Asn
               485                 490                 495
   Gln Asn Asn Asn Ser Asn Phe Ala Trp Thr Gly Ala Thr Lys Tyr His
           500                 505                 510
   Leu Asn Gly Arg Asp Ser Leu Val Asn Pro Gly Val Ala Met Ala Thr
       515                 520                 525
   His Lys Asp Asp Glu Asp Arg Phe Phe Pro Ser Ser Gly Val Leu Ile
   530                 535                 540
   Phe Gly Lys Gln Gly Ala Gly Asn Asp Asn Val Asp Tyr Ser Asn Val
   545                 550                 555                 560
   Met Ile Thr Ser Glu Glu Glu Ile Lys Thr Thr Asn Pro Val Ala Thr
                   565                 570                 575
   Glu Glu Tyr Gly Val Val Ala Thr Asn Leu Gln Ser Ala Asn Thr Ala
               580                 585                 590
   Pro Gln Thr Gly Thr Val Asn Ser Gln Gly Ala Leu Pro Gly Met Val
           595                 600                 605
   Trp Gln Asn Arg Asp Val Tyr Leu Gln Gly Pro Ile Trp Ala Lys Ile
       610                 615                 620
   Pro His Thr Asp Gly Asn Phe His Pro Ser Pro Leu Met Gly Gly Phe
   625                 630                 635                 640
   Gly Leu Lys His Pro Pro Pro Gln Ile Leu Ile Lys Asn Thr Pro Val
                   645                 650                 655
   Pro Ala Asp Pro Pro Thr Thr Phe Asn Gln Ala Lys Leu Asn Ser Phe
               660                 665                 670
   Ile Thr Gln Tyr Ser Thr Gly Gln Val Ser Val Glu Ile Glu Trp Glu
           675                 680                 685
   Leu Gln Lys Glu Asn Ser Lys Arg Trp Asn Pro Glu Ile Gln Tyr Thr
       690                 695                 700
   Ser Asn Tyr Tyr Lys Ser Thr Asn Val Asp Phe Ala Val Asn Thr Glu
   705                 710                 715                 720
   Gly Val Tyr Ser Glu Pro Arg Pro Ile Gly Thr Arg Tyr Leu Thr Arg
                   725                 730                 735
   Asn Leu
<210> 6
   <211> 2214
   <212> DNA
   <213> Adeno-associated virus
<220>
   <221> variation
   <222> (472)..(474)
   <223> /replace="agc"
<220>
   <221> variation
   <222> (505)..(507)
   <223> /replace="aga"
<220>
   <221> variation
   <222> (1690)..(1692)
   <223> /replace="aac"
<220>
   <221> misc_feature
   <222> (1)..(2214)
   <223> /note="Variation nucleotides given in the sequence have no preference with respect to those in the annotations for variation positions"
<400> 6
   atggctgccg atggttatct tccagattgg ctcgaggaca acctctctga gggcattcgc      60
   gagtggtggg acctgaaacc tggagccccg aaacccaaag ccaaccagca aaagcaggac     120
   gacggccggg gtctggtgct tcctggctac aagtacctcg gacccttcaa cggactcgac     180
   aagggggagc ccgtcaacgc ggcggacgca gcggccctcg agcacgacaa ggcctacgac     240
   cagcagctca aagcgggtga caatccgtac ctgcggtata atcacgccga cgccgagttt     300
   caggagcgtc tgcaagaaga tacgtctttt gggggcaacc tcgggcgagc agtcttccag     360
   gccaagaagc gggttctcga acctctcggt ctggttgagg aaggcgctaa gacggctcct     420
   ggaaagaaga gaccggtaga gcagtcacca cagcgtgagc ccgactcctc cacgggcatc     480
   ggcaagaaag gccagcagcc cgccaaaaag agactcaatt tcggtcagac tggcgactca     540
   gagtcagtcc ccgaccctca acctctcgga gaacctccag cagcgccctc tggtgtggga     600
   tctaatacaa tggctgcagg cggtggcgca ccaatggcag acaataacga aggtgccgac     660
   ggagtgggta attcctcggg aaattggcat tgcgattcca catggctggg cgacagagtc     720
   atcaccacca gcacccgaac ctgggccctg cccacctaca acaaccacct ctacaagcaa     780
   atctccaacg ggacctcggg aggcagcacc aacgacaaca cctactttgg ctacagcacc     840
   ccctgggggt attttgactt taacagattc cactgccact tctcaccacg tgactggcag     900
   cgactcatca acaacaactg gggattccgg cccaagagac tcaacttcaa gctcttcaac 960
   atccaggtca aagaggtcac gacgaatgaa ggcaccaaga ccatcgccaa taacctcacc 1020
   agcaccgtcc aggtgtttac ggactcggaa taccagctgc cgtacgtcct cggctctgcc 1080
   caccagggct gcctgcctcc gttcccggcg gacgtcttca tgattcctca gtacggctac 1140
   ctgactctca acaacggtag tcaggccgtg ggacgttcct ccttctactg cctggagtac 1200
   ttcccctctc agatgctgag aacgggcaac aactttcaat tcagctacac tttcgaggac 1260
   gtgcctttcc acagcagcta cgcgcacagc cagagtttgg acaggctgat gaatcctctc 1320
   atcgaccagt acctgtacta cctgtcaaga acccagacta cgggaggcac agcgggaacc 1380
   cagacgttgc agttttctca ggccgggcct agcagcatgg cgaatcaggc caaaaactgg 1440
   ctgcctggac cctgctacag acagcagcgc gtctccacga caacgaatca aaacaacaac 1500
   agcaactttg cctggactgg tgccaccaag tatcatctga acggcagaga ctctctggtg 1560
   aatccgggcg tcgccatggc aacccacaag gacgacgagg accgcttctt cccatccagc 1620
   ggcgtcctca tatttggcaa gcagggagct ggaaatgaca acgtggacta tagcaacgtg 1680
   atgataacca gcgaggaaga aatcaagacc accaaccccg tggccacaga agagtatggc 1740
   gtggtggcta ctaacctaca gtcggcaaac accgctcctc aaacggggac cgtcaacagc 1800
   cagggagcct tacctggcat ggtctggcag aaccgggacg tgtacctgca gggtcctatt 1860
   tgggccaaga ttcctcacac agatggcaac tttcacccgt ctcctttaat gggcggcttt 1920
   ggacttaaac atccgcctcc tcagatcctc atcaaaaaca ctcctgttcc tgcggatcct 1980
   ccaacaacgt tcaaccaggc caagctgaat tctttcatca cgcagtacag caccggacaa 2040
   gtcagcgtgg agatcgagtg ggagctgcag aaggagaaca gcaagcgctg gaacccagag 2100
   attcagtata cttccaacta ctacaaatct acaaatgtgg actttgctgt taatactgag 2160
   ggtgtttact ctgagcctcg ccccattggc actcgttacc tcacccgtaa tctg 2214
<210> 7
   <211> 738
   <212> PRT
   <213> Adeno-associated virus
<220>
   <221> VARIANT
   <222> (158)..(158)
   <223> /replace="Ser"
<220>
   <221> VARIANT
   <222> (169)..(169)
   <223> /replace="Lys"
<220>
   <221> VARIANT
   <222> (315)..(315)
   <223> /replace="Ser"
<220>
   <221> VARIANT
   <222> (413)..(413)
   <223> /replace="Glu"
<220>
   <221> VARIANT
   <222> (472)..(472)
   <223> /replace="Thr" or "Ser"
<220>
   <221> VARIANT
   <222> (534)..(534)
   <223> /replace="Glu"
<220>
   <221> VARIANT
   <222> (542)..(542)
   <223> /replace="Val"
<220>
   <221> VARIANT
   <222> (595)..(595)
   <223> /replace="Val"
<220>
   <221> misc_feature
   <222> (1)..(738)
   <223> /note="Variant residues given in the sequence have no preference with respect to those in the annotations for variant positions"
<400> 7
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Arg Glu Pro Asp Ser Ser Thr Gly Ile
   145                 150                 155                 160
   Gly Lys Lys Gly Gln Gln Pro Ala Arg Lys Arg Leu Asn Phe Gly Gln
                   165                 170                 175
   Thr Gly Asp Ser Glu Ser Val Pro Asp Pro Gln Pro Leu Gly Glu Pro
               180                 185                 190
   Pro Ala Ala Pro Ser Gly Val Gly Ser Asn Thr Met Ala Ala Gly Gly
           195                 200                 205
   Gly Ala Pro Met Ala Asp Asn Asn Glu Gly Ala Asp Gly Val Gly Ser
       210                 215                 220
   Ser Ser Gly Asn Trp His Cys Asp Ser Thr Trp Leu Gly Asp Arg Val
   225                 230                 235                 240
   Ile Thr Thr Ser Thr Arg Thr Trp Ala Leu Pro Thr Tyr Asn Asn His
                   245                 250                 255
   Leu Tyr Lys Gln Ile Ser Asn Gly Thr Ser Gly Gly Ser Thr Asn Asp
               260                 265                 270
   Asn Thr Tyr Phe Gly Tyr Ser Thr Pro Trp Gly Tyr Phe Asp Phe Asn
           275                 280                 285
   Arg Phe His Cys His Phe Ser Pro Arg Asp Trp Gln Arg Leu Ile Asn
       290                 295                 300
   Asn Asn Trp Gly Phe Arg Pro Lys Arg Leu Asn Phe Lys Leu Phe Asn
   305                 310                 315                 320
   Ile Gln Val Lys Glu Val Thr Gln Asn Glu Gly Thr Lys Thr Ile Ala
                   325                 330                 335
   Asn Asn Leu Thr Ser Thr Ile Gln Val Phe Thr Asp Ser Glu Tyr Gln
               340                 345                 350
   Leu Pro Tyr Val Leu Gly Ser Ala His Gln Gly Cys Leu Pro Pro Phe
           355                 360                 365
   Pro Ala Asp Val Phe Met Ile Pro Gln Tyr Gly Tyr Leu Thr Leu Asn
   370 375 380
   Asn Gly Ser Gln Ala Val Gly Arg Ser Ser Phe Tyr Cys Leu Glu Tyr
   385 390 395 400
   Phe Pro Ser Gln Met Leu Arg Thr Gly Asn Asn Phe Gln Phe Ser Tyr
   405 410 415
   Thr Phe Glu Asp Val Pro Phe His Ser Ser Tyr Ala His Ser Gln Ser
   420 425 430
   Leu Asp Arg Leu Met Asn Pro Leu Ile Asp Gln Tyr Leu Tyr Tyr Leu
   435 440 445
   Ser Arg Thr Gln Thr Thr Gly Gly Thr Ala Gly Thr Gln Thr Leu Gln
   450 455 460
   Phe Ser Gln Ala Gly Pro Ser Asn Met Ala Asn Gln Ala Lys Asn Trp
   465 470 475 480
   Leu Pro Gly Pro Cys Tyr Arg Gln Gln Arg Val Ser Thr Thr Thr Ser
   485 490 495
   Gln Asn Asn Asn Ser Asn Phe Ala Trp Thr Gly Ala Thr Lys Tyr His
   500 505 510
   Leu Asn Gly Arg Asp Ser Leu Val Asn Pro Gly Val Ala Met Ala Thr
   515 520 525
   His Lys Asp Asp Glu Asp Arg Phe Phe Pro Ser Ser Gly Ile Leu Ile
   530 535 540
   Phe Gly Lys Gln Gly Ala Gly Lys Asp Asn Val Asp Tyr Ser Asn Val
   545 550 555 560
   Met Leu Thr Ser Glu Glu Glu Ile Lys Thr Thr Asn Pro Val Ala Thr
   565 570 575
   Glu Glu Tyr Gly Val Val Ala Asp Asn Leu Gln Gln Gln Asn Thr Ala
   580 585 590
   Pro Gln Ile Gly Thr Val Asn Ser Gln Gly Ala Leu Pro Gly Met Val
   595 600 605
   Trp Gln Asn Arg Asp Val Tyr Leu Gln Gly Pro Ile Trp Ala Lys Ile
       610                 615                 620
   Pro His Thr Asp Gly Asn Phe His Pro Ser Pro Leu Met Gly Gly Phe
   625                 630                 635                 640
   Gly Leu Lys His Pro Pro Pro Gln Ile Leu Ile Lys Asn Thr Pro Val
                   645                 650                 655
   Pro Ala Asp Pro Pro Thr Thr Phe Asn Gln Ala Lys Leu Asn Ser Phe
               660                 665                 670
   Ile Thr Gln Tyr Ser Thr Gly Gln Val Ser Val Glu Ile Glu Trp Glu
           675                 680                 685
   Leu Gln Lys Glu Asn Ser Lys Arg Trp Asn Pro Glu Ile Gln Tyr Thr
       690                 695                 700
   Ser Asn Tyr Tyr Lys Ser Thr Asn Val Asp Phe Ala Val Asn Thr Glu
   705                 710                 715                 720
   Gly Val Tyr Ser Glu Pro Arg Pro Ile Gly Thr Arg Tyr Leu Thr Arg
                   725                 730                 735
   Asn Leu
<210> 8
   <211> 2214
   <212> DNA
   <213> Adeno-associated virus
<220>
   <221> variation
   <222> (472)..(474)
   <223> /replace="agc"
<220>
   <221> variation
   <222> (505)..(507)
   <223> /replace="aag"
<220>
   <221> variation
   <222> (943)..(945)
   <223> /replace="agc"
<220>
   <221> variation
   <222> (1237)..(1239)
   <223> /replace="gaa"
<220>
   <221> variation
   <222> (1414)..(1416)
   <223> /replace="aac" or "agc"
<220>
   <221> variation
   <222> (1600)..(1602)
   <223> /replace="gag"
<220>
   <221> variation
   <222> (1624)..(1626)
   <223> /replace="gtc"
<220>
   <221> variation
   <222> (1783)..(1785)
   <223> /replace="gta"
<220>
   <221> misc_feature
   <222> (1)..(2214)
   <223> /note="Variation nucleotides given in the sequence have no preference with respect to those in the annotations for variation positions"
<400> 8
   atggctgccg atggttatct tccagattgg ctcgaggaca acctctctga gggcattcgc 60
   gagtggtggg acctgaaacc tggagccccg aaacccaaag ccaaccagca aaagcaggac 120
   gacggccggg gtctggtgct tcctggctac aagtacctcg gacccttcaa cggactcgac 180
   aagggggagc ccgtcaacgc ggcggacgca gcggccctcg agcacgacaa ggcctacgac 240
   cagcagctca aagcgggtga caatccgtac ctgcggtata atcacgccga cgccgagttt 300
   caggagcgtc tgcaagaaga tacgtctttt gggggcaacc tcgggcgagc agtcttccag 360
   gccaagaagc gggttctcga acctctcggt ctggttgagg aaggcgctaa gacggctcct 420
   ggaaagaaga gaccggtaga gcagtcacca cagcgtgagc ccgactcctc cacgggcatc 480
   ggcaagaaag gccagcagcc cgccagaaag agactcaatt tcggtcagac tggcgactca 540
   gagtcagtcc ccgaccctca acctctcgga gaacctccag cagcgccctc tggtgtggga 600
   tctaatacaa tggctgcagg cggtggcgca ccaatggcag acaataacga aggtgccgac 660
   ggagtgggta gttcctcggg aaattggcat tgcgattcca catggctggg cgacagagtc 720
   atcaccacca gcacccgaac ctgggccctg cccacctaca acaaccacct ctacaagcaa 780
   atctccaacg ggacctcggg aggcagcacc aacgacaaca cctactttgg ctacagcacc 840
   ccctgggggt attttgactt taacagattc cactgccact tctcaccacg tgactggcag 900
   cgactcatca acaacaactg gggattccgg cccaagagac tcaacttcaa gctcttcaac 960
   atccaggtca aagaggtcac gcagaatgaa ggcaccaaga ccatcgccaa taacctcacc 1020
   agcaccatcc aggtgtttac ggactcggaa taccagctgc cgtacgtcct cggctctgcc 1080
   caccagggct gcctgcctcc gttcccggcg gacgtcttca tgattcctca gtacggctac 1140
   ctgactctca acaacggtag tcaggccgtg ggacgttcct ccttctactg cctggagtac 1200
   ttcccctctc agatgctgag aacgggcaac aactttcaat tcagctacac tttcgaggac 1260
   gtgcctttcc acagcagcta cgcgcacagc cagagtttgg acaggctgat gaatcctctc 1320
   atcgaccagt acctgtacta cctgtcaaga acccagacta cgggaggcac agcgggaacc 1380
   cagacgttgc agttttctca ggccgggcct agcaacatgg cgaatcaggc caaaaactgg 1440
   ctgcctggac cctgctacag acagcagcgc gtctccacga caacgtcgca aaacaacaac 1500
   agcaactttg cctggactgg tgccaccaag tatcatctga acggcagaga ctctctggtg 1560
   aatccgggcg tcgccatggc aacccacaag gacgacgagg accgcttctt cccatccagc 1620
   ggcatcctca tatttggcaa gcagggagct ggaaaagaca acgtggacta tagcaacgtg 1680
   atgctaacca gcgaggaaga aatcaagacc accaaccccg tggccacaga agagtatggc 1740
   gtggtggctg ataacctaca gcagcaaaac accgctcctc aaatagggac cgtcaacagc 1800
   cagggagcct tacctggcat ggtctggcag aaccgggacg tgtacctgca gggtcctatt 1860
   tgggccaaga ttcctcacac agatggcaac tttcacccgt ctcctttaat gggcggcttt 1920
   ggacttaaac atccgcctcc tcagatcctc atcaaaaaca ctcctgttcc tgcggatcct 1980
   ccaacaacgt tcaaccaggc caagctgaat tctttcatca cgcagtacag caccggacaa 2040
   gtcagcgtgg agatcgagtg ggagctgcag aaggagaaca gcaagcgctg gaacccagag 2100
   attcagtata cttccaacta ctacaaatct acaaatgtgg actttgctgt taatactgag 2160
   ggtgtttact ctgagcctcg ccccattggc actcgttacc tcacccgtaa tctg 2214
<210> 9
   <211> 738
   <212> PRT
   <213> Adeno-associated virus
<220>
   <221> VARIANT
   <222> (169)..(169)
   <223> /replace="Lys"
<220>
   <221> VARIANT
   <222> (315)..(315)
   <223> /replace="Ser"
<220>
   <221> VARIANT
   <222> (534)..(534)
   <223> /replace="Glu"
<220>
   <221> VARIANT
   <222> (542)..(542)
   <223> /replace="Val"
<220>
   <221> misc_feature
   <222> (1)..(738)
   <223> /note="Variant residues given in the sequence have no preference with respect to those in the annotations for variant positions"
<400> 9
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Pro Ser Pro Gln Arg Ser Pro Asp Ser Ser Thr Gly Ile
   145                 150                 155                 160
   Gly Lys Lys Gly Gln Gln Pro Ala Arg Lys Arg Leu Asn Phe Gly Gln
                   165                 170                 175
   Thr Gly Asp Ser Glu Ser Val Pro Asp Pro Gln Pro Ile Gly Glu Pro
               180                 185                 190
   Pro Ala Ala Pro Ser Gly Val Gly Ser Gly Thr Met Ala Ala Gly Gly
           195                 200                 205
   Gly Ala Pro Met Ala Asp Asn Asn Glu Gly Ala Asp Gly Val Gly Ser
       210                 215                 220
   Ser Ser Gly Asn Trp His Cys Asp Ser Thr Trp Leu Gly Asp Arg Val
   225                 230                 235                 240
   Ile Thr Thr Ser Thr Arg Thr Trp Ala Leu Pro Thr Tyr Asn Asn His
                   245                 250                 255
   Leu Tyr Lys Gln Ile Ser Asn Gly Thr Ser Gly Gly Ser Thr Asn Asp
               260                 265                 270
   Asn Thr Tyr Phe Gly Tyr Ser Thr Pro Trp Gly Tyr Phe Asp Phe Asn
           275                 280                 285
   Arg Phe His Cys His Phe Ser Pro Arg Asp Trp Gln Arg Leu Ile Asn
       290                 295                 300
   Asn Asn Trp Gly Phe Arg Pro Lys Arg Leu Asn Phe Lys Leu Phe Asn
   305                 310                 315                 320
   Ile Gln Val Lys Glu Val Thr Gln Asn Glu Gly Thr Lys Thr Ile Ala
                   325                 330                 335
   Asn Asn Leu Thr Ser Thr Ile Gln Val Phe Thr Asp Ser Glu Tyr Gln
               340                 345                 350
   Leu Pro Tyr Val Leu Gly Ser Ala His Gln Gly Cys Leu Pro Pro Phe
           355                 360                 365
   Pro Ala Asp Val Phe Met Ile Pro Gln Tyr Gly Tyr Leu Thr Leu Asn
       370                 375                 380
   Asn Gly Ser Gln Ala Val Gly Arg Ser Ser Phe Tyr Cys Leu Glu Tyr
   385                 390                 395                 400
   Phe Pro Ser Gln Met Leu Arg Thr Gly Asn Asn Phe Glu Phe Ser Tyr
                   405                 410                 415
   Thr Phe Glu Asp Val Pro Phe His Ser Ser Tyr Ala His Ser Gln Ser
               420                 425                 430
   Leu Asp Arg Leu Met Asn Pro Leu Ile Asp Gln Tyr Leu Tyr Tyr Leu
           435                 440                 445
   Ser Arg Thr Gln Ser Thr Gly Gly Thr Ala Gly Thr Gln Gln Leu Leu
       450                 455                 460
   Phe Ser Gln Ala Gly Pro Ser Asn Met Ser Ala Gln Ala Lys Asn Trp
   465 470 475 480
   Leu Pro Gly Pro Cys Tyr Arg Gln Gln Arg Val Ser Thr Thr Leu Ser
   485 490 495
   Gln Asn Asn Asn Ser Asn Phe Ala Trp Thr Gly Ala Thr Lys Tyr His
   500 505 510
   Leu Asn Gly Arg Asp Ser Leu Val Asn Pro Gly Val Ala Met Ala Thr
   515 520 525
   His Lys Asp Asp Glu Asp Arg Phe Phe Pro Ser Ser Gly Ile Leu Met
   530 535 540
   Phe Gly Lys Gln Gly Ala Gly Lys Asp Asn Val Asp Tyr Ser Asn Val
   545 550 555 560
   Met Leu Thr Ser Glu Glu Glu Ile Lys Thr Thr Asn Pro Val Ala Thr
   565 570 575
   Glu Gln Tyr Gly Val Val Ala Asp Asn Leu Gln Gln Gln Asn Thr Ala
   580 585 590
   Pro Ile Val Gly Ala Val Asn Ser Gln Gly Ala Leu Pro Gly Met Val
   595 600 605
   Trp Gln Asn Arg Asp Val Tyr Leu Gln Gly Pro Ile Trp Ala Lys Ile
   610 615 620
   Pro His Thr Asp Gly Asn Phe His Pro Ser Pro Leu Met Gly Gly Phe
   625 630 635 640
   Gly Leu Lys His Pro Pro Pro Gln Ile Leu Ile Lys Asn Thr Pro Val
   645 650 655
   Pro Ala Asp Pro Pro Thr Thr Phe Asn Gln Ala Lys Leu Asn Ser Phe
   660 665 670
   Ile Thr Gln Tyr Ser Thr Gly Gln Val Ser Val Glu Ile Glu Trp Glu
   675 680 685
   Leu Gln Lys Glu Asn Ser Lys Arg Trp Asn Pro Glu Ile Gln Tyr Thr
   690 695 700
   Ser Asn Tyr Tyr Lys Ser Thr Asn Val Asp Phe Ala Val Asn Thr Glu
   705 710 715 720
   Gly Val Tyr Ser Glu Pro Arg Pro Ile Gly Thr Arg Tyr Leu Thr Arg
   725 730 735
   Asn Leu
<210> 10
   <211> 2214
   <212> DNA
   <213> Adeno-associated virus
<220>
   <221> variation
   <222> (505)..(507)
   <223> /replace="aaa"
<220>
   <221> variation
   <222> (943)..(945)
   <223> /replace="agc"
<220>
   <221> variation
   <222> (1600)..(1602)
   <223> /replace="gag"
<220>
   <221> variation
   <222> (1624)..(1626)
   <223> /replace="gtc"
<220>
   <221> misc_feature
   <222> (1)..(2214)
   <223> /note="Variation nucleotides given in the sequence have no preference with respect to those in the annotations for variation positions"
<400> 10
   atggctgccg atggttatct tccagattgg ctcgaggaca acctctctga gggcattcgc      60
   gagtggtggg acctgaaacc tggagccccg aaacccaaag ccaaccagca aaagcaggac     120
   gacggccggg gtctggtgct tcctggctac aagtacctcg gacccttcaa cggactcgac     180
   aagggggagc ccgtcaacgc ggcggacgca gcggccctcg agcacgacaa ggcctacgac     240
   cagcagctca aagcgggtga caatccgtac ctgcggtata atcacgccga cgccgagttt     300
   caggagcgtc tgcaagaaga tacgtctttt gggggcaacc tcgggcgagc agtcttccag     360
   gccaagaagc gggttctcga acctctcggt ctggttgagg aaggcgctaa gacggctcct     420
   ggaaagaaga gaccggtaga gccgtcacca cagcgttccc ccgactcctc cacgggcatc     480
   ggcaagaaag gccagcagcc cgccagaaag agactcaatt tcggtcagac tggcgactca     540
   gagtcagtcc ccgaccctca acctatcgga gaacctccag cagcgccctc tggtgtggga     600
   tctggtacaa tggctgcagg cggtggcgca ccaatggcag acaataacga aggtgccgac     660
   ggagtgggta gttcctcggg aaattggcat tgcgattcca catggctggg cgacagagtc      720
   atcaccacca gcacccgaac ctgggccctg cccacctaca acaaccacct ctacaagcaa      780
   atctccaacg ggacctcggg aggcagcacc aacgacaaca cctactttgg ctacagcacc      840
   ccctgggggt attttgactt taacagattc cactgccact tctcaccacg tgactggcag      900
   cgactcatca acaacaactg gggattccgg cccaagagac tcaacttcaa gctcttcaac      960
   atccaggtca aagaggtcac gcagaatgaa ggcaccaaga ccatcgccaa taacctcacc     1020
   agcaccatcc aggtgtttac ggactcggaa taccagctgc cgtacgtcct cggctctgcc     1080
   caccagggct gcctgcctcc gttcccggcg gacgtcttca tgattcctca gtacggctac     1140
   ctgactctca acaacggtag tcaggccgtg ggacgttcct ccttctactg cctggagtac     1200
   ttcccctctc agatgctgag aacgggcaac aactttgagt tcagctacac tttcgaggac     1260
   gtgcctttcc acagcagcta cgcgcacagc cagagtttgg acaggctgat gaatcctctc     1320
   atcgaccagt acctgtacta cctgtcaaga acccagtcta cgggaggcac agcgggaacc     1380
   cagcagttgc tgttttctca ggccgggcct agcaacatgt cggctcaggc caaaaactgg     1440
   ctgcctggac cctgctacag acagcagcgc gtctccacga cactgtcgca aaacaacaac     1500
   agcaactttg cctggactgg tgccaccaag tatcatctga acggcagaga ctctctggtg     1560
   aatccgggcg tcgccatggc aacccacaag gacgacgagg accgcttctt cccatccagc     1620
   ggcatcctca tgtttggcaa gcagggagct ggaaaagaca acgtggacta tagcaacgtg     1680
   atgctaacca gcgaggaaga aatcaagacc accaaccccg tggccacaga acagtatggc     1740
   gtggtggctg ataacctaca gcagcaaaac accgctccta ttgtgggggc cgtcaacagc     1800
   cagggagcct tacctggcat ggtctggcag aaccgggacg tgtacctgca gggtcctatt     1860
   tgggccaaga ttcctcacac agatggcaac tttcacccgt ctcctttaat gggcggcttt     1920
   ggacttaaac atccgcctcc tcagatcctc atcaaaaaca ctcctgttcc tgcggatcct     1980
   ccaacaacgt tcaaccaggc caagctgaat tctttcatca cgcagtacag caccggacaa     2040
   gtcagcgtgg agatcgagtg ggagctgcag aaggagaaca gcaagcgctg gaacccagag     2100
   attcagtata cttccaacta ctacaaatct acaaatgtgg actttgctgt taatactgag     2160
   ggtgtttact ctgagcctcg ccccattggc actcgttacc tcacccgtaa tctg           2214
<210> 11
   <211> 738
   <212> PRT
   <213> Adeno-associated virus
<220>
   <221> VARIANT
   <222> (471)..(471)
   <223> /replace="Asn"
<220>
   <221> misc_feature
   <222> (1)..(738)
   <223> /note="Variant residues given in the sequence have no preference with respect to those in the annotations for variant positions"
<400> 11
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Pro Ser Pro Gln Arg Ser Pro Asp Ser Ser Thr Gly Ile
   145                 150                 155                 160
   Gly Lys Lys Gly Gln Gln Pro Ala Lys Lys Arg Leu Asn Phe Gly Gln
                   165                 170                 175
   Thr Gly Asp Ser Glu Ser Val Pro Asp Pro Gln Pro Ile Gly Glu Pro
               180                 185                 190
   Pro Ala Gly Pro Ser Gly Leu Gly Ser Gly Thr Met Ala Ala Gly Gly
           195                 200                 205
   Gly Ala Pro Met Ala Asp Asn Asn Glu Gly Ala Asp Gly Val Gly Ser
   210 215 220
   Ser Ser Gly Asn Trp His Cys Asp Ser Thr Trp Leu Gly Asp Arg Val
   225 230 235 240
   Ile Thr Thr Ser Thr Arg Thr Trp Ala Leu Pro Thr Tyr Asn Asn His
   245 250 255
   Leu Tyr Lys Gln Ile Ser Asn Gly Thr Ser Gly Gly Ser Thr Asn Asp
   260 265 270
   Asn Thr Tyr Phe Gly Tyr Ser Thr Pro Trp Gly Tyr Phe Asp Phe Asn
   275 280 285
   Arg Phe His Cys His Phe Ser Pro Arg Asp Trp Gln Arg Leu Ile Asn
   290 295 300
   Asn Asn Trp Gly Phe Arg Pro Lys Arg Leu Asn Phe Lys Leu Phe Asn
   305 310 315 320
   Ile Gln Val Lys Glu Val Thr Gln Asn Glu Gly Thr Lys Thr Ile Ala
   325 330 335
   Asn Asn Leu Thr Ser Thr Ile Gln Val Phe Thr Asp Ser Glu Tyr Gln
   340 345 350
   Leu Pro Tyr Val Leu Gly Ser Ala His Gln Gly Cys Leu Pro Pro Phe
   355 360 365
   Pro Ala Asp Val Phe Met Ile Pro Gln Tyr Gly Tyr Leu Thr Leu Asn
   370 375 380
   Asn Gly Ser Gln Ala Val Gly Arg Ser Ser Phe Tyr Cys Leu Glu Tyr
   385 390 395 400
   Phe Pro Ser Gln Met Leu Arg Thr Gly Asn Asn Phe Glu Phe Ser Tyr
   405 410 415
   Thr Phe Glu Asp Val Pro Phe His Ser Ser Tyr Ala His Ser Gln Ser
   420 425 430
   Leu Asp Arg Leu Met Asn Pro Leu Ile Asp Gln Tyr Leu Tyr Tyr Leu
   435 440 445
   Ser Arg Thr Gln Ser Thr Gly Gly Thr Ala Gly Thr Gln Gln Leu Leu
   450 455 460
   Phe Ser Gln Ala Gly Pro Ser Asn Met Ser Ala Gln Ala Lys Asn Trp
   465 470 475 480
   Leu Pro Gly Pro Cys Tyr Arg Gln Gln Arg Val Ser Thr Thr Leu Ser
   485 490 495
   Gln Asn Asn Asn Ser Asn Phe Ala Trp Thr Gly Ala Thr Lys Tyr His
   500 505 510
   Leu Asn Gly Arg Asp Ser Leu Val Asn Pro Gly Val Ala Met Ala Thr
   515 520 525
   His Lys Asp Asp Glu Glu Arg Phe Phe Pro Ser Ser Gly Val Leu Met
   530 535 540
   Phe Gly Lys Gln Gly Ala Gly Lys Asp Asn Val Asp Tyr Ser Ser Val
   545 550 555 560
   Met Leu Thr Ser Glu Glu Glu Ile Lys Thr Thr Asn Pro Val Ala Thr
   565 570 575
   Glu Gln Tyr Gly Val Val Ala Asp Asn Leu Gln Gln Gln Asn Thr Ala
   580 585 590
   Pro Ile Val Gly Ala Val Asn Ser Gln Gly Ala Leu Pro Gly Met Val
   595 600 605
   Trp Gln Asn Arg Asp Val Tyr Leu Gln Gly Pro Ile Trp Ala Lys Ile
   610 615 620
   Pro His Thr Asp Gly Asn Phe His Pro Ser Pro Leu Met Gly Gly Phe
   625 630 635 640
   Gly Leu Lys His Pro Pro Pro Gln Ile Leu Ile Lys Asn Thr Pro Val
   645 650 655
   Pro Ala Asp Pro Pro Thr Thr Phe Ser Gln Ala Lys Leu Ala Ser Phe
   660 665 670
   Ile Thr Gln Tyr Ser Thr Gly Gln Val Ser Val Glu Ile Glu Trp Glu
   675 680 685
   Leu Gln Lys Glu Asn Ser Lys Arg Trp Asn Pro Glu Ile Gln Tyr Thr
   690 695 700
   Ser Asn Tyr Tyr Lys Ser Thr Asn Val Asp Phe Ala Val Asn Thr Glu
   705 710 715 720
   Gly Thr Tyr Ser Glu Pro Arg Pro Ile Gly Thr Arg Tyr Leu Thr Arg
                   725                 730                 735
   Asn Leu
<210> 12
   <211> 2214
   <212> DNA
   <213> Adeno-associated virus
<220>
   <221> variation
   <222> (1411)..(1413)
   <223> /replace="aat"
<220>
   <221> misc_feature
   <222> (1)..(2214)
   <223> /note="Variation nucleotides given in the sequence have no preference with respect to those in the annotations for variation positions"
<400> 12
   atggctgccg atggttatct tccagattgg ctcgaggaca acctctctga gggcattcgc      60
   gagtggtggg acttgaaacc tggagccccg aaacccaaag ccaaccagca aaagcaggac     120
   gacggccggg gtctggtgct tcctggctac aagtacctcg gacccttcaa cggactcgac     180
   aagggggagc ccgtcaacgc ggcggacgca gcggccctcg agcacgacaa ggcctacgac     240
   cagcagctca aagcgggtga caatccgtac ctgcggtata accacgccga cgccgagttt     300
   caggagcgtc tgcaagaaga tacgtctttt gggggcaacc tcgggcgagc agtcttccag     360
   gccaagaagc gggttctcga acctctcggt ctggttgagg aaggcgctaa gacggctcct     420
   ggaaagaaga gaccggtaga gccatcaccc cagcgttctc cagactcctc tacgggcatc     480
   ggcaagaaag gccagcagcc cgcgaaaaag agactcaact ttgggcagac tggcgactca     540
   gagtcagtgc ccgaccctca accaatcgga gaaccccccg caggcccctc tggtctggga      600
   tctggtacaa tggctgcagg cggtggcgct ccaatggcag acaataacga aggcgccgac     660
   ggagtgggta gttcctcagg aaattggcat tgcgattcca catggctggg cgacagagtc     720
   atcaccacca gcacccgaac ctgggccctc cccacctaca acaaccacct ctacaagcaa     780
   atctccaacg ggacttcggg aggaagcacc aacgacaaca cctacttcgg ctacagcacc     840
   ccctgggggt attttgactt taacagattc cactgccact tctcaccacg tgactggcag     900
   cgactcatca acaacaactg gggattccgg cccaagagac tcaacttcaa gctcttcaac     960
   atccaggtca aggaggtcac gcagaatgaa ggcaccaaga ccatcgccaa taaccttacc    1020
   agcacgattc aggtctttac ggactcggaa taccagctcc cgtacgtcct cggctctgcg    1080
   caccagggct gcctgcctcc gttcccggcg gacgtcttca tgattcctca gtacgggtac 1140
   ctgactctga acaatggcag tcaggccgtg ggccgttcct ccttctactg cctggagtac 1200
   tttccttctc aaatgctgag aacgggcaac aactttgagt tcagctacac gtttgaggac 1260
   gtgccttttc acagcagcta cgcgcacagc caaagcctgg accggctgat gaaccccctc 1320
   atcgaccagt acctgtacta cctgtctcgg actcagtcca cgggaggtac cgcaggaact 1380
   cagcagttgc tattttctca ggccgggcct agtaacatgt cggctcaggc caaaaactgg 1440
   ctacccgggc cctgctaccg gcagcaacgc gtctccacga cactgtcgca aaataacaac 1500
   agcaactttg cctggaccgg tgccaccaag tatcatctga atggcagaga ctctctggta 1560
   aatcccggtg tcgctatggc aacccacaag gacgacgaag agcgattttt tccgtccagc 1620
   ggagtcttaa tgtttgggaa acagggagct ggaaaagaca acgtggacta tagcagcgtt 1680
   atgctaacca gtgaggaaga aattaaaacc accaacccag tggccacaga acagtacggc 1740
   gtggtggccg ataacctgca acagcaaaac accgctccta ttgtaggggc cgtcaacagt 1800
   caaggagcct tacctggcat ggtctggcag aaccgggacg tgtacctgca gggtcctatc 1860
   tgggccaaga ttcctcacac ggacggaaac tttcatccct cgccgctgat gggaggcttt 1920
   ggactgaaac acccgcctcc tcagatcctg attaagaata cacctgttcc cgcggatcct 1980
   ccaactacct tcagtcaagc taagctggcg tcgttcatca cgcagtacag caccggacag 2040
   gtcagcgtgg aaattgaatg ggagctgcag aaagaaaaca gcaaacgctg gaacccagag 2100
   attcaataca cttccaacta ctacaaatct acaaatgtgg actttgctgt taacacagaa 2160
   ggcacttatt ctgagcctcg ccccatcggc acccgttacc tcacccgtaa tctg 2214
<210> 13
   <211> 737
   <212> PRT
   <213> Adeno-associated virus
<220>
   <221> VARIANT
   <222> (148)..(148)
   <223> /replace="Gln"
<220>
   <221> VARIANT
   <222> (169)..(169)
   <223> /replace="Arg"
<220>
   <221> VARIANT
   <222> (314)..(314)
   <223> /replace="Asn"
<220>
   <221> VARIANT
   <222> (466)..(466)
   <223> /replace="His"
<220>
   <221> VARIANT
   <222> (563)..(563)
   <223> /replace="Ser"
<220>
   <221> VARIANT
   <222> (580)..(580)
   <223> /replace="Ile"
<220>
   <221> VARIANT
   <222> (588)..(588)
   <223> /replace="Ser"
<220>
   <221> misc_feature
   <222> (1)..(737)
   <223> /note="Variant residues given in the sequence have no preference with respect to those in the annotations for variant positions"
<400> 13
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Pro Ser Pro Gln Arg Ser Pro Asp Ser Ser Thr Gly Ile
   145 150 155 160
   Gly Lys Lys Gly Gln Gln Pro Ala Lys Lys Arg Leu Asn Phe Gly Gln
   165 170 175
   Thr Gly Asp Ser Glu Ser Val Pro Asp Pro Gln Pro Leu Gly Glu Pro
   180 185 190
   Pro Ala Ala Pro Ser Gly Val Gly Ser Gly Thr Met Ala Ala Gly Gly
   195 200 205
   Gly Ala Pro Met Ala Asp Asn Asn Glu Gly Ala Asp Gly Val Gly Asn
   210 215 220
   Ala Ser Gly Asn Trp His Cys Asp Ser Thr Trp Leu Gly Asp Arg Val
   225 230 235 240
   Ile Thr Thr Ser Thr Arg Thr Trp Ala Leu Pro Thr Tyr Asn Asn His
   245 250 255
   Leu Tyr Lys Gln Ile Ser Ser Gln Ser Ala Gly Ser Thr Asn Asp Asn
   260 265 270
   Thr Tyr Phe Gly Tyr Ser Thr Pro Trp Gly Tyr Phe Asp Phe Asn Arg
   275 280 285
   Phe His Cys His Phe Ser Pro Arg Asp Trp Gln Arg Leu Ile Asn Asn
   290 295 300
   Asn Trp Gly Phe Arg Pro Lys Lys Leu Arg Phe Lys Leu Phe Asn Ile
   305 310 315 320
   Gln Val Lys Glu Val Thr Thr Asn Asp Gly Val Thr Thr Ile Ala Asn
   325 330 335
   Asn Leu Thr Ser Thr Val Gln Val Phe Ser Asp Ser Glu Tyr Gln Leu
   340 345 350
   Pro Tyr Val Leu Gly Ser Ala His Gln Gly Cys Leu Pro Pro Phe Pro
   355 360 365
   Ala Asp Val Phe Met Ile Pro Gln Tyr Gly Tyr Leu Thr Leu Asn Asn
   370 375 380
   Gly Ser Gln Ser Val Gly Arg Ser Ser Phe Tyr Cys Leu Glu Tyr Phe
   385 390 395 400
   Pro Ser Gln Met Leu Arg Thr Gly Asn Asn Phe Glu Phe Ser Tyr Thr
   405 410 415
   Phe Glu Asp Val Pro Phe His Ser Ser Tyr Ala His Ser Gln Ser Leu
   420 425 430
   Asp Arg Leu Met Asn Pro Leu Ile Asp Gln Tyr Leu Tyr Tyr Leu Ala
   435 440 445
   Arg Thr Gln Ser Thr Thr Gly Gly Thr Ala Gly Asn Arg Glu Leu Gln
   450 455 460
   Phe Tyr Gln Ala Gly Pro Ser Thr Met Ala Glu Gln Ala Lys Asn Trp
   465 470 475 480
   Leu Pro Gly Pro Cys Tyr Arg Gln Gln Arg Val Ser Lys Thr Leu Asp
   485 490 495
   Gln Asn Asn Asn Ser Asn Phe Ala Trp Thr Gly Ala Thr Lys Tyr His
   500 505 510
   Leu Asn Gly Arg Asn Ser Leu Val Asn Pro Gly Val Ala Met Ala Thr
   515 520 525
   His Lys Asp Asp Glu Asp Arg Phe Phe Pro Ser Ser Gly Val Leu Ile
   530 535 540
   Phe Gly Lys Thr Gly Ala Ala Asn Lys Thr Thr Leu Glu Asn Val Leu
   545 550 555 560
   Met Thr Asn Glu Glu Glu Ile Lys Thr Thr Asn Pro Val Ala Thr Glu
   565 570 575
   Glu Tyr Gly Val Val Ser Ser Asn Leu Gln Ser Ala Asn Thr Ala Pro
   580 585 590
   Gln Thr Gln Thr Val Asn Ser Gln Gly Ala Leu Pro Gly Met Val Trp
   595 600 605
   Gln Asn Arg Asp Val Tyr Leu Gln Gly Pro Ile Trp Ala Lys Ile Pro
   610 615 620
   His Thr Asp Gly Asn Phe His Pro Ser Pro Leu Met Gly Gly Phe Gly
   625 630 635 640
   Leu Lys His Pro Pro Pro Gln Ile Leu Ile Lys Asn Thr Pro Val Pro
   645 650 655
   Ala Asn Pro Pro Glu Val Phe Thr Pro Ala Lys Phe Ala Ser Phe Ile
   660 665 670
   Thr Gln Tyr Ser Thr Gly Gln Val Ser Val Glu Ile Glu Trp Glu Leu
   675 680 685
   Gln Lys Glu Asn Ser Lys Arg Trp Asn Pro Glu Ile Gln Tyr Thr Ser
   690 695 700
   Asn Tyr Asp Lys Ser Thr Asn Val Asp Phe Ala Val Asp Ser Glu Gly
   705 710 715 720
   Val Tyr Ser Glu Pro Arg Pro Ile Gly Thr Arg Tyr Leu Thr Arg Asn
   725 730 735
   Leu
<210> 14
   <211> 2211
   <212> DNA
   <213> Adeno-associated virus
<220>
   <221> variation
   <222> (442)..(444)
   <223> /replace="cag"
<220>
   <221> variation
   <222> (505)..(507)
   <223> /replace="aga"
<220>
   <221> variation
   <222> (940)..(942)
   <223> /replace="aac"
<220>
   <221> variation
   <222> (1396)..(1398)
   <223> /replace="cac"
<220>
   <221> variation
   <222> (1687)..(1689)
   <223> /replace="agt"
<220>
   <221> variation
   <222> (1738)..(1740)
   <223> /replace="ata"
<220>
   <221> variation
   <222> (1762)..(1764)
   <223> /replace="tct"
<220>
   <221> misc_feature
   <222> (1)..(2211)
   <223> /note="Variation nucleotides given in the sequence have no preference with respect to those in the annotations for variation positions"
<400> 14
   atggctgccg atggttatct tccagattgg ctcgaggaca acctctctga gggcattcgc 60
   gagtggtggg acctgaaacc tggagccccg aaacccaaag ccaaccagca aaagcaggac 120
   gacggccggg gtctggtgct tcctggctac aagtacctcg gacccttcaa cggactcgac 180
   aagggggagc ccgtcaacgc ggcggacgca gcggccctcg agcacgacaa ggcctacgac 240
   cagcagctca aagcgggtga caatccgtac ctgcggtata accacgccga cgccgagttt 300
   caggagcgtc tgcaagaaga tacgtcattt gggggcaacc tcgggcgagc agtcttccag 360
   gccaagaagc gggttctcga acctctcggt ctggttgagg aaggcgctaa gacggctcct 420
   ggaaagaaga gaccggtaga gccgtcacct cagcgttccc ccgactcctc cacgggcatc 480
   ggcaagaaag gccagcagcc cgccaaaaag agactcaatt tcggtcagac tggcgactca 540
   gagtcagtcc ccgaccctca acctctcgga gaacctccag cagcgccctc tggtgtggga 600
   tctggtacaa tggctgcagg cggtggcgca ccaatggcag acaataacga aggtgccgac 660
   ggagtgggta atgcctcagg aaattggcat tgcgattcca catggctggg cgacagagtc 720
   attaccacca gcacccgaac ctgggccctg cccacctaca acaaccacct ctacaagcaa 780
   atctccagtc aaagtgcagg tagtaccaac gacaacacct acttcggcta cagcaccccc 840
   tgggggtatt ttgactttaa cagattccac tgccacttct caccacgtga ctggcagcga 900
   ctcatcaaca acaactgggg attccggccc aagaagctgc ggttcaagct cttcaacatc 960
   caggtcaagg aggtcacgac gaatgacggc gttacgacca tcgctaataa ccttaccagc 1020
   acggttcagg tattctcgga ctcggaatac cagctgccgt acgtcctcgg ctctgcgcac 1080
   cagggctgcc tgcctccgtt cccggcggac gtcttcatga ttcctcagta cggctacctg 1140
   actctcaaca atggcagtca gtctgtggga cgttcctcct tctactgcct ggagtacttc 1200
   ccctctcaga tgctgagaac gggcaacaac tttgagttca gctacacctt cgaggacgtg 1260
   cctttccaca gcagctacgc acacagccag agcctggacc ggctgatgaa tcccctcatc 1320
   gaccagtact tgtactacct ggccagaaca cagagtacca caggaggcac agctggcaat 1380
   cgggaactgc agttttacca ggccgggcct tcaactatgg ccgaacaagc caagaattgg 1440
   ttacctggac cttgctaccg gcaacaaaga gtctccaaaa cgctggatca aaacaacaac 1500
   agcaactttg cttggactgg tgccaccaaa tatcacctga acggcagaaa ctcgttggtt 1560
   aatcccggcg tcgccatggc aactcacaag gacgacgagg accgcttttt cccatccagc 1620
   ggagtcctga tttttggaaa aactggagca gctaacaaaa ctacattgga aaatgtgtta 1680
   atgacaaatg aagaagaaat taaaactact aatcctgtag ccacggaaga atacggggta 1740
   gtcagcagca acttacaatc ggctaatact gcaccccaga cacaaactgt caacagccag 1800
   ggagccttac ctggcatggt ctggcagaac cgggacgtgt acctgcaggg tcccatctgg 1860
   gccaagattc ctcacacgga tggcaacttt cacccgtctc ctttgatggg cggctttgga 1920
   cttaaacatc cgcctcctca gatcctgatc aagaacactc ccgttcccgc taatcctccg 1980
   gaggtgttta ctcctgccaa gtttgcttcg ttcatcacac agtacagcac cggacaagtc 2040
   agcgtggaaa tcgagtggga gctgcagaag gaaaacagca agcgctggaa cccggagatt 2100
   cagtacacct ccaactatga taagtcgact aatgtggact ttgccgttga cagcgagggt 2160
   gtttactctg agcctcgccc tattggcact cgttacctca cccgtaatct g 2211
<210> 15
   <211> 735
   <212> PRT
   <213> Adeno-associated virus
<220>
   <221> VARIANT
   <222> (162)..(162)
   <223> /replace="Thr"
<220>
   <221> VARIANT
   <222> (168)..(168)
   <223> /replace="Arg"
<220>
   <221> VARIANT
   <222> (224)..(224)
   <223> /replace="Ser"
<220>
   <221> VARIANT
   <222> (310)..(310)
   <223> /replace="Lys"
<220>
   <221> VARIANT
   <222> (410)..(410)
   <223> /replace="Gln"
<220>
   <221> VARIANT
   <222> (446)..(446)
   <223> /replace="Asn"
<220>
   <221> VARIANT
   <222> (461)..(461)
   <223> /replace="Leu"
<220>
   <221> VARIANT
   <222> (471)..(471)
   <223> /replace="Ser"
<220>
   <221> VARIANT
   <222> (708)..(708)
   <223> /replace="Thr"
<220>
   <221> misc_feature
   <222> (1)..(735)
   <223> /note="Variant residues given in the sequence have no preference with respect to those in the annotations for variant positions"
<400> 15
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Ser Gly Gln Gln Pro Ala Lys Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ser Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Ala Ser Asn Asp Asn His Tyr
               260                 265                 270
   Phe Gly Tyr Ser Thr Pro Trp Gly Tyr Phe Asp Phe Asn Arg Phe His
           275                 280                 285
   Cys His Phe Ser Pro Arg Asp Trp Gln Arg Leu Ile Asn Asn Asn Trp
       290                 295                 300
   Gly Phe Arg Pro Lys Arg Leu Asn Phe Lys Leu Phe Asn Ile Gln Val
   305                 310                 315                 320
   Lys Glu Val Thr Thr Asn Asp Gly Thr Thr Thr Ile Ala Asn Asn Leu
                   325                 330                 335
   Thr Ser Thr Val Gln Val Phe Thr Asp Ser Glu Tyr Gln Leu Pro Tyr
               340                 345                 350
   Val Leu Gly Ser Ala His Gln Gly Cys Leu Pro Pro Phe Pro Ala Asp
           355                 360                 365
   Val Phe Met Ile Pro Gln Tyr Gly Tyr Leu Thr Leu Asn Asn Gly Ser
       370                 375                 380
   Gln Ala Val Gly Arg Ser Ser Phe Tyr Cys Leu Glu Tyr Phe Pro Ser
   385                 390                 395                 400
   Gln Met Leu Arg Thr Gly Asn Asn Phe Thr Phe Ser Tyr Thr Phe Glu
                   405                 410                 415
   Asp Val Pro Phe His Ser Ser Tyr Ala His Ser Gln Ser Leu Asp Arg
               420                 425                 430
   Leu Met Asn Pro Leu Ile Asp Gln Tyr Leu Tyr Tyr Leu Ser Arg Thr
           435                 440                 445
   Gln Thr Thr Ser Gly Thr Ala Gln Asn Arg Glu Leu Gln Phe Ser Gln
       450                 455                 460
   Ala Gly Pro Ser Ser Met Ala Asn Gln Ala Lys Asn Trp Leu Pro Gly
   465                 470                 475                 480
   Pro Cys Tyr Arg Gln Gln Arg Val Ser Lys Thr Ala Asn Asp Asn Asn
                   485                 490                 495
   Asn Ser Asn Phe Ala Trp Thr Gly Ala Thr Lys Tyr His Leu Asn Gly
               500                 505                 510
   Arg Asp Ser Leu Val Asn Pro Gly Pro Ala Met Ala Ser His Lys Asp
           515                 520                 525
   Asp Glu Asp Lys Phe Phe Pro Met Ser Gly Val Leu Ile Phe Gly Lys
       530                 535                 540
   Gln Gly Ala Gly Ala Ser Asn Val Asp Leu Asp Asn Val Met Ile Thr
   545                 550                 555                 560
   Asp Glu Glu Glu Ile Lys Thr Thr Asn Pro Val Ala Thr Glu Gln Tyr
                   565                 570                 575
   Gly Thr Val Ala Thr Asn Leu Gln Ser Ser Asn Thr Ala Pro Ala Thr
               580                 585                 590
   Gly Thr Val Asn Ser Gln Gly Ala Leu Pro Gly Met Val Trp Gln Asp
           595                 600                 605
   Arg Asp Val Tyr Leu Gln Gly Pro Ile Trp Ala Lys Ile Pro His Thr
       610                 615                 620
   Asp Gly His Phe His Pro Ser Pro Leu Met Gly Gly Phe Gly Leu Lys
   625                 630                 635                 640
   His Pro Pro Pro Gln Ile Leu Ile Lys Asn Thr Pro Val Pro Ala Asn
                   645                 650                 655
   Pro Pro Thr Thr Phe Ser Pro Ala Lys Phe Ala Ser Phe Ile Thr Gln
               660                 665                 670
   Tyr Ser Thr Gly Gln Val Ser Val Glu Ile Glu Trp Glu Leu Gln Lys
           675                 680                 685
   Glu Asn Ser Lys Arg Trp Asn Pro Glu Ile Gln Tyr Thr Ser Asn Tyr
   690 695 700
   Asn Lys Ser Ala Asn Val Asp Phe Thr Val Asp Thr Asn Gly Val Tyr
   705 710 715 720
   Ser Glu Pro Arg Pro Ile Gly Thr Arg Tyr Leu Thr Arg Asn Leu
   725 730 735
<210> 16
   <211> 2205
   <212> DNA
   <213> Adeno-associated virus
<220>
   <221> variation
   <222> (484)..(486)
   <223> /replace="aca"
<220>
   <221> variation
   <222> (502)..(504)
   <223> /replace="aga"
<220>
   <221> variation
   <222> (670)..(672)
   <223> /replace="tcc"
<220>
   <221> variation
   <222> (928)..(930)
   <223> /replace="aaa"
<220>
   <221> variation
   <222> (1228)..(1230)
   <223> /replace="cag"
<220>
   <221> variation
   <222> (1336)..(1338)
   <223> /replace="aac"
<220>
   <221> variation
   <222> (1381)..(1383)
   <223> /replace="ctg"
<220>
   <221> variation
   <222> (1411) .. (1413)
   <223> /replace="tct"
<220>
   <221> variation
   <222> (2122) .. (2124)
   <223> /replace="acc"
<220>
   <221> misc_feature
   <222> (1)..(2205)
   <223> /note="Variation nucleotides given in the sequence have no preference with respect to those in the annotations for variation positions"
<400> 16
   atggctgccg atggttatct tccagattgg ctcgaggaca acctctctga gggcattcgc      60
   gagtggtggg acttgaaacc tggagccccg aaacccaaag ccaaccagca aaagcaggac     120
   gacggccggg gtctggtgct tcctggctac aagtacctcg gacccttcaa cggactcgac     180
   aagggggagc ccgtcaacgc ggcggatgca gcggccctcg agcacgacaa ggcctacgac     240
   cagcagctca aagcgggtga caatccgtac ctgcggtata accacgccga cgccgagttt     300
   caggagcgtc tgcaagaaga tacgtctttt gggggcaacc tcgggcgagc agtcttccag     360
   gccaagaaga gggttctcga acctcttggt ctggttgagg aaggtgctaa gacggctcct     420
   ggaaagaaac gtccggtaga gcagtcgcca caagagccag actcctcctc gggcattggc     480
   aagtcaggcc agcagcccgc taaaaagaga ctcaattttg gtcagactgg cgactcagag     540
   tcagtccccg acccacaacc tctcggagaa cctccagcag ccccctctgg tgtgggatct     600
   aatacaatgg cttcaggcgg tggcgcacca atggcagaca ataacgaagg cgccgacgga     660
   gtgggtaatg cctcaggaaa ttggcattgc gattccacat ggctgggcga cagagtcatc     720
   accaccagca cccgaacatg ggccttgccc acctataaca accacctcta caagcaaatc     780
   tccagtcaat caggggccag caacgacaac cactacttcg gctacagcac cccctggggg     840
   tattttgatt tcaacagatt ccactgccat ttctcaccac gtgactggca gcgactcatc     900
   aacaacaatt ggggattccg gcccaagaga ctcaacttca agctcttcaa catccaagtc     960
   aaggaggtca cgacgaatga tggcaccacg accatcgcta ataaccttac cagcacggtt    1020
   caagtcttca cggactcgga gtaccagttg ccgtacgtcc tcggctctgc gcaccagggc    1080
   tgcctccctc cgttcccggc ggacgtgttc atgattccgc agtacggcta cctaacgctc    1140
   aacaatggca gccaggcagt gggacggtca tccttttact gcctggaata tttcccatcg    1200
   cagatgctga gaacgggcaa taactttacc ttcagctaca ccttcgagga cgtgcctttc    1260
   cacagcagct acgcgcacag ccagagcctg gaccggctga tgaatcctct catcgaccag    1320
   tacctgtatt acctgagcag aactcagact acgtccggaa ctgcccaaaa cagggagttg    1380
   cagtttagcc aggcgggtcc atctagcatg gctaatcagg ccaaaaactg gctacctgga    1440
   ccctgttacc ggcagcagcg cgtttctaaa acagcaaatg acaacaacaa cagcaacttt    1500
   gcctggactg gtgctacaaa atatcacctt aatgggcgtg attctttagt caaccctggc    1560
   cctgctatgg cctcacacaa agacgacgaa gacaagttct ttcccatgag cggtgtcttg    1620
   atttttggaa agcagggcgc cggagcttca aacgttgatt tggacaatgt catgatcaca 1680
   gacgaagagg aaatcaaaac cactaacccc gtggccaccg aacaatatgg gactgtggca 1740
   accaatctcc agagcagcaa cacagcccct gcgaccggaa ctgtgaattc tcagggagcc 1800
   ttacctggaa tggtgtggca agacagagac gtatacctgc agggtcctat ttgggccaaa 1860
   attcctcaca cggatggaca ctttcacccg tctcctctca tgggcggctt tggacttaag 1920
   cacccgcctc ctcagatcct catcaaaaac acgcctgttc ctgcgaatcc tccgacaacg 1980
   ttttcgcctg caaagtttgc ttcattcatc acccagtatt ccacaggaca agtgagcgtg 2040
   gagattgaat gggagctgca gaaagaaaac agcaaacgct ggaatcccga aatacagtat 2100
   acatctaact ataataaatc tgccaacgtt gatttcactg tggacaccaa tggagtttat 2160
   agtgagcctc gccccattgg cacccgttac ctcacccgta acctg 2205
<210> 17
   <211> 735
   <212> PRT
   <213> Adeno-associated virus
<220>
   <221> VARIANT
   <222> (42) .. (42)
   <223> /replace="Ser"
<220>
   <221> VARIANT
   <222> (168)..(168)
   <223> /replace="Lys"
<220>
   <221> VARIANT
   <222> (310) .. (310)
   <223> /replace="Arg"
<220>
   <221> VARIANT
   <222> (410)..(410)
   <223> /replace="Gln"
<220>
   <221> VARIANT
   <222> (446)..(446)
   <223> /replace="Arg"
<220>
   <221> VARIANT
   <222> (461)..(461)
   <223> /replace="Leu"
<220>
   <221> VARIANT
   <222> (471)..(471)
   <223> /replace="Ser"
<220>
   <221> VARIANT
   <222> (475)..(475)
   <223> /replace="Arg"
<220>
   <221> VARIANT
   <222> (504) .. (504)
   <223> /replace="Ala"
<220>
   <221> VARIANT
   <222> (539)..(539)
   <223> /replace="Asn"
<220>
   <221> misc_feature
   <222> (1)..(735)
   <223> /note="Variant residues given in the sequence have no preference with respect to those in the annotations for variant positions"
<400> 17
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Gln Pro
               20                  25                  30
   Lys Ala Asn Gln Gln His Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Glu Ala Asp Ala Ala Ala Leu Glu His Asp Lys Ala Tyr Asp
   65                  70                  75                  80
   Gln Gln Leu Lys Ala Gly Asp Asn Pro Tyr Leu Lys Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Ala Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Ser Gly Gln Gln Pro Ala Arg Lys Arg Leu Asn Phe Gly Gln Thr
   165 170 175
   Gly Asp Ser Glu Ser Val Pro Asp Pro Gln Pro Leu Gly Glu Pro Pro
   180 185 190
   Ala Ala Pro Ser Gly Val Gly Ser Asn Thr Met Ala Ser Gly Gly Gly
   195 200 205
   Ala Pro Met Ala Asp Asn Asn Glu Gly Ala Asp Gly Val Gly Asn Ser
   210 215 220
   Ser Gly Asn Trp His Cys Asp Ser Thr Trp Leu Gly Asp Arg Val Ile
   225 230 235 240
   Thr Thr Ser Thr Arg Thr Trp Ala Leu Pro Thr Tyr Asn Asn His Leu
   245 250 255
   Tyr Lys Gln Ile Ser Ser Gln Ser Gly Ala Ser Asn Asp Asn His Tyr
   260 265 270
   Phe Gly Tyr Ser Thr Pro Trp Gly Tyr Phe Asp Phe Asn Arg Phe His
   275 280 285
   Cys His Phe Ser Pro Arg Asp Trp Gln Arg Leu Ile Asn Asn Asn Trp
   290 295 300
   Gly Phe Arg Pro Lys Lys Leu Asn Phe Lys Leu Phe Asn Ile Gln Val
   305 310 315 320
   Lys Glu Val Thr Gln Asn Asp Gly Thr Thr Thr Ile Ala Asn Asn Leu
   325 330 335
   Thr Ser Thr Val Gln Val Phe Thr Asp Ser Glu Tyr Gln Leu Pro Tyr
   340 345 350
   Val Leu Gly Ser Ala His Gln Gly Cys Leu Pro Pro Phe Pro Ala Asp
   355 360 365
   Val Phe Met Ile Pro Gln Tyr Gly Tyr Leu Thr Leu Asn Asn Gly Ser
   370 375 380
   Gln Ala Val Gly Arg Ser Ser Phe Tyr Cys Leu Glu Tyr Phe Pro Ser
   385 390 395 400
   Gln Met Leu Arg Thr Gly Asn Asn Phe Thr Phe Ser Tyr Thr Phe Glu
   405 410 415
   Asp Val Pro Phe His Ser Ser Tyr Ala His Ser Gln Ser Leu Asp Arg
   420 425 430
   Leu Met Asn Pro Leu Ile Asp Gln Tyr Leu Tyr Tyr Leu Ser Arg Thr
   435 440 445
   Gln Thr Thr Ser Gly Thr Thr Gln Gln Ser Arg Leu Gln Phe Ser Gln
   450 455 460
   Ala Gly Pro Ser Ser Met Ala Gln Gln Ala Lys Asn Trp Leu Pro Gly
   465 470 475 480
   Pro Cys Tyr Arg Gln Gln Arg Val Ser Lys Thr Ala Asn Asp Asn Asn
   485 490 495
   Asn Ser Asn Phe Ala Trp Thr Gly Ala Thr Lys Tyr His Leu Asn Gly
   500 505 510
   Arg Asp Ser Leu Val Asn Pro Gly Pro Ala Met Ala Ser His Lys Asp
   515 520 525
   Asp Glu Glu Lys Phe Phe Pro Met His Gly Val Leu Ile Phe Gly Lys
   530 535 540
   Gln Gly Thr Gly Ala Ser Asn Val Asp Leu Asp Asn Val Met Ile Thr
   545 550 555 560
   Asp Glu Glu Glu Ile Arg Thr Thr Asn Pro Val Ala Thr Glu Gln Tyr
   565 570 575
   Gly Thr Val Ala Thr Asn Leu Gln Ser Ser Asn Thr Ala Pro Ala Thr
   580 585 590
   Gly Thr Val Asn Ser Gln Gly Ala Leu Pro Gly Met Val Trp Gln Asp
   595 600 605
   Arg Asp Val Tyr Leu Gln Gly Pro Ile Trp Ala Lys Ile Pro His Thr
   610 615 620
   Asp Gly His Phe His Pro Ser Pro Leu Met Gly Gly Phe Gly Leu Lys
   625 630 635 640
   His Pro Pro Pro Gln Ile Leu Ile Lys Asn Thr Pro Val Pro Ala Asn
   645 650 655
   Pro Pro Thr Thr Phe Ser Pro Ala Lys Phe Ala Ser Phe Ile Thr Gln
   660 665 670
   Tyr Ser Thr Gly Gln Val Ser Val Glu Ile Glu Trp Glu Leu Gln Lys
   675 680 685
   Glu Asn Ser Lys Arg Trp Asn Pro Glu Ile Gln Tyr Thr Ser Asn Tyr
   690 695 700
   Asn Lys Ser Val Asn Val Asp Phe Thr Val Asp Thr Asn Gly Val Tyr
   705 710 715 720
   Ser Glu Pro Arg Pro Ile Gly Thr Arg Tyr Leu Thr Arg Asn Leu
   725 730 735
<210> 18
   <211> 2205
   <212> DNA
   <213> Adeno-associated virus
<220>
   <221> variation
   <222> (124)..(126)
   <223> /replace="agt"
<220>
   <221> variation
   <222> (502)..(504)
   <223> /replace="aaa"
<220>
   <221> variation
   <222> (928)..(930)
   <223> /replace="aga"
<220>
   <221> variation
   <222> (1228) .. (1230)
   <223> /replace="cag"
<220>
   <221> variation
   <222> (1336) .. (1338)
   <223> /replace="aga"
<220>
   <221> variation
   <222> (1381)..(1383)
   <223> /replace="ctc"
<220>
   <221> variation
   <222> (1411) .. (1413)
   <223> /replace="tct"
<220>
   <221> variation
   <222> (1423)..(1425)
   <223> /replace="aga"
<220>
   <221> variation
   <222> (1510) .. (1512)
   <223> /replace="gcg"
<220>
   <221> variation
   <222> (1615)..(1617)
   <223> /replace="gac"
<220>
   <221> misc_feature
   <222> (1)..(2205)
   <223> /note="Variation nucleotides given in the sequence have no preference with respect to those in the annotations for variation positions"
<400> 18
   atggctgctg acggttatct tccagattgg ctcgaggaca acctttctga aggcattcgt 60
   gagtggtggg atctgaaacc tggagcccct caacccaaag cgaaccaaca acaccaggac 120
   gacggtcggg gtcttgtgct tccgggttac aaatacctcg gaccctttaa cggactcgac 180
   aaaggagagc cggtcaacga ggcggacgcg gcagccctcg aacacgacaa agcttacgac 240
   cagcagctca aggccggtga caacccgtac ctcaagtaca accacgccga cgccgagttt 300
   caggagcgtc ttcaagaaga tacgtctttt gggggcaacc ttggcagagc agtcttccag 360
   gccaaaaaga gggtccttga gcctcttggt ctggttgagg aagcagctaa aacggctcct 420
   ggaaagaaga ggcctgtaga acagtctcct caggaaccgg actcatcatc tggtattggc 480
   aaatcgggcc aacagcctgc cagaaaaaga ctaaatttcg gtcagactgg agactcagag 540
   tcagtcccag accctcaacc tctcggagaa ccaccagcag ccccctcagg tgtgggatct 600
   aatacaatgg cttcaggcgg tggcgcacca atggcagaca ataacgaggg tgccgatgga 660
   gtgggtaatt cctcaggaaa ttggcattgc gattccacat ggctgggcga cagagtcatc 720
   accaccagca ccagaacctg ggccctgccc acttacaaca accatctcta caagcaaatc 780
   tccagccaat caggagcttc aaacgacaac cactactttg gctacagcac cccttggggg 840
   tattttgact ttaacagatt ccactgccac ttctcaccac gtgactggca gcgactcatt 900
   aacaacaact ggggattccg gcccaagaaa ctcaacttca agctcttcaa catccaagtt 960
   aaagaggtca cgcagaacga tggcacgacg actattgcca ataaccttac cagcacggtt 1020
   caagtgttta cggactcgga gtatcagctc ccgtacgtgc tcgggtcggc gcaccaaggc 1080
   tgtctcccgc cgtttccagc ggacgtcttc atgatccctc agtatggata cctcaccctg 1140
   aacaacggaa gtcaagcggt gggacgctca tccttttact gcctggagta cttcccttcg 1200
   cagatgctaa ggactggaaa taacttcaca ttcagctata ccttcgagga tgtacctttt 1260
   cacagcagct acgctcacag ccagagtttg gatcgcttga tgaatcctct tattgatcag 1320
   tatctgtact acctgagcag aacgcaaaca acctctggaa caacccaaca atcacggctg     1380
   caatttagcc aggctgggcc ttcgtctatg gctcagcagg ccaaaaattg gctacctggg     1440
   ccctgctacc ggcaacagag agtttcaaag actgctaacg acaacaacaa cagtaacttt     1500
   gcttggacag gggccaccaa atatcatctc aatggccgcg actcgctggt gaatccagga     1560
   ccagctatgg ccagtcacaa ggacgatgaa gaaaaatttt tccctatgca cggcgttcta     1620
   atatttggca aacaagggac aggggcaagt aacgtagatt tagataatgt aatgattacg     1680
   gatgaagaag agattcgtac caccaatcct gtggcaacag agcagtatgg aactgtggca     1740
   actaacttgc agagctcaaa tacagctccc gcgactggaa ctgtcaatag tcagggggcc    1800
   ttacctggca tggtgtggca agatcgtgac gtgtaccttc aaggacctat ctgggcaaag     1860
   attcctcaca cggatggaca ctttcatcct tctcctctga tgggaggctt tggactgaaa     1920
   catccgcctc ctcaaatctt gatcaaaaat actccggtac cggcaaatcc tccgacgact     1980
   ttcagcccgg ccaagtttgc ttcatttatc actcagtact ccactggaca ggtcagcgtg     2040
   gaaattgagt gggagctaca gaaagaaaac agcaaacgtt ggaatccaga gattcagtac     2100
   acttccaact acaacaagtc tgttaatgtg gactttactg tagacactaa tggtgtttat     2160
   agtgaacctc gccctattgg aacccggtat ctcacacgaa acttg                    2205
<210> 19
   <211> 736
   <212> PRT
   <213> Adeno-associated virus
<400> 19
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Arg Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ala Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Gly Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Arg Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
               340                 345                 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Glu Phe Ser Tyr Thr Phe
   405 410 415
   Glu Asp Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Ser Arg
   435 440 445
   Thr Gln Thr Thr Ser Gly Thr Ala Gly Asn Arg Thr Leu Gln Phe Ser
   450 455 460
   Gln Ala Gly Pro Ser Ser Met Ala Asn Gln Ala Lys Asn Trp Leu Pro
   465 470 475 480
   Gly Pro Cys Tyr Arg Gln Gln Arg Val Ser Lys Thr Ala Asn Gln Asn
   485 490 495
   Asn Asn Ser Asn Phe Ala Trp Thr Gly Ala Thr Lys Tyr His Leu Asn
   500 505 510
   Gly Arg Asp Ser Leu Val Asn Pro Gly Pro Ala Met Ala Thr His Lys
   515 520 525
   Asp Asp Glu Asp Lys Phe Phe Pro Met Ser Gly Val Leu Ile Phe Gly
   530 535 540
   Lys Gln Gly Ala Gly Asn Ser Asn Val Asp Leu Asp Asn Val Met Ile
   545 550 555 560
   Thr Asn Glu Glu Glu Ile Lys Thr Thr Asn Pro Val Ala Thr Glu Gln
   565 570 575
   Tyr Gly Thr Val Ala Thr Asn Leu Gln Ser Ala Asn Thr Ala Pro Ala
   580 585 590
   Thr Gly Thr Val Asn Ser Gln Gly Ala Leu Pro Gly Met Val Trp Gln
   595 600 605
   Asp Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 20
   <211> 736
   <212> PRT
   <213> Adeno-associated virus
<400> 20
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Lys Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ser Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Ala Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Arg Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
               340                 345                 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Gln Phe Ser Tyr Thr Phe
   405 410 415
   Glu Asp Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Ser Arg
   435 440 445
   Thr Gln Thr Thr Ser Gly Thr Ala Gly Asn Arg Glu Leu Gln Phe Ser
   450 455 460
   Gln Ala Gly Pro Ser Ser Met Ala Asn Gln Ala Lys Asn Trp Leu Pro
   465 470 475 480
   Gly Pro Cys Tyr Arg Gln Gln Arg Val Ser Lys Thr Thr Asn Gln Asn
   485 490 495
   Asn Asn Ser Asn Phe Ala Trp Thr Gly Ala Thr Lys Tyr His Leu Asn
   500 505 510
   Gly Arg Asp Ser Leu Val Asn Pro Gly Pro Ala Met Ala Thr His Lys
   515 520 525
   Asp Asp Glu Asp Lys Phe Phe Pro Met Ser Gly Val Leu Ile Phe Gly
   530 535 540
   Lys Gln Gly Ala Gly Asn Ser Asn Val Asp Leu Asp Asn Val Met Ile
   545 550 555 560
   Thr Asn Glu Glu Glu Ile Lys Thr Thr Asn Pro Val Ala Thr Glu Glu
   565 570 575
   Tyr Gly Thr Val Ala Thr Asn Leu Gln Ser Ala Asn Thr Ala Pro Ala
   580 585 590
   Thr Gly Thr Val Asn Ser Gln Gly Ala Leu Pro Gly Met Val Trp Gln
   595 600 605
   Asn Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 21
   <211> 736
   <212> PRT
   <213> Adeno-associated virus
<400> 21
   Met Ala Ala Asp Gly Tyr Leu Pro Asp Trp Leu Glu Asp Asn Leu Ser
   1 5 10 15
   Glu Gly Ile Arg Glu Trp Trp Asp Leu Lys Pro Gly Ala Pro Lys Pro
   20 25 30
   Lys Ala Asn Gln Gln Lys Gln Asp Asp Gly Arg Gly Leu Val Leu Pro
   35 40 45
   Gly Tyr Lys Tyr Leu Gly Pro Phe Asn Gly Leu Asp Lys Gly Glu Pro
   50 55 60
   Val Asn Ala Ala Asp Ala Ala Ala Leu Glu His Asp Lys Ala Tyr Asp
   65 70 75 80
   Gln Gln Leu Lys Ala Gly Asp Asn Pro Tyr Leu Arg Tyr Asn His Ala
   85 90 95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Arg Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ala Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Gly Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Arg Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
               340                 345                 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Glu Phe Ser Tyr Thr Phe
   405 410 415
   Glu Asp Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Ser Arg
   435 440 445
   Thr Gln Thr Thr Ser Gly Thr Ala Gly Asn Arg Glu Leu Gln Phe Ser
   450 455 460
   Gln Ala Gly Pro Ser Ser Met Ala Asn Gln Ala Lys Asn Trp Leu Pro
   465 470 475 480
   Gly Pro Cys Tyr Arg Gln Gln Arg Val Ser Lys Thr Thr Asn Gln Asn
   485 490 495
   Asn Asn Ser Asn Phe Ala Trp Thr Gly Ala Thr Lys Tyr His Leu Asn
   500 505 510
   Gly Arg Asp Ser Leu Val Asn Pro Gly Pro Ala Met Ala Thr His Lys
   515 520 525
   Asp Asp Glu Asp Lys Phe Phe Pro Met Ser Gly Val Leu Ile Phe Gly
   530 535 540
   Lys Gln Gly Ala Gly Asn Ser Asn Val Asp Leu Asp Asn Val Met Ile
   545 550 555 560
   Thr Ser Glu Glu Glu Ile Lys Thr Thr Asn Pro Val Ala Thr Glu Glu
   565 570 575
   Tyr Gly Thr Val Ala Thr Asn Leu Gln Ser Ser Asn Thr Ala Pro Ala
   580 585 590
   Thr Gly Thr Val Asn Ser Gln Gly Ala Leu Pro Gly Met Val Trp Gln
   595 600 605
   Glu Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 22
   <211> 736
   <212> PRT
   <213> Adeno-associated virus
<400> 22
   Met Ala Ala Asp Gly Tyr Leu Pro Asp Trp Leu Glu Asp Asn Leu Ser
   1 5 10 15
   Glu Gly Ile Arg Glu Trp Trp Asp Leu Lys Pro Gly Ala Pro Lys Pro
   20 25 30
   Lys Ala Asn Gln Gln Lys Gln Asp Asp Gly Arg Gly Leu Val Leu Pro
   35 40 45
   Gly Tyr Lys Tyr Leu Gly Pro Phe Asn Gly Leu Asp Lys Gly Glu Pro
   50 55 60
   Val Asn Ala Ala Asp Ala Ala Ala Leu Glu His Asp Lys Ala Tyr Asp
   65 70 75 80
   Gln Gln Leu Lys Ala Gly Asp Asn Pro Tyr Leu Arg Tyr Asn His Ala
   85 90 95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Arg Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ser Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Gly Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Lys Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
               340                 345                 350
   Tyr Val Leu Gly Ser Ala His Gln Gly Cys Leu Pro Pro Phe Pro Ala
           355                 360                 365
   Asp Val Phe Met Ile Pro Gln Tyr Gly Tyr Leu Thr Leu Asn Asn Gly
       370                 375                 380
   Ser Gln Ala Val Gly Arg Ser Ser Phe Tyr Cys Leu Glu Tyr Phe Pro
   385                 390                 395                 400
   Ser Gln Met Leu Arg Thr Gly Asn Asn Phe Glu Phe Ser Tyr Thr Phe
                   405                 410                 415
   Glu Asp Val Pro Phe His Ser Ser Tyr Ala His Ser Gln Ser Leu Asp
               420                 425                 430
   Arg Leu Met Asn Pro Leu Ile Asp Gln Tyr Leu Tyr Tyr Leu Ser Arg
           435                 440                 445
   Thr Gln Thr Thr Ser Gly Thr Ala Gly Asn Arg Glu Leu Gln Phe Ser
       450                 455                 460
   Gln Ala Gly Pro Ser Ser Met Ala Asn Gln Ala Lys Asn Trp Leu Pro
   465                 470                 475                 480
   Gly Pro Cys Tyr Arg Gln Gln Arg Val Ser Lys Thr Thr Asn Gln Asn
                   485                 490                 495
   Asn Asn Ser Asn Phe Ala Trp Thr Gly Ala Thr Lys Tyr His Leu Asn
               500                 505                 510
   Gly Arg Asp Ser Leu Val Asn Pro Gly Pro Ala Met Ala Thr His Lys
           515                 520                 525
   Asp Asp Glu Asp Lys Phe Phe Pro Met Ser Gly Val Leu Ile Phe Gly
       530                 535                 540
   Lys Gln Gly Ala Gly Asn Ser Asn Val Asp Leu Asp Asn Val Met Ile
   545                 550                 555                 560
   Thr Ser Glu Glu Glu Ile Lys Thr Thr Asn Pro Val Ala Thr Glu Glu
                   565                 570                 575
   Tyr Gly Thr Val Ala Thr Asn Leu Gln Ser Ala Asn Thr Ala Pro Ala
               580                 585                 590
   Thr Gly Thr Val Asn Ser Gln Gly Ala Leu Pro Gly Met Val Trp Gln
   595 600 605
   Asp Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 23
   <211> 736
   <212> PRT
   <213> Adeno-associated virus
<400> 23
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
   100 105 110
   Asn Leu Gly Arg Ala Val Phe Gln Ala Lys Lys Arg Val Leu Glu Pro
   115 120 125
   Leu Gly Leu Val Glu Glu Gly Ala Lys Thr Ala Pro Gly Lys Lys Arg
   130 135 140
   Pro Val Glu Gln Ser Pro Gln Glu Pro Asp Ser Ser Ser Gly Ile Gly
   145 150 155 160
   Lys Lys Gly Gln Gln Pro Ala Arg Lys Arg Leu Asn Phe Gly Gln Thr
   165 170 175
   Gly Asp Ser Glu Ser Val Pro Asp Pro Gln Pro Leu Gly Glu Pro Pro
   180 185 190
   Ala Ala Pro Ser Gly Val Gly Ser Asn Thr Met Ala Ala Gly Gly Gly
   195 200 205
   Ala Pro Met Ala Asp Asn Asn Glu Gly Ala Asp Gly Val Gly Asn Ala
   210 215 220
   Ser Gly Asn Trp His Cys Asp Ser Thr Trp Leu Gly Asp Arg Val Ile
   225 230 235 240
   Thr Thr Ser Thr Arg Thr Trp Ala Leu Pro Thr Tyr Asn Asn His Leu
   245 250 255
   Tyr Lys Gln Ile Ser Ser Gln Ser Gly Gly Ser Thr Asn Asp Asn Thr
   260 265 270
   Tyr Phe Gly Tyr Ser Thr Pro Trp Gly Tyr Phe Asp Phe Asn Arg Phe
   275 280 285
   His Cys His Phe Ser Pro Arg Asp Trp Gln Arg Leu Ile Asn Asn Asn
   290 295 300
   Trp Gly Phe Arg Pro Lys Lys Leu Asn Phe Lys Leu Phe Asn Ile Gln
   305 310 315 320
   Val Lys Glu Val Thr Thr Asn Asp Gly Thr Thr Thr Ile Ala Asn Asn
   325 330 335
   Leu Thr Ser Thr Val Gln Val Phe Thr Asp Ser Glu Tyr Gln Leu Pro
   340 345 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Gln Phe Ser Tyr Thr Phe
   405 410 415
   Glu Asp Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Ser Arg
   435 440 445
   Thr Gln Thr Thr Ser Gly Thr Ala Gly Asn Arg Thr Leu Gln Phe Ser
   450 455 460
   Gln Ala Gly Pro Ser Ser Met Ala Asn Gln Ala Lys Asn Trp Leu Pro
   465 470 475 480
   Gly Pro Cys Tyr Arg Gln Gln Arg Val Ser Lys Thr Thr Asn Gln Asn
   485 490 495
   Asn Asn Ser Asn Phe Ala Trp Thr Gly Ala Thr Lys Tyr His Leu Asn
   500 505 510
   Gly Arg Asp Ser Leu Val Asn Pro Gly Pro Ala Met Ala Thr His Lys
   515 520 525
   Asp Asp Glu Asp Lys Phe Phe Pro Met Ser Gly Val Leu Ile Phe Gly
   530 535 540
   Lys Gln Gly Ala Gly Asn Ser Asn Val Asp Leu Asp Asn Val Met Ile
   545 550 555 560
   Thr Asn Glu Glu Glu Ile Lys Thr Thr Asn Pro Val Ala Thr Glu Glu
   565 570 575
   Tyr Gly Thr Val Ala Thr Asn Leu Gln Ser Ala Asn Thr Ala Pro Ala
   580 585 590
   Thr Gly Thr Val Asn Ser Gln Gly Ala Leu Pro Gly Met Val Trp Gln
   595 600 605
   Asp Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 24
   <211> 736
   <212> PRT
   <213> Adeno-associated virus
<400> 24
   Met Ala Ala Asp Gly Tyr Leu Pro Asp Trp Leu Glu Asp Asn Leu Ser
   1               5                   10                  15
   Glu Gly Ile Arg Glu Trp Trp Asp Leu Lys Pro Gly Ala Pro Lys Pro
               20                  25                  30
   Lys Ala Asn Gln Gln Lys Gln Asp Asp Gly Arg Gly Leu Val Leu Pro
           35                  40                  45
   Gly Tyr Lys Tyr Leu Gly Pro Phe Asn Gly Leu Asp Lys Gly Glu Pro
       50                  55                  60
   Val Asn Ala Ala Asp Ala Ala Ala Leu Glu His Asp Lys Ala Tyr Asp
   65                  70                  75                  80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Lys Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ala Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Gly Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Lys Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
               340                 345                 350
   Tyr Val Leu Gly Ser Ala His Gln Gly Cys Leu Pro Pro Phe Pro Ala
           355                 360                 365
   Asp Val Phe Met Ile Pro Gln Tyr Gly Tyr Leu Thr Leu Asn Asn Gly
       370                 375                 380
   Ser Gln Ala Val Gly Arg Ser Ser Phe Tyr Cys Leu Glu Tyr Phe Pro
   385                 390                 395                 400
   Ser Gln Met Leu Arg Thr Gly Asn Asn Phe Glu Phe Ser Tyr Thr Phe
                   405                 410                 415
   Glu Asp Val Pro Phe His Ser Ser Tyr Ala His Ser Gln Ser Leu Asp
               420                 425                 430
   Arg Leu Met Asn Pro Leu Ile Asp Gln Tyr Leu Tyr Tyr Leu Ser Arg
           435                 440                 445
   Thr Gln Thr Thr Ser Gly Thr Ala Gly Asn Arg Thr Leu Gln Phe Ser
       450                 455                 460
   Gln Ala Gly Pro Ser Ser Met Ala Asn Gln Ala Lys Asn Trp Leu Pro
   465                 470                 475                 480
   Gly Pro Cys Tyr Arg Gln Gln Arg Val Ser Lys Thr Ala Asn Gln Asn
                   485                 490                 495
   Asn Asn Ser Asn Phe Ala Trp Thr Gly Ala Thr Lys Tyr His Leu Asn
               500                 505                 510
   Gly Arg Asp Ser Leu Val Asn Pro Gly Pro Ala Met Ala Thr His Lys
           515                 520                 525
   Asp Asp Glu Asp Lys Phe Phe Pro Met Ser Gly Val Leu Ile Phe Gly
       530                 535                 540
   Lys Gln Gly Ala Gly Asn Ser Asn Val Asp Leu Asp Asn Val Met Ile
   545                 550                 555                 560
   Thr Ser Glu Glu Glu Ile Lys Thr Thr Asn Pro Val Ala Thr Glu Gln
                   565                 570                 575
   Tyr Gly Thr Val Ala Thr Asn Leu Gln Ser Ser Asn Thr Ala Pro Ala
               580                 585                 590
   Thr Gly Thr Val Asn Ser Gln Gly Ala Leu Pro Gly Met Val Trp Gln
   595 600 605
   Asn Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 25
   <211> 736
   <212> PRT
   <213> Adeno-associated virus
<400> 25
   Met Ala Ala Asp Gly Tyr Leu Pro Asp Trp Leu Glu Asp Asn Leu Ser
   1 5 10 15
   Glu Gly Ile Arg Glu Trp Trp Asp Leu Lys Pro Gly Ala Pro Lys Pro
   20 25 30
   Lys Ala Asn Gln Gln Lys Gln Asp Asp Gly Arg Gly Leu Val Leu Pro
   35 40 45
   Gly Tyr Lys Tyr Leu Gly Pro Phe Asn Gly Leu Asp Lys Gly Glu Pro
   50 55 60
   Val Asn Ala Ala Asp Ala Ala Ala Leu Glu His Asp Lys Ala Tyr Asp
   65 70 75 80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Lys Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ser Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Gly Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Lys Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
   340 345 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Glu Phe Ser Tyr Thr Phe
   405 410 415
   Glu Asp Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Ser Arg
   435 440 445
   Thr Gln Thr Thr Ser Gly Thr Ala Gly Asn Arg Thr Leu Gln Phe Ser
   450 455 460
   Gln Ala Gly Pro Ser Ser Met Ala Asn Gln Ala Lys Asn Trp Leu Pro
   465 470 475 480
   Gly Pro Cys Tyr Arg Gln Gln Arg Val Ser Lys Thr Ala Asn Gln Asn
   485 490 495
   Asn Asn Ser Asn Phe Ala Trp Thr Gly Ala Thr Lys Tyr His Leu Asn
   500 505 510
   Gly Arg Asp Ser Leu Val Asn Pro Gly Pro Ala Met Ala Thr His Lys
   515 520 525
   Asp Asp Glu Asp Lys Phe Phe Pro Met Ser Gly Val Leu Ile Phe Gly
   530 535 540
   Lys Gln Gly Ala Gly Asn Ser Asn Val Asp Leu Asp Asn Val Met Ile
   545 550 555 560
   Thr Ser Glu Glu Glu Ile Lys Thr Thr Asn Pro Val Ala Thr Glu Glu
   565 570 575
   Tyr Gly Thr Val Ala Thr Asn Leu Gln Ser Ser Asn Thr Ala Pro Ala
   580 585 590
   Thr Gly Thr Val Asn Ser Gln Gly Ala Leu Pro Gly Met Val Trp Gln
   595 600 605
   Asn Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 26
   <211> 736
   <212> PRT
   <213> Adeno-associated virus
<400> 26
   Met Ala Ala Asp Gly Tyr Leu Pro Asp Trp Leu Glu Asp Asn Leu Ser
   1 5 10 15
   Glu Gly Ile Arg Glu Trp Trp Asp Leu Lys Pro Gly Ala Pro Lys Pro
   20 25 30
   Lys Ala Asn Gln Gln Lys Gln Asp Asp Gly Arg Gly Leu Val Leu Pro
   35 40 45
   Gly Tyr Lys Tyr Leu Gly Pro Phe Asn Gly Leu Asp Lys Gly Glu Pro
   50 55 60
   Val Asn Ala Ala Asp Ala Ala Ala Leu Glu His Asp Lys Ala Tyr Asp
   65 70 75 80
   Gln Gln Leu Lys Ala Gly Asp Asn Pro Tyr Leu Arg Tyr Asn His Ala
                   85                  90                  95
   Asp Ala Glu Phe Gln Glu Arg Leu Gln Glu Asp Thr Ser Phe Gly Gly
               100                 105                 110
   Asn Leu Gly Arg Ala Val Phe Gln Ala Lys Lys Arg Val Leu Glu Pro
           115                 120                 125
   Leu Gly Leu Val Glu Glu Gly Ala Lys Thr Ala Pro Gly Lys Lys Arg
       130                 135                 140
   Pro Val Glu Gln Ser Pro Gln Glu Pro Asp Ser Ser Ser Gly Ile Gly
   145                 150                 155                 160
   Lys Lys Gly Gln Gln Pro Ala Lys Lys Arg Leu Asn Phe Gly Gln Thr
                   165                 170                 175
   Gly Asp Ser Glu Ser Val Pro Asp Pro Gln Pro Leu Gly Glu Pro Pro
               180                 185                 190
   Ala Ala Pro Ser Gly Val Gly Ser Asn Thr Met Ala Ser Gly Gly Gly
           195                 200                 205
   Ala Pro Met Ala Asp Asn Asn Glu Gly Ala Asp Gly Val Gly Asn Ala
       210                 215                 220
   Ser Gly Asn Trp His Cys Asp Ser Thr Trp Leu Gly Asp Arg Val Ile
   225                 230                 235                 240
   Thr Thr Ser Thr Arg Thr Trp Ala Leu Pro Thr Tyr Asn Asn His Leu
                   245                 250                 255
   Tyr Lys Gln Ile Ser Ser Gln Ser Gly Gly Ser Thr Asn Asp Asn Thr
               260                 265                 270
   Tyr Phe Gly Tyr Ser Thr Pro Trp Gly Tyr Phe Asp Phe Asn Arg Phe
           275                 280                 285
   His Cys His Phe Ser Pro Arg Asp Trp Gln Arg Leu Ile Asn Asn Asn
       290                 295                 300
   Trp Gly Phe Arg Pro Lys Lys Leu Asn Phe Lys Leu Phe Asn Ile Gln
   305                 310                 315                 320
   Val Lys Glu Val Thr Thr Asn Asp Gly Thr Thr Thr Ile Ala Asn Asn
                   325                 330                 335
   Leu Thr Ser Thr Val Gln Val Phe Thr Asp Ser Glu Tyr Gln Leu Pro
   340 345 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Gln Phe Ser Tyr Thr Phe
   405 410 415
   Glu Asp Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Ser Arg
   435 440 445
   Thr Gln Thr Thr Ser Gly Thr Ala Gly Asn Arg Glu Leu Gln Phe Ser
   450 455 460
   Gln Ala Gly Pro Ser Ser Met Ala Asn Gln Ala Lys Asn Trp Leu Pro
   465 470 475 480
   Gly Pro Cys Tyr Arg Gln Gln Arg Val Ser Lys Thr Thr Asn Gln Asn
   485 490 495
   Asn Asn Ser Asn Phe Ala Trp Thr Gly Ala Thr Lys Tyr His Leu Asn
   500 505 510
   Gly Arg Asp Ser Leu Val Asn Pro Gly Pro Ala Met Ala Thr His Lys
   515 520 525
   Asp Asp Glu Asp Lys Phe Phe Pro Met Ser Gly Val Leu Ile Phe Gly
   530 535 540
   Lys Gln Gly Ala Gly Asn Ser Asn Val Asp Leu Asp Asn Val Met Ile
   545 550 555 560
   Thr Asn Glu Glu Glu Ile Lys Thr Thr Asn Pro Val Ala Thr Glu Gln
   565 570 575
   Tyr Gly Thr Val Ala Thr Asn Leu Gln Ser Ala Asn Thr Ala Pro Ala
   580 585 590
   Thr Gly Thr Val Asn Ser Gln Gly Ala Leu Pro Gly Met Val Trp Gln
   595 600 605
   Asp Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys His Pro Pro Pro Gln Ile Leu Ile Lys Asn Thr Pro Val Pro Ala
   645 650 655
   Asn Pro Pro Thr Thr Phe Ser Pro Ala Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Ile Gln Tyr Thr Ser Asn
   690 695 700
   Tyr Asn Lys Ser Thr Asn Val Asp Phe Ala Val Asp Thr Asn Gly Val
   705 710 715 720
   Tyr Ser Glu Pro Arg Pro Ile Gly Thr Arg Tyr Leu Thr Arg Asn Leu
   725 730 735
<210> 27
   <211> 97
   <212> DNA
   <213> Artificial Sequence
<220>
   <223> sequence encoding shRNA
<400> 27
   tgctgttgac agtgagcgag cagatgagct cttagctgat tagtgaagcc acagatgtaa 60
   tcagctaaga gctcatctgc ctgcctactg cctcgga 97
<210> 28
   <211> 97
   <212> DNA
   <213> Artificial Sequence
<220>
   <223> sequence encoding shRNA
<400> 28
   tgctgttgac agtgagcgaa cccaacctgt ccatcattat tagtgaagcc acagatgtaa 60
   taatgatgga caggttgggt gtgcctactg cctcgga                          97
<210> 29
   <211> 97
   <212> DNA
   <213> Artificial Sequence
<220>
   <223> sequence encoding shRNA
<400> 29
   tgctgttgac agtgagcgag ctgagttcct caaggtcaag tagtgaagcc acagatgtac 60
   ttgaccttga ggaactcagc ctgcctactg cctcgga                          97
<210> 30
   <211> 97
   <212> DNA
   <213> Artificial Sequence
<220>
   <223> sequence encoding shRNA
<400> 30
   tgctgttgac agtgagcgct cagtcaagag cattgccaag tagtgaagcc acagatgtac 60
   ttggcaatgc tcttgactga atgcctactg cctcgga                          97
<210> 31
   <211> 97
   <212> DNA
   <213> Artificial Sequence
<220>
   <223> sequence encoding shRNA
<400> 31
   tgctgttgac agtgagcgac ctgtctggct tctcttctac tagtgaagcc acagatgtag 60
   tagaagagaa gccagacagg gtgcctactg cctcgga                          97
<210> 32
   <211> 97
   <212> DNA
   <213> Artificial Sequence
<220>
   <223> sequence encoding shRNA
<400> 32
   tgctgttgac agtgagcgag ccgagttcct caaggtcaag tagtgaagcc acagatgtac 60
   ttgaccttga ggaactcggc ctgcctactg cctcgga                          97
<210> 33
   <211> 97
   <212> DNA
   <213> Artificial Sequence
<220>
   <223> shRNA to green fluorescence protein
<400> 33
   tgctgttgac agtgagcgct ctccgaacgt gtatcacgtt tagtgaagcc acagatgtaa 60
   acgtgataca cgttcggaga ttgcctactg cctcgga                          97
<210> 34
   <211> 6231
   <212> DNA
   <213> Artificial Sequence
<220>
   <223> pZac2.1-CASI.PRPR31 vector
<400> 34
   ctgcgcgctc gctcgctcac tgaggccgcc cgggcaaagc ccgggcgtcg ggcgaccttt 60
   ggtcgcccgg cctcagtgag cgagcgagcg cgcagagagg gagtggccaa ctccatcact 120
   aggggttcct tgtagttaat gattaacccg ccatgctact tatctacgta gccatgctct 180
   aggaagatcg gaattcggag ttccgcgtta cataacttac ggtaaatggc ccgcctggct 240
   gaccgcccaa cgacccccgc ccattgacgt caataatgac gtatgttccc atagtaacgc 300
   caatagggac tttccattga cgtcaatggg tggagtattt acggtaaact gcccacttgg 360
   cagtacatca agtgtatcat atgccaagta cgccccctat tgacgtcaat gacggtaaat 420
   ggcccgcctg gcattatgcc cagtacatga ccttatggga ctttcctact tggcagtaca 480
   tctacgtatt agtcatcgct attaccatgg tcgaggtgag ccccacgttc tgcttcactc 540
   tccccatctc ccccccctcc ccacccccaa ttttgtattt atttattttt taattatttt 600
   gtgcagcgat gggggcgggg gggggggggg ggcgcgcgcc aggcggggcg gggcggggcg 660
   aggggcgggg cggggcgagg cggagaggtg cggcggcagc caatcagagc ggcgcgctcc 720
   gaaagtttcc ttttatggcg aggcggcggc ggcggcggcc ctataaaaag cgaagcgcgc 780
   ggcgggcggg agtcgctgcg cgctgccttc gccccgtgcc ccgctccgcc gccgcctcgc 840
   gccgcccgcc ccggctctga ctgaccgcgt tactaaaaca ggtaagtccg gcctccgcgc 900
   cgggttttgg cgcctcccgc gggcgccccc ctcctcacgg cgagcgctgc cacgtcagac 960
   gaagggcgca gcgagcgtcc tgatccttcc gcccggacgc tcaggacagc ggcccgctgc 1020
   tcataagact cggccttaga accccagtat cagcagaagg acattttagg acgggacttg 1080
   ggtgactcta gggcactggt tttctttcca gagagcggaa caggcgagga aaagtagtcc 1140
   cttctcggcg attctgcgga gggatctccg tggggcggtg aacgccgatg atgcctctac 1200
   taaccatgtt catgttttct ttttttttct acaggtcctg ggtgacgaac aggctagcgc 1260
   caccatgggt aagcctatcc ctaaccctct cctcggtctc gattctacgg ccgccaccat 1320
   gtctctggca gatgagctct tagctgatct cgaagaggca gcagaagagg aggaaggagg 1380
   aagctatggg gaggaagaag aggagccagc gatcgaggat gtgcaggagg agacacagct 1440
   ggatctttcc ggggattcag tcaagaccat cgccaagcta tgggatagta agatgtttgc 1500
   tgagattatg atgaagattg aggagtatat cagcaagcaa gccaaagctt cagaagtgat 1560
   gggaccagtg gaggccgcgc ctgaataccg cgtcatcgtg gatgccaaca acctgaccgt 1620
   ggagatcgaa aacgagctga acatcatcca taagttcatc cgggataagt actcaaagag 1680
   attccctgaa ctggagtcct tggtccccaa tgcactggat tacatccgca cggtcaagga 1740
   gctgggcaac agcctggaca agtgcaagaa caatgagaac ctgcagcaga tcctcaccaa 1800
   tgccaccatc atggtcgtca gcgtcaccgc ctccaccacc caggggcagc agctgtcgga 1860
   ggaggagctg gagcggctgg aggaggcctg cgacatggcg ctggagctga acgcctccaa 1920
   gcaccgcatc tacgagtatg tggagtcccg gatgtccttc atcgcaccca acctgtccat 1980
   cattatcggg gcatccacgg ccgccaagat catgggtgtg gccggcggcc tgaccaacct 2040
   ctccaagatg cccgcctgca acatcatgct gctcggggcc cagcgcaaga cgctgtcggg 2100
   cttctcgtct acctcagtgc tgccccacac cggctacatc taccacagtg acatcgtgca 2160
   gtccctgcca ccggatctgc ggcggaaagc ggcccggctg gtggccgcca agtgcacact 2220
   ggcagcccgt gtggacagtt tccacgagag cacagaaggg aaggtgggct acgaactgaa 2280
   ggatgagatc gagcgcaaat tcgacaagtg gcaggagccg ccgcctgtga agcaggtgaa 2340
   gccgctgcct gcgcccctgg atggacagcg gaagaagcga ggcggccgca ggtaccgcaa 2400
   gatgaaggag cggctggggc tgacggagat ccggaagcag gccaaccgta tgagcttcgg 2460
   agagatcgag gaggacgcct accaggagga cctgggattc agcctgggcc acctgggcaa 2520
   gtcgggcagt gggcgtgtgc ggcagacaca ggtaaacgag gccaccaagg ccaggatctc 2580
   caagacgctg cagcggaccc tgcagaagca gagcgtcgta tatggcggga agtccaccat 2640
   ccgcgaccgc tcctcgggca cggcctccag cgtggccttc accccactcc agggcctgga 2700
   gattgtgaac ccacaggcgg cagagaagaa ggtggctgag gccaaccaga agtatttctc 2760
   cagcatggct gagttcctca aggtcaaggg cgagaagagt ggccttatgt ccacctgaac 2820
   cggttggcta ataaaggaaa tttattttca ttgcaatagt gtgttggaat tttttgtgtc 2880
   tctcactcgg aaggacatat gggagggcaa atcatttaaa acatcagaat gagtatttgg 2940
   tttagagttt ggcaacatat gcccatatgc tggctgccat gaacaaaggt tggctataaa 3000
   gaggtcatca gtatatgaaa cagccccctg ctgtccattc cttattccat agaaaagcct 3060
   tgacttgagg ttagattttt tttatatttt gttttgtgtt atttttttct ttaacatccc 3120
   taaaattttc cttacatgtt ttactagcca gatttttcct cctctcctga ctactcccag 3180
   tcatagctgt ccctcttctc ttatggagat cggatccgaa ttcccgataa ggatcttcct 3240
   agagcatggc tacgtagata agtagcatgg cgggttaatc attaactaca aggaacccct 3300
   agtgatggag ttggccactc cctctctgcg cgctcgctcg ctcactgagg ccgggcgacc 3360
   aaaggtcgcc cgacgcccgg gctttgcccg ggcggcctca gtgagcgagc gagcgcgcag 3420
   ccttaattaa cctaattcac tggccgtcgt tttacaacgt cgtgactggg aaaaccctgg 3480
   cgttacccaa cttaatcgcc ttgcagcaca tccccctttc gccagctggc gtaatagcga 3540
   agaggcccgc accgatcgcc cttcccaaca gttgcgcagc ctgaatggcg aatgggacgc 3600
   gccctgtagc ggcgcattaa gcgcggcggg tgtggtggtt acgcgcagcg tgaccgctac 3660
   acttgccagc gccctagcgc ccgctccttt cgctttcttc ccttcctttc tcgccacgtt 3720
   cgccggcttt ccccgtcaag ctctaaatcg ggggctccct ttagggttcc gatttagtgc 3780
   tttacggcac ctcgacccca aaaaacttga ttagggtgat ggttcacgta gtgggccatc 3840
   gccctgatag acggtttttc gccctttgac gttggagtcc acgttcttta atagtggact 3900
   cttgttccaa actggaacaa cactcaaccc tatctcggtc tattcttttg atttataagg 3960
   gattttgccg atttcggcct attggttaaa aaatgagctg atttaacaaa aatttaacgc 4020
   gaattttaac aaaatattaa cgtttataat ttcaggtggc atctttcggg gaaatgtgcg 4080
   cggaacccct atttgtttat ttttctaaat acattcaaat atgtatccgc tcatgagaca 4140
   ataaccctga taaatgcttc aataatattg aaaaaggaag agtatgagta ttcaacattt 4200
   ccgtgtcgcc cttattccct tttttgcggc attttgcctt cctgtttttg ctcacccaga 4260
   aacgctggtg aaagtaaaag atgctgaaga tcagttgggt gcacgagtgg gttacatcga 4320
   actggatctc aatagtggta agatccttga gagttttcgc cccgaagaac gttttccaat 4380
   gatgagcact tttaaagttc tgctatgtgg cgcggtatta tcccgtattg acgccgggca 4440
   agagcaactc ggtcgccgca tacactattc tcagaatgac ttggttgagt actcaccagt 4500
   cacagaaaag catcttacgg atggcatgac agtaagagaa ttatgcagtg ctgccataac 4560
   catgagtgat aacactgcgg ccaacttact tctgacaacg atcggaggac cgaaggagct 4620
   aaccgctttt ttgcacaaca tgggggatca tgtaactcgc cttgatcgtt gggaaccgga 4680
   gctgaatgaa gccataccaa acgacgagcg tgacaccacg atgcctgtag taatggtaac 4740
   aacgttgcgc aaactattaa ctggcgaact acttactcta gcttcccggc aacaattaat 4800
   agactggatg gaggcggata aagttgcagg accacttctg cgctcggccc ttccggctgg 4860
   ctggtttatt gctgataaat ctggagccgg tgagcgtggg tctcgcggta tcattgcagc 4920
   actggggcca gatggtaagc cctcccgtat cgtagttatc tacacgacgg ggagtcaggc 4980
   aactatggat gaacgaaata gacagatcgc tgagataggt gcctcactga ttaagcattg 5040
   gtaactgtca gaccaagttt actcatatat actttagatt gatttaaaac ttcattttta 5100
   atttaaaagg atctaggtga agatcctttt tgataatctc atgaccaaaa tcccttaacg 5160
   tgagttttcg ttccactgag cgtcagaccc cgtagaaaag atcaaaggat cttcttgaga 5220
   tccttttttt ctgcgcgtaa tctgctgctt gcaaacaaaa aaaccaccgc taccagcggt 5280
   ggtttgtttg ccggatcaag agctaccaac tctttttccg aaggtaactg gcttcagcag 5340
   agcgcagata ccaaatactg tccttctagt gtagccgtag ttaggccacc acttcaagaa 5400
   ctctgtagca ccgcctacat acctcgctct gctaatcctg ttaccagtgg ctgctgccag 5460
   tggcgataag tcgtgtctta ccgggttgga ctcaagacga tagttaccgg ataaggcgca 5520
   gcggtcgggc tgaacggggg gttcgtgcac acagcccagc ttggagcgaa cgacctacac 5580
   cgaactgaga tacctacagc gtgagctatg agaaagcgcc acgcttcccg aagggagaaa 5640
   ggcggacagg tatccggtaa gcggcagggt cggaacagga gagcgcacga gggagcttcc 5700
   agggggaaac gcctggtatc tttatagtcc tgtcgggttt cgccacctct gacttgagcg 5760
   tcgatttttg tgatgctcgt caggggggcg gagcctatgg aaaaacgcca gcaacgcggc 5820
   ctttttacgg ttcctggcct tttgctgcgg ttttgctcac atgttctttc ctgcgttatc 5880
   ccctgattct gtggataacc gtattaccgc ctttgagtga gctgataccg ctcgccgcag 5940
   ccgaacgacc gagcgcagcg agtcagtgag cgaggaagcg gaagagcgcc caatacgcaa 6000
   accgcctctc cccgcgcgtt ggccgattca ttaatgcagc tggcacgaca ggtttcccga 6060
   ctggaaagcg ggcagtgagc gcaacgcaat taatgtgagt tagctcactc attaggcacc 6120
   ccaggcttta cactttatgc ttccggctcg tatgttgtgt ggaattgtga gcggataaca 6180
   atttcacaca ggaaacagct atgaccatga ttacgccaga tttaattaag g          6231

## Description

### TECHNICAL FIELD

The present disclosure relates to methods and compositions for gene therapy of retinitis pigmentosa related to mutations in pre-mRNA processing factor 31 (PRPF31).

### BACKGROUND

Mutations in the Pre-mRNA Processing Factor 31 (PRPF31) cause non-syndromic retinitis pigmentosa (RP) in humans, an inherited retinal dystrophy (IRD). It is currently unclear what mechanisms, or which tissues, are affected when mutations are present in these ubiquitously expressed proteins.

### SUMMARY

The invention is defined in the claims. Described herein are methods and compositions for gene therapy of retinitis pigmentosa related to mutations in pre-mRNA processing factor 31 (PRPF31).

Thus, provided herein are vectors and compositions for use in treating retinitis pigmentosa caused by mutations in PRPF31 in a human subject, or for increasing expression of PRPF31 in the eye of a human subject. The methods, that form part of the present disclosure, include delivering to the eye of the subject a therapeutically effective amount of an adeno-associated viral vector, e.g., an Adeno-associated virus type 2 (AAV2) vector, comprising a sequence encoding human PRPF31, operably linked to a promoter that drives expression in retinal pigment epithelial (RPE) cells.

The promoter can be RPE-specific or can be a general promoter that drives expression in other cells types as well, e.g., CASI or CAG. In some embodiments, the promoter is an RPE65 or VMD2 promotor.

In some embodiments, the PRPF31 sequence is codon optimized, e.g., for expression in human cells where the subject is a human. In some embodiments, the PRPF31 sequence is or comprises, or encodes the same protein as, nts 1319-2818 of SEQ ID NO:34.

In some aspects of the disclosure, the vector is delivered via sub-retinal injection.

In some aspects of the disclosure, the vector comprises, or comprises a sequence encoding, an AAV capsid polypeptide described in WO 2015054653.

Also provided herein are adeno-associated viral vectors, e.g., adeno-associated virus type 2 (AAV2) vectors comprising a sequence encoding human PRPF31, operably linked to a promotor that drives expression in retinal pigment epithelial (RPE) cells. The promoter can be RPE-specific or can be a general promoter that drives expression in other cells types as well, e.g., CASI or CAG. In some embodiments, the promotor is an RPE65 or VMD2 promotor. In some embodiments, the PRPF31 sequence is codon optimized, e.g., for expression in human cells. Also provided are pharmaceutical compositions comprising the vector, preferably formulated for delivery via sub-retinal injection.

Also provided herein is the use of the nucleic acids, vectors, and pharmaceutical compositions described herein
Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. In case of conflict, the present specification, including definitions, will control.

Other features and advantages of the invention will be apparent from the following detailed description and figures, and from the claims.

### DESCRIPTION OF DRAWINGS

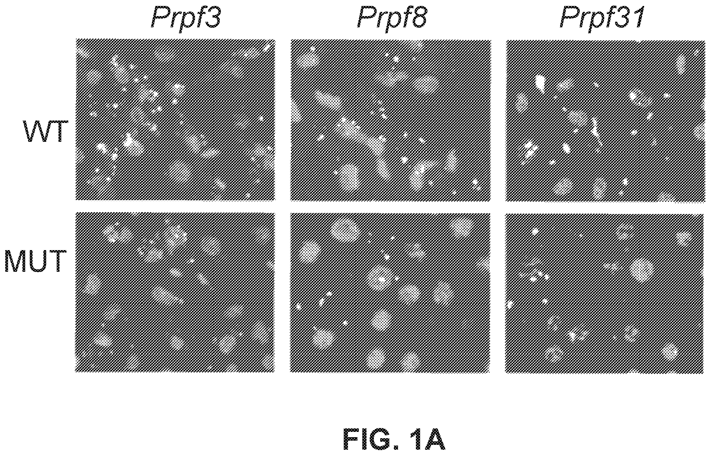

Figs. 1A-F. Inhibition of phagocytosis in Prpf-mutant mice. Retinal pigment epithelial (RPE) primary cultures were established from 9-10-day old Prpf-mutant mice and their littermate controls, then challenged with FITC-labeled porcine photoreceptor outer segments and nuclei labeled with DAPI (blue). (A) Qualitative representation of primary RPE cells (blue DAPI staining of nuclei) from wild-type (WT) or Prpf3T494M/T494M, Prpf8H2309P/H2309P, Prpf31+/- mutant (MUT) mice. A difference in POS uptake was observed between the mutants and controls. (B) Quantitative analysis of the phagocytic ratio demonstrates a significant decrease in phagocytosis in the mutant (MUT) mice compared to wild-type littermates (WT) for the 3 mutant mouse strains as indicated (* P < 0.05, N = 3-5). (C) Binding and internalization ratios of POS was compared between Prpf31+/- (MUT) mice compared to wild-type controls (WT) showing a significant decrease in binding (Bind.), but no significant change in POS internalization (Intern.) in mutant mice (* P < 0.05, N = 2-5). (D) A stable line of shRNA-mediated knockdown of PRPF31 in ARPE-19 cells. A difference in POS uptake was also observed between the control shRNA-transfected ARPE-19 cells and anti-PRPF31 shRNA-transfected ARPE-19 cells. (E) Cell viability assay to determine the effect of PRPF31-knockdown in ARPE-19 cells shows that there are no significant differences in cell growth or viability following shRNA-knockdown of PRPF31 relative to the non-targeted control (P > 0.05, N = 6). (F) shRNA-mediated knockdown of PRPF31 in the human ARPE-19 cell line also inhibits phagocytosis significantly as showed by the decreased number of POS per cell compared to non-targeting constructs (NTC) (* P < 0.05, N = 3). Error bars represent standard deviation from the mean.

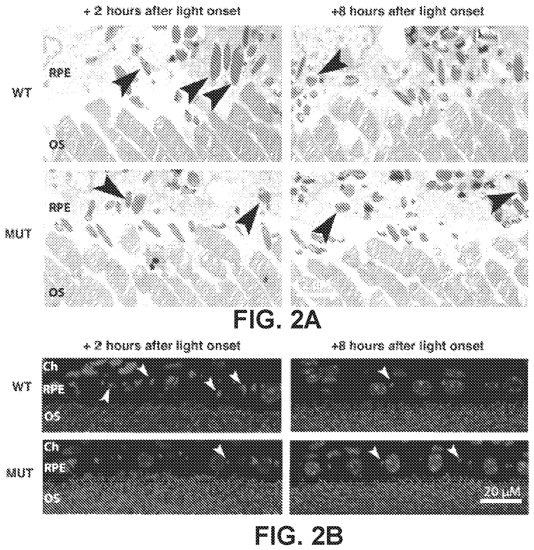

Figs. 2A-C. The diurnal rhythmicity of phagocytosis in Prpf-mutant mice is disrupted. Phagocytosis was assayed in vivo at 2 hours before light onset (-2), at light onset (0), and 2, 4, and 6 (+2, +4, +6) hours after light onset. (A, B) Representative pictures are shown at +2 (phagocytic peak) and +8 (outside of the phagocytic peak) hours after light onset as indicated. RPE: retinal pigment epithelium, OS: photoreceptor outer segments, Ch: choroid. (A) Detection of early phagosomes in Prpf3- and Prpf8-mutant mice was performed using electron microscopy and counting phagosomes that were 1) in the cytoplasm of the RPE and 2) contained visible lamellar structure (black arrowheads). Scale bar 2 µm. (B) The diurnal rhythm of Prpf31+/- mice was determined using immunofluorescent staining for rhodopsin (Ig-AlexaFluor488) and detection of phagosomes (white arrowheads) located in the RPE cell layer (DAPI-stained nuclei) across 100 µm of intact retina. Scale bar 20 µm. (C) Phagosome quantification across all time points demonstrates the consistent significant disruption of the phagocytic burst in all Prpf-mutant mice (* P < 0.05, N = 2 for Prpf3- and Prpf8-mutant and N = 3-5 for Prpf31-mutant mice). Error bars represent standard deviation from the mean.

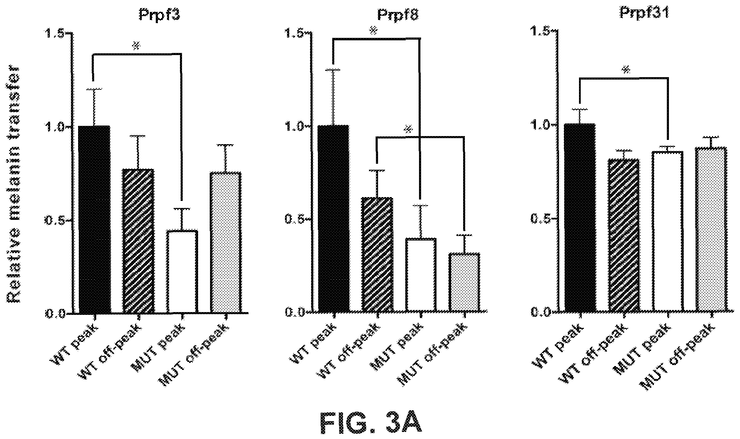

Figs. 3A-B. Alterations in retinal adhesion in Prpf-mutant mice at the peak time-point. Adhesive strength between RPE apical microvilli and POS was determined by quantifying the amount of RPE pigments or proteins that adheres to the neural retina, relative to the WT control. (A) Melanin quantification demonstrates that adhesion is decreased in all three mutant mice at the peak time-point 3.5 hours after light onset, and in Prpf8H2309P/H2309P mice at the off-peak time-point (* P <0.05, N = 3-7). (B) Quantitative measurements of RPE65 proteins on immunoblots confirm the melanin findings in all three mutant mice at the peak time-point, however only a trend is observed for decrease in adhesion at the off-peak time-point in Prpf8-mutant mice (* P <0.05, N = 4-7). Error bars represent standard deviation from the mean.

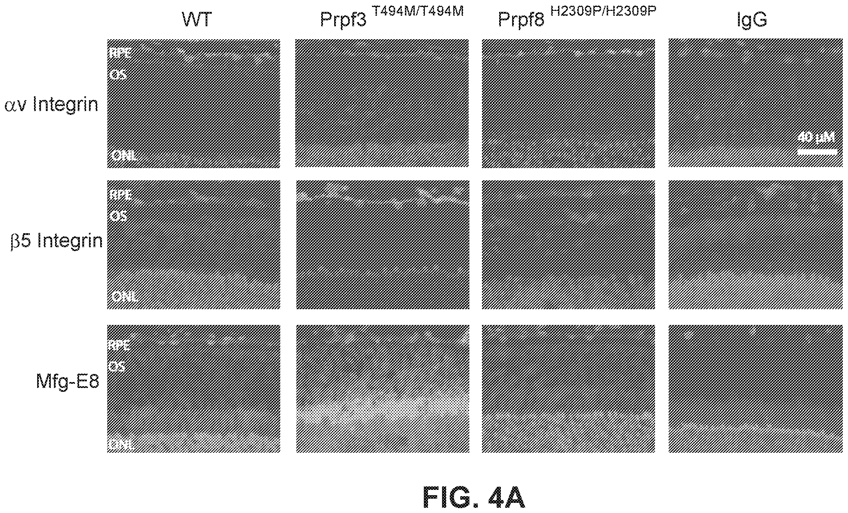

Figs. 4A-C. Localization and expression of some adhesion and phagocytosis markers are perturbed in Prpf3- and Prpf8-mutant mice. Representative images of the expression and localization of (A) αv and β5 integrin receptor subunits and associated Mfg-E8 ligand, (B) FAK intracellular signaling protein, and (C) MerTK receptors and associated Gas6 and Protein S ligands on wild-type control (WT) as well as Prpf3- and Prpf8-mutant retinal cryosections as indicated. Images from sections probed with non-immune IgG (IgG) are included for each antigen. RPE: retinal pigment epithelium, OS: photoreceptor outer segments, ONL: outer nuclear layer. Localization of β5 integrin to the basal side of the RPE was observed in both Prpf3- and Prpf8-mutant mice. Additionally, FAK was mislocalized in Prpf8-mutant mice to the basal side of the RPE. Each protein of interest was stained with Ig-AlexaFluor488 and nuclei are stained with DAPI. Scale bar 40 µm.

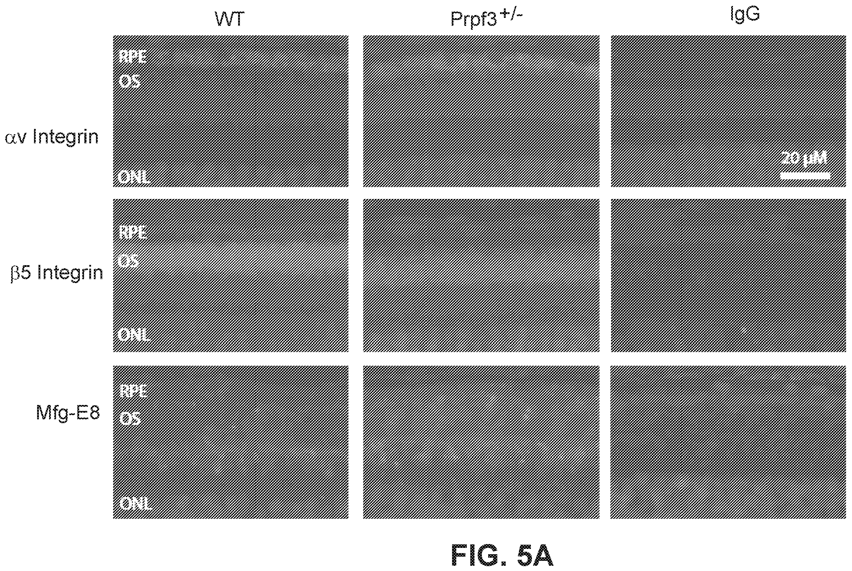

Figs. 5A-C. Localization and expression of some adhesion and phagocytosis markers are perturbed in Prpf31-mutant mice. Representative images of the expression and localization of (A) αv and β5 integrin receptor subunits and associated Mfg-E8 ligand, (B) FAK intracellular signaling protein, and (C) MerTK receptors and associated Gas6 and Protein S ligands or non-immune IgG (IgG) on wild-type control (WT) as well as Prpf31-mutant retinal paraffin sections as indicated. RPE: retinal pigment epithelium, OS: photoreceptor outer segments, ONL: outer nuclear layer. The most notable change in Prpf31-mutant mice is the mislocalization of β5 integrin to the basal side of the RPE, while localization of MerTK is also perturbed. Each protein of interest was stained with IgG-AlexaFluor488 and nuclei are stained with DAPI. Scale bar 20 µm.

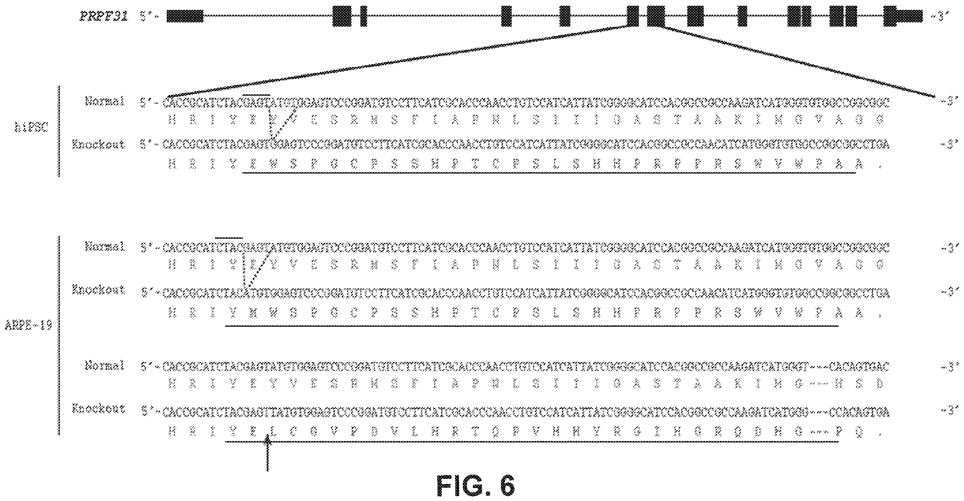

Fig. 6. Sequence of *PRPF31*^{+/-} hiPSC and ARPE-19 cell lines generated via genome editing. The gene model for *PRPF31* is shown above, with sequence detail in exons 6-7 for the three example cell lines shown below. The knockout hiPSC cell line has a heterozygous 4bp deletion (deleted bases shown overlined in normal sequence), which results in a frame shift (underlined amino acids), and premature stop. The knockout ARPE-19 cell lines depicted have a 4bp deletion or a single base insertion which also result in frameshifts and null alleles.

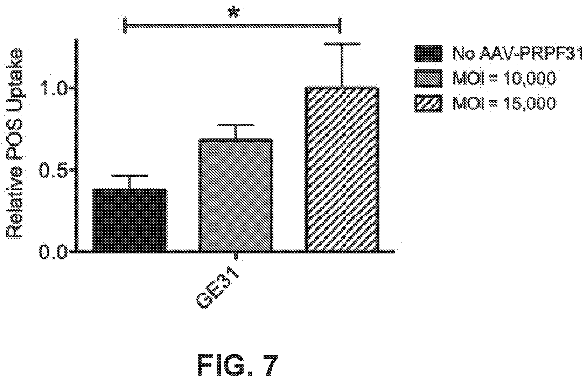

Fig. 7. Relative POS uptake following treatment with AAV.CASI.PRPF31. Genome-edited PRPF31 (GE31) ARPE-19 cells were transduced with AAV.CASI.PRPF31 at MOIs of 0, 10,000, and 15,000 for 3 days. Subsequently, the cells were incubated with FITC-POS for 1 hour and FITC positive cells were counted by flow cytometry. *P<0.05.

### DETAILED DESCRIPTION

The invention is defined in the claims. Mutations in genes that encode RNA splicing factors are the second most common cause of the dominant form of the blinding disorder retinitis pigmentosa (RP), and thus are an important cause of vision loss (Hartong et al., Lancet. 2006;368:1795-809; Daiger et al., Archives Ophthalmology. 2007;125:151-8; Sullivan et al., Investigative Ophthalmology & Visual Science. 2013;54:6255-61. PMCID: 3778873). The splicing factors affected, pre-mRNA processing factor (PRPF) 3, PRPF4, PRPF6, PRPF8, PRPF31, and SNRNP200 are highly conserved components of the spliceosome, the complex which excises introns from nascent RNA transcripts to generate mature mRNAs (McKie et al., Human Molecular Genetics. 2001;10:1555-62; Vithana et al., Molecular Cell. 2001;8:375-81; Chakarova et al., Human Molecular Genetics. 2002;11:87-92; Keen et al., European Journal Human Genetics. 2002;10:245-9; Nilsen, Bioessays. 2003;25:1147-9; Sullivan et al., Investigative Ophthalmology & Visual Science. 2006;47:4579-88; Zhao et al., American Journal Human Genetics. 2009;85:617-27; Tanackovic et al., American Journal Human Genetics. 2011;88:643-9; Chen et al., Human Molecular Genetics. 2014;23:2926-39.). Mutations in the PRPF31 gene are the most common cause of RNA splicing factor RP, and are estimated to account for 2400 to 8500 affected individuals in the US and 55,000 to 193,000 people worldwide (Daiger et al., Archives Ophthalmology. 2007;125:151-8; Sullivan et al., Investigative Ophthalmology & Visual Science. 2013;54:6255-61. PMCID: 3778873). Since RNA splicing is required in all cells, it is not clear how mutations in these ubiquitous proteins lead to retina-specific disease.

To understand the mechanism(s) by which mutations in RNA splicing factors cause retinal degeneration, the phenotypes of Prpf3, Prpf8 and Prpf31 mutant mice were studied. Cell autonomous defects were identified in retinal pigment epithelial (RPE) cell function in gene targeted mice; however, genetic and phenotypic differences in disease between the mouse models and the human condition make conclusions drawn in mice potentially difficult to translate to humans.

There is some evidence that mutations in PRPF31 cause disease via haploinsuffiency, and thus that this form of dominant RP is amenable to treatment with gene augmentation therapy. Many of the mutations identified in PRPF31 are either large chromosomal deletions or are nonsense and frameshift mutations that lead to premature termination codons that undergo nonsense mediated mRNA decay and result in null alleles (Vithana et al., Molecular Cell. 2001;8:375-81; Sullivan et al., Investigative Ophthalmology & Visual Science. 2006;47:4579-88.; Wang et al., American Journal Medical Genetics A. 2003;121A:235-9; Xia et al., Molecular Vision. 2004;10:361-5; Sato et al., American Journal Ophthalmology. 2005;140:537-40; Abu-Safieh et al., MolVis. 2006;12:384-8; Rivolta et al., Human Mutation. 2006;27:644-53; Waseem et al., Investigative Ophthalmology & Visual Science. 2007;48:1330-4; Rio Frio et al., Human Mutation. 2009;30:1340-7. PMCID: 2753193; Rose et al., Investigative Ophthalmology & Visual Science. 2011;52:6597-603; Saini et al., Experimental Eye Research. 2012;104:82-8). Thus, it is thought that PRPF31-associated retinal degeneration is caused by haploinsufficiency. Consistent with this hypothesis, the level of PRPF31 expression from the wild-type allele correlates with the severity of disease in patients with mutations in PRPF31 (Rio et al., Journal Clinical Investigation. 2008;118:1519-31; Vithana et al., Investigative Ophthalmology & Visual Science. 2003;44:4204-9; McGee et al., American Journal Human Genetics. 1997;61:1059-66). Two mechanisms have been reported to contribute to regulation of expression of the wild-type PRPF31 allele. First, CNOT3 regulates PRPF31 expression via transcriptional repression; in asymptomatic carriers of PRPF31 mutations, CNOT3 is expressed at low levels, allowing higher amounts of wild-type PRPF31 transcripts to be produced and preventing manifestation of retinal degeneration (Venturini et al., PLoS genetics. 2012;8:e1003040. PMCID: 3493449; Rose et al., Annals Human Genetics. 2013). Second, MSR1 has been identified as a cis regulatory element upstream of the PRPF31. Thus, human genetic variation has provided evidence that augmentation of PRPF31 gene expression can reduce or eliminate vision loss in this disorder.

As described herein, the present inventors have identified RPE cells as the primary cells affected in RNA splicing factor RP; this creates an opportunity to move forward with development of gene augmentation therapy for disease caused by mutations in PRPF31 (see **Example 1**). To achieve this goal, described herein are AAV vectors for expressing human PRPF31, which can be used to ameliorate the phenotype in human subjects.

The sequence of human PRPF31, also known as U4/U6 small nuclear ribonucleoprotein Prp31, is available in GenBank at Accession Nos. NM_015629.3 (nucleic acid) and NP_056444.3 (Protein). Subjects having RP associated with mutations in PRPF31 can be identified by methods known in the art, e.g., by sequencing the PRPF31 gene (NG_009759.1, Range: 5001 to 21361) or NC_000019.10 Reference GRCh38.p2 Primary Assembly, Range 54115376 to 54131719). A large number of mutations in affected individuals have been identified; see, e.g., Villanueva et al. Invest Ophthalmol Vis Sci, 2014; Dong et al. Mol Vis, 2013; Lu F, et al. PLoS One, 2013; Utz et al. Ophthalmic Genet, 2013; and Xu F, et al. Mol Vis, 2012; Saini et al., Exp Eye Res. 2012 Nov;104:82-8; Rose et al., Invest Ophthalmol Vis Sci. 2011 Aug 22;52(9):6597-603; Audo et al., BMC Med Genet. 2010 Oct 12;11:145; and Tanackovic and Rivolta, Ophthalmic Genet. 2009 Jun;30(2):76-83.

Thus described herein are targeted expression vectors for in vivo transfection and expression of a polynucleotide that encodes a PRPF31 polypeptide as described herein, in RPE cells, e.g., primarily or only in RPE cells. Expression constructs of such components can be administered in any effective carrier, e.g., any formulation or composition capable of effectively delivering the component gene to cells in vivo. Approaches include insertion of the gene in viral vectors, including recombinant retroviruses, adenovirus, adeno-associated virus, lentivirus, and herpes simplex virus-1, alphavirus, vaccinia virus, or recombinant bacterial or eukaryotic plasmids; preferred viral vectors are adeno-associated virus type 2 (AAV2). Viral vectors transfect cells directly; plasmid DNA can be delivered naked or with the help of, for example, cationic liposomes (lipofectamine) or derivatized (e.g., antibody conjugated), cationic dendrimers, inorganic vectors (e.g., iron oxide magnetofection), lipidoids, cell-penetrating peptides, cyclodextrin polymer (CDP), polylysine conjugates, gramacidin S, artificial viral envelopes or other such intracellular carriers, as well as direct injection of the gene construct or CaPO4 precipitation carried out in vivo.

An exemplary approach for in vivo introduction of nucleic acid into a cell is by use of a viral vector containing nucleic acid, e.g., a cDNA. Infection of cells with a viral vector has the advantage that a large proportion of the targeted cells can receive the nucleic acid. Additionally, molecules encoded within the viral vector, e.g., by a cDNA contained in the viral vector, are expressed efficiently in cells that have taken up viral vector nucleic acid.

Viral vectors can be used as a recombinant gene delivery system for the transfer of exogenous genes *in vivo*, particularly into humans. These vectors provide efficient delivery of genes into cells, and in some cases the transferred nucleic acids are stably integrated into the chromosomal DNA of the host. Protocols for producing recombinant viruses and for infecting cells in vitro or in vivo with such viruses can be found in Ausubel, et al., eds., Gene Therapy Protocols Volume 1: Production and In Vivo Applications of Gene Transfer Vectors, Humana Press, (2008), pp. 1-32 and other standard laboratory manuals.

A preferred viral vector system useful for delivery of nucleic acids is the adeno-associated virus (AAV). Adeno-associated virus is a naturally occurring defective virus that requires another virus, such as an adenovirus or a herpes virus, as a helper virus for efficient replication and a productive life cycle. (For a review see Muzyczka et al., Curr. Topics in Micro and Immunol. 158:97-129 (1992)). AAV vectors efficiently transduce various cell types and can produce long-term expression of transgenes *in vivo*. Although AAV vector genomes can persist within cells as episomes, vector integration has been observed (see for example Deyle and Russell, Curr Opin Mol Ther. 2009 Aug; 11(4): 442-447; Asokan et al., Mol Ther. 2012 April; 20(4): 699-708; Flotte et al., Am. J. Respir. Cell. Mol. Biol. 7:349-356 (1992); Samulski et al., J. Virol. 63:3822-3828 (1989); and McLaughlin et al., J. Virol. 62:1963-1973 (1989)). AAV vectors, particularly AAV2, have been extensively used for gene augmentation or replacement and have shown therapeutic efficacy in a range of animal models as well as in the clinic; see, e.g., Mingozzi and High, Nature Reviews Genetics 12, 341-355 (2011); Deyle and Russell, Curr Opin Mol Ther. 2009 Aug; 11(4): 442-447; Asokan et al., Mol Ther. 2012 April; 20(4): 699-708. AAV vectors containing as little as 300 base pairs of AAV can be packaged and can produce recombinant protein expression. Space for exogenous DNA is limited to about 4.5 kb. For example, an AAV1, 2, 4, 5, or 8 vector can be used to introduce DNA into RPE cells (such as those described in Maguire et al. (2008). Safety and efficacy of gene transfer for Leber's congenital amaurosis. N Engl J Med 358: 2240-2248. Maguire et al. (2009). Age-dependent effects of RPE65 gene therapy for Leber's congenital amaurosis: a phase 1 dose-escalation trial. Lancet 374: 1597-1605; Bainbridge et al. (2008). Effect of gene therapy on visual function in Leber's congenital amaurosis. N Engl J Med 358: 2231-2239; Hauswirth et al. (2008). Treatment of leber congenital amaurosis due to RPE65 mutations by ocular subretinal injection of adeno-associated virus gene vector: short-term results of a phase I trial. Hum Gene Ther 19: 979-990; Cideciyan et al. (2008). Human gene therapy for RPE65 isomerase deficiency activates the retinoid cycle of vision but with slow rod kinetics. Proc Natl Acad Sci USA 105: 15112-15117. Cideciyan et al. (2009). Vision 1 year after gene therapy for Leber's congenital amaurosis. N Engl J Med 361: 725-727; Simonelli et al. (2010). Gene therapy for Leber's congenital amaurosis is safe and effective through 1.5 years after vector administration. Mol Ther 18: 643-650; Acland, et al. (2005). Long-term restoration of rod and cone vision by single dose rAAV-mediated gene transfer to the retina in a canine model of childhood blindness. Mol Ther 12: 1072-1082; Le Meur et al. (2007). Restoration of vision in RPE65-deficient Briard dogs using an AAV serotype 4 vector that specifically targets the retinal pigmented epithelium. Gene Ther 14: 292-303; Stieger et al. (2008). Subretinal delivery of recombinant AAV serotype 8 vector in dogs results in gene transfer to neurons in the brain. Mol Ther 16: 916-923; and Vandenberghe et al. (2011). Dosage thresholds for AAV2 and AAV8 photoreceptor gene therapy in monkey. Sci Transl Med 3: 88ra54). In some embodiments, the AAV vector can include (or include a sequence encoding) an AAV capsid polypeptide described in WO 2015054653; for example, a virus particle comprising an AAV capsid polypeptide having an amino acid sequence selected from the group consisting of SEQ ID NOs: 1, 3, 5, 7, 9, 11, 13, 15, and 17 of WO 2015054653, and a PRPF31-encoding sequence as described herein. In some embodiments, the AAV capsid polypeptide is as shown in Table 1 of WO 2015054653, reproduced here:

| Node | Polypeptide (SEQ ID NO) | Nucleic Acid (SEQ ID NO) |
|---|---|---|
| Anc80 | 1 | 2 |
| Anc81 | 3 | 4 |
| Anc82 | 5 | 6 |
| Anc83 | 7 | 8 |
| Anc84 | 9 | 10 |
| Anc94 | 11 | 12 |
| Anc113 | 13 | 14 |
| Anc126 | 15 | 16 |
| Anc127 | 17 | 18 |

In some embodiments, the AAV capsid polypeptide is an Anc80 polypeptide, e.g., an exemplary polypeptide shown in SEQ ID NO: 19 (Anc80L27); SEQ ID NO: 20 (Anc80L59); SEQ ID NO: 21 (Anc80L60); SEQ ID NO: 22 (Anc80L62); SEQ ID NO: 23 (Anc80L65); SEQ ID NO: 24 (Anc80L33); SEQ ID NO: 25 (Anc80L36); and SEQ ID NO:26 (Anc80L44).

A variety of nucleic acids have been introduced into different cell types using AAV vectors (see for example the references cited above and those cited in Asokan et al., Molecular Therapy (2012); 20 4, 699-708; and Hermonat et al., Proc. Natl. Acad. Sci. USA 81:6466-6470 (1984); Tratschin et al., Mol. Cell. Biol. 4:2072-2081 (1985); Wondisford et al., Mol. Endocrinol. 2:32-39 (1988); Tratschin et al., J. Virol. 51:611-619 (1984); and Flotte et al., J. Biol. Chem. 268:3781-3790 (1993).

Retroviruses can also be used. The development of specialized cell lines (termed "packaging cells") which produce only replication-defective retroviruses has increased the utility of retroviruses for gene therapy, and defective retroviruses are characterized for use in gene transfer for gene therapy purposes (for a review see Katz et al., Human Gene Therapy 24:914 (2013)). A replication defective retrovirus can be packaged into virions, which can be used to infect a target cell through the use of a helper virus by standard techniques. Examples of suitable retroviruses include pLJ, pZIP, pWE and pEM which are known to those skilled in the art. Examples of suitable packaging virus lines for preparing both ecotropic and amphotropic retroviral systems include ΨCrip, ΨCre, Ψ2 and ΨAm. Retroviruses have been used to introduce a variety of genes into many different cell types, including epithelial cells, in vitro and/or in vivo (see for example Eglitis, et al. (1985) Science 230:1395-1398; Danos and Mulligan (1988) Proc. Natl. Acad. Sci. USA 85:6460-6464; Wilson et al. (1988) Proc. Natl. Acad. Sci. USA 85:3014-3018; Armentano et al. (1990) Proc. Natl. Acad. Sci. USA 87:6141-6145; Huber et al. (1991) Proc. Natl. Acad. Sci. USA 88:8039-8043; Ferry et al. (1991) Proc. Natl. Acad. Sci. USA 88:8377-8381; Chowdhury et al. (1991) Science 254:1802-1805; van Beusechem et al. (1992) Proc. Natl. Acad. Sci. USA 89:7640-7644; Kay et al. (1992) Human Gene Therapy 3:641-647; Dai et al. (1992) Proc. Natl. Acad. Sci. USA 89:10892-10895; Hwu et al. (1993) J. Immunol. 150:4104-4115; U.S. Patent No. 4,868,116; U.S. Patent No. 4,980,286; PCT Application WO 89/07136; PCT Application WO 89/02468; PCT Application WO 89/05345; and PCT Application WO 92/07573).

Another viral gene delivery system useful in the present methods utilizes adenovirus-derived vectors. The genome of an adenovirus can be manipulated, such that it encodes and expresses a gene product of interest but is inactivated in terms of its ability to replicate in a normal lytic viral life cycle. See, for example, Berkner et al., BioTechniques 6:616 (1988); Rosenfeld et al., Science 252:431-434 (1991); and Rosenfeld et al., Cell 68:143-155 (1992). Suitable adenoviral vectors derived from the adenovirus strain Ad type 5 dl324 or other strains of adenovirus (e.g., Ad2, Ad3, or Ad7 etc.) are known to those skilled in the art. Recombinant adenoviruses can be advantageous in certain circumstances, in that they are not capable of infecting non-dividing cells and can be used to infect a wide variety of cell types, including epithelial cells (Rosenfeld et al., (1992) supra). Furthermore, the virus particle is relatively stable and amenable to purification and concentration, and as above, can be modified so as to affect the spectrum of infectivity. Additionally, introduced adenoviral DNA (and foreign DNA contained therein) is not integrated into the genome of a host cell but remains episomal, thereby avoiding potential problems that can occur as a result of insertional mutagenesis in situ, where introduced DNA becomes integrated into the host genome (e.g., retroviral DNA). Moreover, the carrying capacity of the adenoviral genome for foreign DNA is large (up to 8 kilobases) relative to other gene delivery vectors (Berkner et al., supra; Haj-Ahmand and Graham, J. Virol. 57:267 (1986).

In some aspects of the disclosure, a gene encoding PRPF31 is entrapped in liposomes bearing positive charges on their surface (e.g., lipofectins), which can be tagged with antibodies against cell surface antigens of the target tissue (Mizuno et al., No Shinkei Geka 20:547-551 (1992); PCT publication WO91/06309; Japanese patent application 1047381; and European patent publication EP-A-43075).

In clinical settings, the gene delivery systems for the therapeutic gene can be introduced into a subject by any of a number of methods, each of which is familiar in the art. Although other methods can be used, in some aspects of the disclosure, the route of choice for delivery of gene therapy vectors to the retina is via sub-retinal injection. This provides access to the RPE and photoreceptor cells of the retina. Different serotypes of AAV have been shown to transduce these cell populations effectively after sub-retinal injection in animal studies (Vandenberghe et al., PLoS One. 2013;8:e53463. PMCID: 3559681; Vandenberghe and Auricchio, Gene Therapy. 2012;19:162-8; Vandenberghe et al., Science translational medicine. 2011;3:88ra54; Dinculescu et al., HumGene Ther. 2005;16:649-63; Boye et al., Mol Ther. 2013;21:509-19; Alexander and Hauswirth, Adv Exp Med Biol. 2008;613:121-8). The sub-retinal injection approach is being used in the ongoing clinical trials of gene augmentation therapy for retinal degeneration caused by mutations in the *RPE65* and *CHM* genes genetic disease (Maguire et al., New England Journal of Medicine. 2008;358:2240-8; Bainbridge et al., New England Journal of Medicine. 2008;358:2231-9; Cideciyan et al., Proceedings National Academy Sciences USA. 2008;105:15112-7; Maguire et al., Lancet. 2009;374:1597-605; Jacobson et al., Archives Ophthalmology. 2012;130:9-24; Bennett et al., Science translational medicine. 2012;4:120ra15; MacLaren et al., Lancet. 2014;383:1129-37). Sub-retinal injections can be performed using a standard surgical approach (e.g., as described in Maguire et al., 2008 supra; Bainbridge et al., 2008 supra; Cideciyan et al., 2008 supra; MacLaren et al., 2014 supra).

The pharmaceutical preparation of the gene therapy construct can consist essentially of the gene delivery system (viral vector and any associated agents such as helper viruses, proteins, lipids, and so on) in an acceptable diluent, or can comprise a slow release matrix in which the gene delivery vehicle is embedded. Alternatively, where the complete gene delivery system can be produced intact from recombinant cells, e.g., retroviral vectors, the pharmaceutical preparation can comprise one or more cells, which produce the gene delivery system.

### EXAMPLES

The invention is further described in the following examples, which do not limit the scope of the invention described in the claims.

### Example 1. Mutations in Pre-mRNA Processing Factors 3, 8 and 31 cause dysfunction of the retinal pigment epithelium

### Introduction

The spliceosome is a ubiquitous, dynamic ribonucleoprotein macromolecule required for removing introns from a nascent RNA¹. Mutations that cause autosomal dominant retinitis pigmentosa (RP) have been identified in 6 genes that encode proteins (PRPF3, PRPF4, PRPF6, PRPF8, PRPF31, and SNRNP200), which are found in the U4/U6.U5 tri-snRNP². In aggregate, mutations in these genes are the second most common cause of dominant RP³⁻⁵. Defined by progressive, late-onset vision loss, RP is the most common form of inherited retinal degeneration, affecting approximately 1:3500 individuals worldwide⁶. It is genetically heterogeneous, and displays all three modes of Mendelian inheritance⁷. Affected tissues include the neural retina, retinal pigment epithelium (RPE), and choroid⁴. Since the components of the spliceosome are ubiquitously expressed in every cell type, it is not clear why mutations in these splicing factors cause only non-syndromic RP. Further, the specific cell type(s) in the retina affected by these mutations has not yet been identified.

We have previously reported the characterization of mouse models of RNA splicing factor RP due to mutations in the *PRPF3*, *PRPF8* and *PRPF31* genes, including *Prpf3*, *Prpf8* and *Prpf31* knockout mice, and *Prpf3-T494M* and *Prpf8-*H2309P knockin mice^{8, 9}. Based on results from studies of these mouse models, and data from human studies, it is believed that mutations in *PRPF3* and *PRPF8* cause dominant disease via gain-of-function or dominant-negative mechanisms, while mutations in *PRPF31* cause disease via haploinsufficiency⁹⁻¹¹. Morphological changes in the aging RPE, but not the neural retina, of the *Prpf3-*T494M and *Prpf8-*H2309P knockin mice and *Prpf31*^{+/-} mice were of particular interest, where we observed the loss of basal infoldings, the formation of basal deposits beneath the RPE and vacuolization in the cytoplasm. These RPE degenerative changes were observed in heterozygous *Prpf3*^{T494M/+}*, Prpf8*^{H2309P/+} and *Prpf31*^{+/-} mice, and were more pronounced in homozygous *Prpf3*^{T494M/T494M} and *Prpf8*^{H2309P/H2309P} knockin mice⁸.

The RPE is vital for the overall well-being of the retina¹². The daily elimination of spent photoreceptor outer segment extremities (POS) is a highly coordinated process, and phagocytosis of shed POS occur on a rhythmic basis¹³. Some receptors implicated in POS phagocytosis also participate in overall retinal adhesion and its physiological rhythm¹⁴. Peak phagocytosis and retinal adhesion occur approximately 2 and 3.5 hours after light onset, respectively, and are at their minimum levels roughly 10 hours later^{13, 15, 14}. The RPE is a professional macrophage where binding and internalization of a substrate is coordinated by receptors on the RPE cell and ligands in the interphotoreceptor matrix bridging the RPE cell and phosphatidylserines at the POS surface, respectively¹⁶. Some receptors are common between phagocytosis and adhesion, but they use different ligands^{13, 14, 15, 17}. A loss of regulation of any of these important components of phagocytosis leads to vision loss in human disease as well as in rodent models^{13, 18-20}.

Here we report results of studies of RPE phagocytosis and adhesion for the *Prpf3*^{T494M/T494M}, *Prpf8*^{H2309P/H2309P} and *Prpf31*^{+/-} mouse models. Specifically, we measured phagocytosis in primary RPE cultures from 2-week-old mice. Results show a deficiency in phagocytosis, which we also demonstrate in the human RPE cell line, ARPE-19, following shRNA-mediated knockdown of *PRPF31*. Additionally, a loss of diurnal rhythmicity of phagocytosis and adhesion were detected *in vivo*. Interestingly, localization of key factors known to be involved in phagocytosis by RPE cells is modified. We conclude that the RPE is likely to be the primary site of pathogenesis in RNA splicing factor RP.

### Materials and Methods

### Animals

Animal research was performed under the protocols approved by the Institutional Animal Care and Use Committees at the Massachusetts Eye and Ear Infirmary and the Charles Darwin Animal Experimentation Ethics Committee from the Université Pierre et Marie Curie-Paris. An equal number of male and female mice were used in each of the following experiments.

### Primary RPE Cell Culture

RPE cells from 9-10-day-old animals were isolated as described¹³. Briefly, eyecups were digested with 2 mg/ml of hyaluronidase (Sigma) and the neural retina was gently peeled from the eyecup. RPE were peeled from the Bruch's membrane following digestion with 1 mg/ml trypsin (Invitrogen) and seeded onto 5-mm glass coverslips. Cells were grown to confluency for 5-10 days in DMEM with 10% FBS at 37°C, 5% CO₂.

### Primary Peritoneal Macrophage Cell Culture

Resident peritoneal macrophages were isolated as previously described²¹. Euthanized mice were pinned down to a dissection board and the fur dampened using 70% ethanol in a horizontal flow hood. The skin was delicately separated from the peritoneal wall using forceps and scissors. 5 mL of sterile PBS were injected in the abdominal cavity and the belly massaged or the whole body shaken gently for 20-30 seconds. PBS was collected slowly from the cavity and samples from 2 to 3 different animals pooled. Cells were spun for 10 min at 300 g and resuspended in 1 mL RPMI with 10% FBS. Cells were seeded in 96-well plates at 100,000-200,000 cells per well and allowed to adhere for 2 hours. Plates were shaken and wells rinsed once using sterile PBS. Cells were maintained in medium for 2-3 days at 37°C, 5% CO₂.

### Generation of Stable shRNA-PRPF31 Knockdown ARPE-19 and J774.1 Cell Lines and Cell Viability Assay

Three shRNAs were designed to human *PRPF31* or mouse *Prpf31* and cloned into pCAG-mir30 vector containing a puromycin resistance gene. The sequences for these shRNAs are as follows: human shRNA1 - 5'-TGCTGTTGACAGTGAGCGAGCAGATGAGCTCTTAGCTGATTAGTGAAGCC ACAGATGTAATCAGCTAAGAGCTCATCTGCCTGCCTACTGCCTCGGA-3' (SEQ ID NO:27), human shRNA2 - 5'-TGCTGTTGACAGTGAGCGAACCCAACCTGTCCATCATTATTAGTGAAGCC ACAGATGTAATAATGATGGACAGGTTGGGTGTGCCTACTGCCTCGGA-3' (SEQ ID NO:28), and human shRNA3 - 5'-TGCTGTTGACAGTGAGCGAGCTGAGTTCCTCAAGGTCAAGTAGTGAAGCC ACAGATGTACTTGACCTTGAGGAACTCAGCCTGCCTACTGCCTCGGA-3' (SEQ ID NO:29); mouse shRNA1 - 5'-TGCTGTTGACAGTGAGCGCTCAGTCAAGAGCATTGCCAAGTAGTGAAGCC ACAGATGTACTTGGCAATGCTCTTGACTGAATGCCTACTGCCTCGGA-3' (SEQ ID NO:30), mouse shRNA2 - 5'-TGCTGTTGACAGTGAGCGACCTGTCTGGCTTCTCTTCTACTAGTGAAGCCA CAGATGTAGTAGAAGAGAAGCCAGACAGGGTGCCTACTGCCTCGGA-3' (SEQ ID NO:31), and mouse shRNA3 - 5'-TGCTGTTGACAGTGAGCGAGCCGAGTTCCTCAAGGTCAAGTAGTGAAGCC ACAGATGTACTTGACCTTGAGGAACTCGGCCTGCCTACTGCCTCGGA-3' (SEQ ID NO:32). We also cloned an shRNA to green fluorescence protein into this vector as a non-targeted control (5'-TGCTGTTGACAGTGAGCGCTCTCCGAACGTGTATCACGTTTAGTGAAGCCA CAGATGTAAACGTGATACACGTTCGGAGATTGCCTACTGCCTCGGA -3' (SEQ ID NO:33)). The shRNA-containing vectors were linearized with *Pst*I and transfected into separate ARPE-19 (human RPE cell line, ATCC) or J774A.1 (mouse macrophage cell line, ATCC) cultures using the Amaxa electroporation kit V (Amaxa). Transfected cells were transferred to 6-well plates and 2 ml of culture medium (1:1 DMEM:F-12 with 10% FBS). Transfected cells were grown overnight at 37°C, 5% CO₂. Stable cell lines were selected with the addition of 1 (ARPE-19) to 1.25 (J774A.1) µg/ml of puromycin (Sigma) 24 hours following transfection. Media and puromycin were refreshed every 2 days for 10 days. Following selection, the four ARPE-19 and four J774A.1 knockdown lines were grown to confluence. To determine knockdown efficiency, stable lines were transiently transfected with either V5-tagged *PRPF31* in ARPE-19 cells or V5-tagged *Prpf31* cloned in a Gateway Destination vector (Invitrogen). Western blot was performed and V5-tagged *PRPF31* was quantified using an Odyssey Infrared Imager (Li-Cor). Cell viability assays were performed using the Cell Titer-Glo Luminescent Cell Viability Assay (Promega) according to manufacturer's recommendations. Briefly, ARPE-19 cells were seeded at a density of 1,000 cells/well of a 96-well cell culture plate (Corning, Cat# 3904). Cells were grown for 3 days in DMEM with 10% FBS at 37°C, 5% CO₂. Following this period, cell viability was measured by luminescence, and statistical significance was determined using the Student's t-test.

### In Vitro Phagocytosis Assays

Photoreceptor outer segments were isolated from porcine eyes obtained fresh from the slaughterhouse and covalently labeled with FITC dye (Invitrogen) for *in vitro* phagocytosis assays as previously described¹³. Confluent cultured RPE cells were challenged with ∼10 FITC-POS per cell for 1.5 hours. Non-specifically bound POS were thoroughly removed with three washes in PBS with 1 mM MgCl₂ and 0.2 mM CaCl₂. To measure internalized POS, some wells were incubated with trypan blue for 10 min to quench fluorescence of surface-bound FITC-labeled POS as previously described²⁶. Cells were fixed with ice-cold methanol and nuclei were counterstained with Hoechst 33258 (Invitrogen) or DAPI (Euromedex). Cells were imaged using a Nikon Ti2 or a Leica DM6000 Fluorescent microscope at 20X. For RPE primary cultures, FITC/DAPI ratios were calculated on all picture fields, corresponding to the number of POS per cell. FITC-POS were counted on a per cell basis for 100 cells and the average determined for three wells for ARPE-19. For peritoneal macrophages, FITC-POS and DAPI-labeled nuclei were quantified by fluorescence plate reading (Infinite M1000, Magellan 6 software, Tecan). Binding ratios were calculated by subtracting results obtained in internalization wells (trypan blue-treated) from total phagocytosis (untreated) wells. This was performed for three to six independent assays and significance was determined using the Student's t-test (*P* < 0.05).

Prior to phagocytosis, confluent cultures of the stable knockdown J774A.1 lines were opsonized using Zymosan A Bioparticles Opsonizing Reagent (Life Technologies) according to the manufacturer's protocol. Following opsonization, 1 µg of Zymosan A Bioparticles reconstituted in culture medium were applied to each culture well of a 96-well plate. The cultures were incubated at 37°C, 5% CO₂ for 1 hour. Fixation and determination of phagocytosis levels were performed as described above.

### In Vivo Diurnal Rhythm Assays

Mice were euthanized at 2 hours before light onset (-2), at light onset (0), and 2, 4, and 8 hours (+2, +4, +8) after light onset, and processed for either electron microscopy or paraffin embedding as previously described^{13, 15}. For electron microscopy all reagents were purchased from Electron Microscopy Sciences. Mice were perfused with 2% glutaraldehyde + 2% paraformaldehyde, and eyecups were transferred to perfusion buffer with the addition of 0.2 M sodium cacodylate buffer. Sixty to eighty nanometer ultrathin sections were stained with lead citrate/uranyl acetate and early phagosomes were counted from 200 nM out from the optic nerve. An early phagosome is counted if it meets the following criteria: 1) it is contained within the cytoplasm of the RPE and 2) has visible lamellar structure. For light microscopy, eyecups were fixed in formaldehyde/ethanol/acetic acid and embedded in paraffin using Ottix Plus solvent substitute (DiaPath). Five-micrometer sections were cut and the paraffin was removed using SafeSolv solvent substitute. The sections were rehydrated and incubated in 5% H₂O₂ in 1X SSC for 10 minutes under illumination to bleach pigments. After blocking non-specific signals using 10% BSA in 1X TBS, sections were stained with an anti-rhodopsin antibody (Millipore) and anti-mouse IgG-AlexaFluor 488 (Invitrogen). Nuclei were stained with DAPI, and slides mounted with Mowiol (prepared according to standard procedures). Image stacks were acquired on an Olympus FV1000 inverted confocal microscope with a 60x oil objective, a 4-time zoom and 0.41-µm step size scans and processed using the Adobe Photoshop CS6 software. Areas of at least 100 µm of uninterrupted retina/RPE were counted on 10-scan stacks. In each experiment series, phagosome counts were normalized to length of retina and averaged. Significance was determined using the Student's t-test (*P* < 0.05) and *N* = 2-5 for all experiments.

### In Vivo Retinal Adhesion Assays

We performed *in vivo* retinal adhesion assays as described¹⁴. Briefly, lens and cornea were removed from eyecups immediately postmortem in Hanks saline buffer with calcium and magnesium. A radial cut was made to the optic nerve, and the neural retina was gently peeled from the flattened eyecup. Neural retina samples were lysed in 50 mM Tris-HCl (pH 7.5), 2 mM EDTA, 150 mM NaCl, 1% Triton X-100, 0.1% SDS, and 1% Nonidet P-40, with addition of a protease inhibitors cocktail (Sigma) and 1 mM PMSF. Proteins from cleared supernatants were quantified using the Bradford assay and equal concentrations were immunoblotted for RPE65 (Abcam or Millipore) and beta-actin (Abcam or Sigma). Melanin pigments were extracted from the insoluble neural retina pellet with 20% DMSO, 2N NaOH. Samples and commercial melanin standards (Sigma) were quantified by measuring absorbance at 490 nM. Pigment abundance was normalized to protein concentration in each sample to account for different tissue yields. Bands from immunoblots were quantified using the ImageJ software v1.46r using a common sample on all blots as reference, signals were then averaged. Significance was determined using the Student's t-test (*P <* 0.05) and *N* = 3-6 for all experiments.

### Immunofluorescence Microscopy

For cryosections, eyecups were fixed in 2% paraformaldehyde and incubated in 30% sucrose overnight at 4°C. Eyecups were embedded in O.C.T. Compound (Sakura) and 10-µm sections were cut. Sections were individually incubated with primary antibodies against αν integrin (BD Biosciences), β5 integrin (Santa Cruz Biotechnology), MerTK (FabGennix), Mfg-E8 and Gas6 (R&D Systems), FAK clone 2A7 (Millipore), and Protein S (Sigma), followed by IgG-AlexaFluor 488 (Invitrogen). Nuclei were stained with DAPI, and mounted with Fluoromount (Electron Microscopy Sciences). Images were taken with a Nikon Eclipse Ti inverted fluorescence microscope using an oil immersion 60x objective. Images were processed with NIS-Elements AR software (Nikon).

For paraffin sections, animals were sacrificed at the time of the phagocytic peak and eyes were fixed in Davidson fixative for three hours at 4°C, then lens and cornea were removed. Eyecups were embedded in paraffin and 5-µm sections were cut. Sections were treated as described in the In Vivo Diurnal Rhythm Assays section and individually incubated with primary antibodies against αν integrin (Covance), β5 integrin (Santa Cruz Biotechnology), Mfg-E8, MerTK and Gas6 (R&D Systems), FAK clone 2A7 (Millipore), and Protein S (Novus Biologicals), followed by secondary antibody incubation with IgG-AlexaFluor 488 (Invitrogen). Nuclei were stained with DAPI, and slides mounted with Mowiol. Images were taken with a Leica DM6000 B Epifluorescence microscope using a 40x oil immersion objective. Images were processed with ImageJ v1.46r and Photoshop CS6 software.

### Results

### RPE phagocytosis is decreased in Prpf-mutant mice

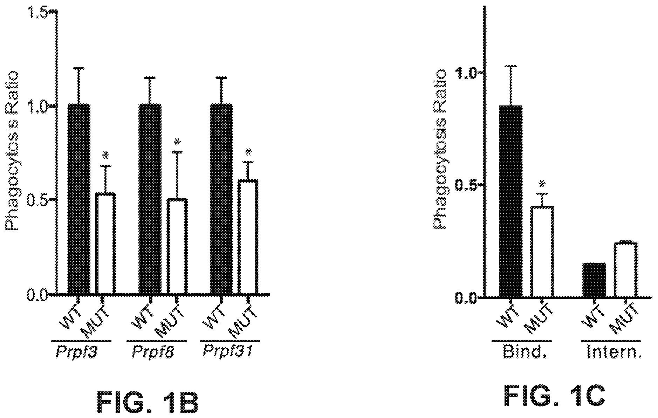

In our original characterization of the *Prpf*-mutant mice, electron microscopy identified morphological changes in the RPE of 1 to 2-year-old mutants⁸. Here, we set out to determine if functional changes precede the observed morphological changes. Since the RPE maintains phagocytic activity in culture, we established independent primary RPE cultures from 9-10-day-old *Prpf3* ^{*T494M*/}*^{T494M}, Prpf8*^{*H2309P*/}*^{H2309P}, Prpf31*^{+/-} mice, and their corresponding littermate controls. Once the cultures were confluent, we used FITC-labeled porcine POS and measured the phagocytosis following a 1.5-hour incubation. Figure 1A (panels 1-3) shows representative images of primary cultures illustrating the POS binding/uptake of RPE cells from the *Prpf*-mutant mice and their littermate controls, and demonstrating the qualitative deficiency in phagocytosis by the mutant mice. In all three mutant models, a 37-48% decrease in phagocytosis was observed (*N =* 3-5, *P* < 0.05) (Figure 1B). To account for non-specific binding of POS to the coverslips, we ran a negative control, in which the phagocytosis assay was performed on coverslips that did not contain cells. We did not observe any non-specific adhesion of the POS to the coverslips (data not shown).

We investigated if a specific step of phagocytosis between binding and internalization is preferentially perturbed in *Prpf31*^{+/-} RPE primary cultures. After performing a 1.5-hour phagocytic challenge, we treated the cells to quench the surface (bound POS) fluorescence in order to quantify solely internalized POS. POS binding was significantly reduced by 53±11% in mutant cells (*N* = 2-5, *P* < 0.05), whereas there was no significant difference in POS internalization rates between wild-type and mutant RPE cultures (Figure 1C).

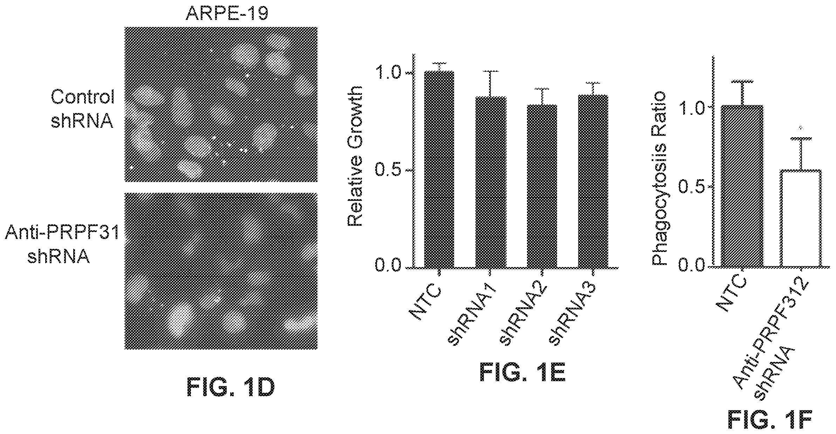

Currently, there are 64 known pathogenic mutations in *PRPF31,* of which many result in a frameshift and are degraded via the non-sense mediated decay pathway^{2, 10, 11, 22}. ARPE-19 is a spontaneously immortalized human RPE cell line that is amenable to transfection and retains the ability to phagocytose²³. To test whether mutations in the splicing factors also affect phagocytosis in a human RPE model, we created three stable ARPE-19 cell lines with shRNA-mediated knockdown of *PRPF31* using 3 distinct shRNAs directed against the 5', 3' and middle regions of the transcript (Figure 1D). We also generated a fourth stable cell line with an shRNA directed against the green fluorescent protein to use as a control. In each of the three *PRPF31* shRNA stable cell lines we achieved approximately 60-95% knockdown of *PRPF31* (data not shown). Cell viability assays of the shRNA-knockdown and non-targeted control ARPE-19 cells showed that no significant decrease occurred in association with the knockdown of *PRPF31* (Figure 1E). Phagocytosis was decreased by approximately 40% in each line tested, compared to the non-targeted control shRNA line (Figure IF). As with the phagocytosis assay performed on primary RPE, we also performed a negative control assay, and did not observe any non-specific adhesion of the POS to the coverslips (data not shown).

In order to determine if disruption of the phagocytic machinery is an RPE-specific mechanism, or can be observed in other phagocytic cells, we knocked down *Prpf31* in the mouse macrophage cell line, J774A.1. Similar to the knockdown studies in the ARPE-19 cell line, three distinct shRNAs were directed to the 5'-, 3'-termini and middle of the transcript. We used the same control shRNA as the previous studies. In each of the stable *Prpf31* cell lines, we achieved approximately 45-70% knockdown of *Prpf31* (Supplemental Figure 1A). We did not observe any phagocytosis deficiency in any of the lines tested (Supplemental Figure 1B). To ensure we did not observe non-specific POS adhesion, we performed a negative control assay as for the previous experiment series (data not shown). Identical experiments were repeated on mouse primary peritoneal macrophages isolated from *Prpf31*^{+/-} mice. Interestingly, neither step of phagocytosis, i.e. binding or internalization, nor total phagocytosis was affected in *Prpf31*-mutant compared to wild-type macrophages (Supplemental Figure 1C).

### The diurnal rhythmicity of phagocytosis is disrupted

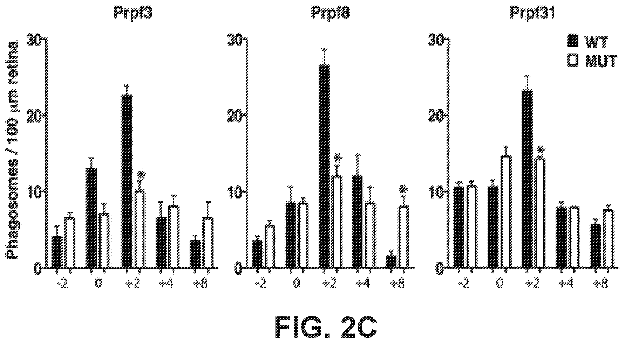

Phagocytosis of shed POS by the RPE follows a strong diurnal, synchronized rhythm peaking at 2 hours after light-onset and remaining relatively inactive for the remainder of the day¹³. We measured phagocytosis *in vivo* at 5 time-points throughout the light cycle using either electron microscopy (Figure 2A, *Prpf3-* and *Prpf8*-mutants) or immunofluorescence (Figure 2B, *Prpf31*-mutant), both recognized techniques to assess the RPE phagocytic rhythm^{13, 15}. For *Prpf3* and *Prpf8* control and mutant mice we counted early phagosomes containing lamellar structures on electron micrographs (Figure 2A, arrowheads, insets show lamellar structures). Phagocytosis rhythmicity was determined in *Prpf31*^{+/-} mice using paraffin embedding and staining for rhodopsin, and we counted phagosomes present in the RPE cell layer (Figure 2B, arrowheads). We observed a phagocytosis burst at 2 hours after light onset in all control mice, identifying 22-26 phagosomes per 100 µm of retinal section (Figure 2C, +2 time-point). In contrast, mutant mice only displayed 10-14 phagosomes at the same peak time-point. During the rest of the light:dark cycle, phagocytosis levels remain relatively low in control mice ("off-peak hours", 2-12 phagosomes/100 µm retina), and these levels are generally increased in mutant mice (6-14 phagosomes/100 µm retina). These results show a decrease in the phagocytic peak intensity in all three types of mutant mice, with a spreading of the time of the peak that lasts longer in *Prpf3-* and *Prpf8*-mutants and starts earlier in *Prpf31*-mutants. Further, the *Prpf8-*mutants have significantly more phagosomes at the off-peak time point (+8hrs), relative to the WT controls.

### Decreased retinal adhesion is observed at the peak time-point

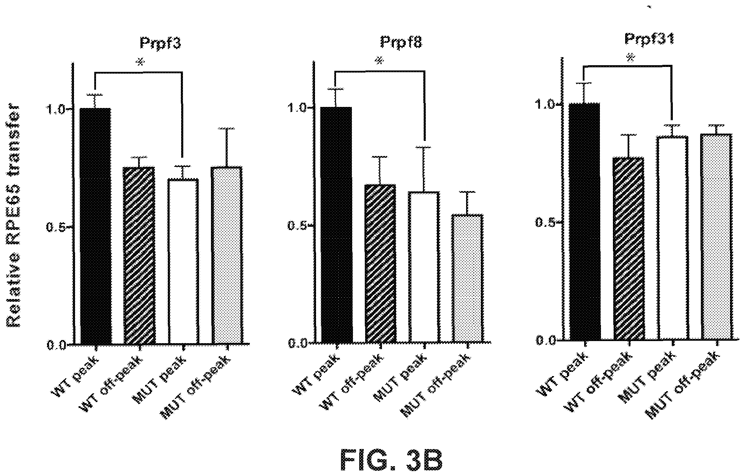

Adhesion between the RPE apical microvilli and distal tips of the POS is known to follow a synchronized rhythm with maximum strength occurring 3.5 hours after light onset, slightly after the phagocytic peak^{14, 15}. Adhesion can be determined by peeling the retina from a flattened eyecup immediately after euthanasia, then quantifying both the RPE melanin content and apical RPE protein markers, such as RPE65, transferred to the retina. Using this method, we assessed adhesion in *Prpf-*mutant mice and littermate controls at 3.5 and 8.5 hours after light onset (peak and off-peak adhesion, respectively). RPE adhesion was quantified first using a standard melanin quantification procedure¹⁴, then western blotting for the presence of RPE65 to confirm the melanin results. We noted a decrease of 56±16% (*N* = 6, *p*<0.05, variation is equal to the standard deviation) of the melanin content in the *Prpf3* ^{T494M/T494M} at peak time and no significant change in adhesion at the off-peak time-point (Figure 3A). Western blot analysis confirmed this observation with a 30±2% decrease in peak adhesion (Figure 3B). Melanin quantification in *Prpf8*^{H2309P/H2309P} mice showed that adhesion was significantly decreased by 61±28% at the peak time-point, and 51±16% at the off-peak time point (*N* = 6, *P* < 0.05 for both time-points) (Figure 3A). Western blot analysis, however, confirmed a significant 36±11% decrease only at the peak time-point (Figure 3B). In the *Prpf31*^{+/-} mice, a 15±1% decrease was observed at the peak time-point (Figure 3A), and confirmed by immunoblot analysis (*N* = 3-7, *P* < 0.05 for both panels) (Figure 3B, 14±1%).

### Localization of phagocytosis and adhesion markers

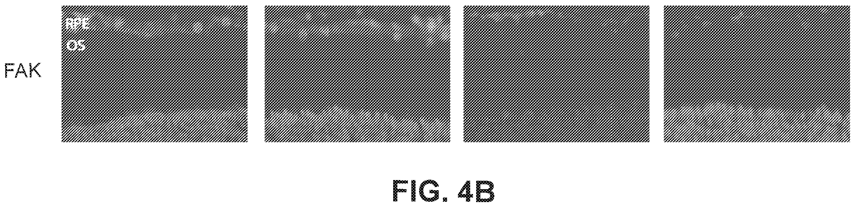

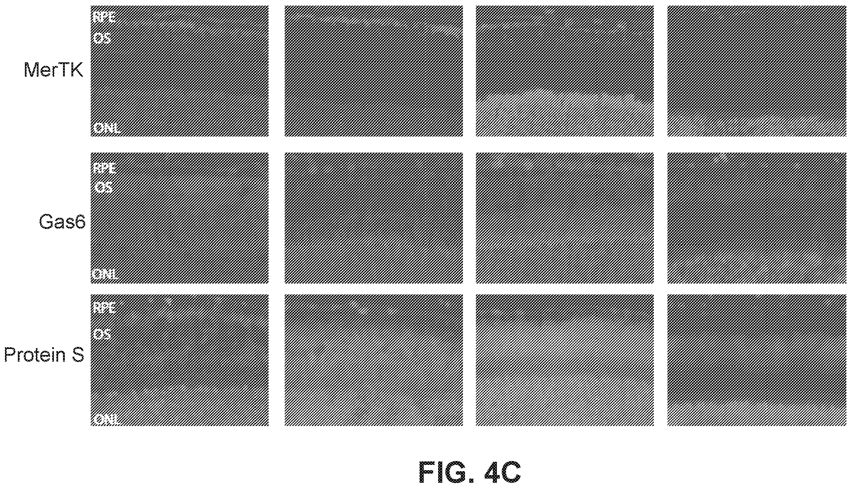

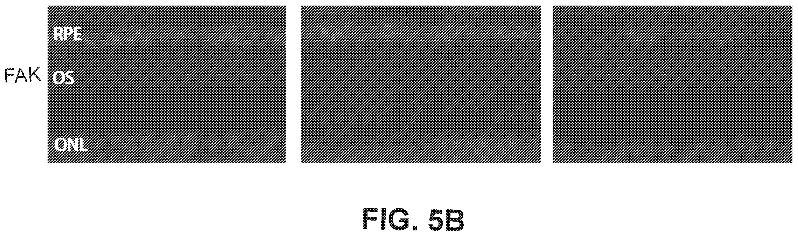

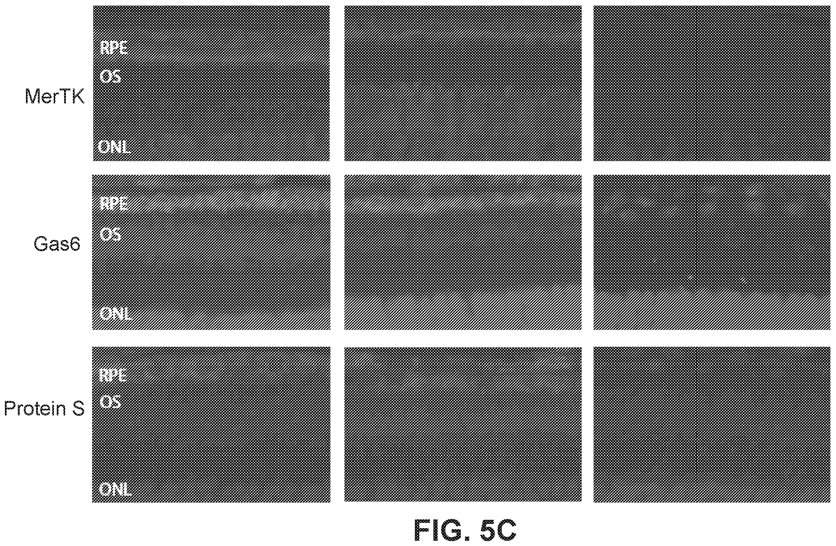

RPE cells are highly polarized, and their function is dependent upon this polarity²⁴. The specific localization of many proteins expressed in the RPE is important, and irregularities in localization may cause retinal dystrophies such as RP or Best disease^{25, 26}. Given the disruption of the diurnal rhythm of both phagocytosis and adhesion in all three *Prpf*-mutant mouse models, we set out to characterize the localization of the proteins that are known to be important for these processes. Protein localization was assayed on cryosections for *Prpf3-* and *Prpf8*-mutant mice (Fig. 4), and on paraffin sections for *Prpf31*-mutant mice (Fig. 5).

As shown previously, the main phagocytic receptors (αvβ5 integrin and MerTK) localize at the RPE apical surface²⁷, while their ligands can be expressed throughout the POS and RPE²⁸. Interestingly, extracellular ligands expressed in the interphotoreceptor matrix can be synthesized by both RPE and photoreceptor cells.

It has been shown that αvβ5-integrin with its associated ligand Mfg-E8 (milk fat globule-EGF8) are important for phagocytosis and are responsible for the diurnal rhythmicity of this function^{13, 15}. In addition, αvβ5-integrin participates in retinal adhesion and its rhythm, but with a ligand different from Mfg-E8^{14, 15, 17}. αv integrin subunits associate in complexes with several β integrin subunits in RPE cells¹⁴, therefore it is more relevant to analyze the expression of β5 integrin subunits. Thus, we probed for the αv and β5 subunits of the αvβ5 integrin receptor separately. In wild-type tissues each integrin localized primarily to the apical side of the RPE, with some expression throughout the RPE cells. In all 3 *Prpf*-mutant tissues, no change was observed in αv-integrin localization (Figures 4A, 5A). In contrast, β5 integrin localized primarily to the basal side of the RPE in the *Prpf3-* and *Prpf31*-mutant tissues, while it displayed expression equally throughout the RPE in *Prpf8*-mutant RPE cells. We did not observe a change in the localization of Mfg-E8 in either the RPE or POS, but seems to be more expressed in both *Prpf8* and *31* mutants.

The downstream signaling protein FAK (focal adhesion kinase) provides a sequential activation link between αvβ5 integrin and MerTK receptors both *in vitro* and *in vivo*^{29, 30, 13}. FAK is found throughout the RPE, and no change to this pattern was observed in the *Prpf3-* or the *Prpf31*-mutant mice (Figure 4B, 5B). *Prpf8*-mutant mice, however, showed FAK localization to the basal side of the RPE.

Phagocytosis is driven by the timely activation of MerTK via phosphorylation at the time of the activity peak^{13, 31, 32}. Gas6 and Protein S are ligands of MerTK that can stimulate uptake of shed outer segments *in vitro*³³. Both ligands are necessary to the internalization of POS as double knockout animals recapitulate the rapid retinal degeneration occurring in rats in whose MerTK receptors are absent³⁴. MerTK expression in wild-type tissues is localized to both the apical and basal membranes of the RPE, whereas MerTK is localized solely to the apical side of *Prpf31*-mutant RPE cells (Figures 4C, 5C). The first MerTK ligand Gas6 localizes to the POS and apical layer of the RPE in wild-type tissues. A decrease of expression is observed in the *Prpf3*-mutant mice POS, with diffuse expression seen throughout the RPE. *Prpf8-*mutant mice maintain Gas6 expression in the POS, but appear to lose apical localization in the RPE, also showing a diffuse expression throughout the RPE. No localization changes can be observed in Prpf31-mutant mice. The expression of the second MerTK ligand Protein S is localized specifically to the POS in wild-type and all *Prpf*-mutant mice (Figures 4C, 5C).

### Discussion

Here, we report the first functional characterization of the RPE in mice with mutations in the RNA splicing factors *Prpf3*, *8*, and *31*. As we have previously reported, the mutant mice do not experience photoreceptor degeneration, but rather morphological changes in the RPE⁸. Since RNA splicing factor RP is a late onset disease, these results are not surprising and the models afford us the ability to study the mechanisms leading to the onset of disease. Our results demonstrate that the RPE is likely to be the primary cell type affected by mutations in these 3 RNA splicing factors in the mouse, and in humans given the similar phagocytic deficiency observed in *PRPF31*-knockdown human ARPE-19 cells. While the exact mechanism of disease pathogenesis remains to be identified, these data allow for research to be focused on the RPE. For example, the identification of the RPE as the primary cell type affected in these disorders will make it possible to extend these studies to human cells, as it is now possible to generate RPE cells from human induced pluripotent stem cells (hiPSCs) of patients with inherited retinal diseases⁴²⁻⁴⁵.

### References for Example 1

[1] Will CL, Luhrmann R: Spliceosome Structure and Function. Cold Spring Harbor Perspectives in Biology 2011, 3.
[2] Liu MM, Zack DJ: Alternative splicing and retinal degeneration. Clinical Genetics 2013, 84: 142-149.
[3] Daiger SP, Bowne SJ, Sullivan LS: Perspective on genes and mutations causing retinitis pigmentosa. Archives of Ophthalmology 2007, 125:151-158.
[4] Hartong DT, Berson EL, Dryja TP: Retinitis pigmentosa. The Lancet 2006, 368: 1795-809.
[5] Sullivan LS, Bowne SJ, Reeves MJ, Blain D, Goetz K, NDifor V, Vitez S, Wang X, Tumminia SJ, Daiger SP: Prevalence of Mutations in eyeGENE Probands With a Diagnosis of Autosomal Dominant Retinitis Pigmentosa. Investigative Ophthalmology & Visual Science 2013, 54:6255-61.
[6] Nishiguchi KM, Rivolta C: Genes Associated with Retinitis Pigmentosa and Allied Diseases Are Frequently Mutated in the General Population. PLoS ONE 2012, 7.
[7] Neveling K, Collin RWJ, Gilissen C, van Huet RAC, Visser L, Kwint MP, Gijsen SJ, Zonneveld MN, Wieskamp N, de Ligt J, Siemiatkowska AM, Hoefsloot LH, Buckley MF, Kellner U, Branham KE, den Hollander AI, Hoischen A, Hoyng C, Klevering BJ, van den Born LI, Veltman JA, Cremers FPM, Scheffer H: Next-generation genetic testing for retinitis pigmentosa. Human Mutation 2012, 33:963-72.
[8] Graziotto JJ, Farkas MH, Bujakowska K, Deramaudt BM, Zhang Q, Nandrot EF, Inglehearn CF, Bhattacharya SS, Pierce EA: Three gene-targeted mouse models of RNA splicing factor RP show late-onset RPE and retinal degeneration. Investigative Ophthalmology & Visual Science 2011, 52:190-8.
[9] Graziotto JJ, Inglehearn CF, Pack MA, Pierce EA: Decreased Levels of the RNA Splicing Factor Prpf3 in Mice and Zebrafish Do Not Cause Photoreceptor Degeneration. Investigative Ophthalmology & Visual Science 2008, 49:3830-8.
[10] Rio Frio T, Wade NM, Ransijn A, Berson EL, Beckmann JS, Rivolta C: Premature termination codons in PRPF31 cause retinitis pigmentosa via haploinsufficiency due to nonsense-mediated mRNA decay. The Journal of Clinical Investigation 2008, 118:1519-31.
[11] Venturini G, Rose AM, Shah AZ, Bhattacharya SS, Rivolta C: CNOT3 is a modifier of PRPF31 mutations in retinitis pigmentosa with incomplete penetrance. PLoS Genetics 2012, 8.
[12] Kevany BM, Palczewski K: Phagocytosis of Retinal Rod and Cone Photoreceptors. Physiology 2010, 25:8-15.
[13] Nandrot EF, Kim Y, Brodie SE, Huang X, Sheppard D, Finnemann SC: Loss of synchronized retinal phagocytosis and age-related blindness in mice lacking alphavbeta5 integrin. The Journal of Experimental Medicine 2004, 200:1539-45.
[14] Nandrot EF, Anand M, Sircar M, Finnemann SC: Novel role for alphavbeta5-integrin in retinal adhesion and its diurnal peak. American Journal of Physiology Cell Physiology 2006, 290(4):C1256-62.
[15] Nandrot EF, Anand M, Almeida D, Atabai K, Sheppard D, Finnemann SC: Essential role for MFG-E8 as ligand for alphavbeta5 integrin in diurnal retinal phagocytosis. Proceedings of the National Academy of Sciences of the United States of America 2007, 104:12005-10.
[16] Ruggiero L, Connor MP, Chen J, Langen R, Finnemann SC: Diurnal, localized exposure of phosphatidylserine by rod outer segment tips in wild-type but not Itgb5-/- or Mfge8-/- mouse retina. Proceedings of the National Academy of Sciences 2012, 109:8145-8.
[17] Nandrot EF, Finnemann SC: Lack of alphavbeta5 integrin receptor or its ligand MFG-E8: distinct effects on retinal function. Ophthalmic Research 2008, 40:120-3.
[18] Nandrot E, Dufour EM, Provost AC, Péquignot MO, Bonnel S, Gogat K, Marchant D, Rouillac C, Sépulchre de Condé B, Bihoreau M-T: Homozygous Deletion in the Coding Sequence of the c-mer Gene in RCS Rats Unravels General Mechanisms of Physiological Cell Adhesion and Apoptosis. Neurobiology of Disease 2000, 7:586-99.
[19] Issa PC, Bolz HJ, Ebermann I, Domeier E, Holz FG, Scholl HP: Characterisation of severe rod-cone dystrophy in a consanguineous family with a splice site mutation in the MERTK gene. British Journal of Ophthalmology 2009, 93:920-5.
[20] Ostergaard E, Duno M, Batbayli M, Vilhelmsen K, Rosenberg T: A novel MERTK deletion is a common founder mutation in the Faroe Islands and is responsible for a high proportion of retinitis pigmentosa cases. Molecular Vision 2011, 17:1485.
[21] Davies JQ and Gordon S: Isolation and culture of murine macrophages. Basic cell culture protocols 2005: 91-103.
[22] Stenson PD, Mort M, Ball EV, Shaw K, Phillips AD, Cooper DN: The Human Gene Mutation Database: building a comprehensive mutation repository for clinical and molecular genetics, diagnostic testing and personalized genomic medicine. Human Genetics 2013:1-9.
[23] Mao Y, Finnemann S: Analysis of Photoreceptor Outer Segment Phagocytosis by RPE Cells in Culture. Retinal Degeneration. Edited by Weber BHF, Langmann T. Humana Press, 2013. pp. 285-95.
[24] Marmorstein AD: The Polarity of the Retinal Pigment Epithelium. Traffic 2001, 2:867-72.
[25] Davidson AE, Millar ID, Urquhart JE, Burgess-Mullan R, Shweikh Y, Parry N, O'Sullivan J, Maher GJ, McKibbin M, Downes SM, Lotery AJ, Jacobson SG, Brown PD, Black GC, Manson FD: Missense mutations in a retinal pigment epithelium protein, bestrophin-1, cause retinitis pigmentosa. American journal of Human Genetics 2009, 85:581-92.
[26] Lopes VS, Gibbs D, Libby RT, Aleman TS, Welch DL, Lillo C, Jacobson SG, Radu RA, Steel KP, Williams DS: The Usher 1B protein, MYO7A, is required for normal localization and function of the visual retinoid cycle enzyme, RPE65. Human Molecular Genetics 2011, 20:2560-70.
[27] Finnemann SC, Bonilha VL, Marmorstein AD, Rodriguez-Boulan E: Phagocytosis of rod outer segments by retinal pigment epithelial cells requires αvβ5 integrin for binding but not for internalization. Proceedings of the National Academy of Sciences 1997, 94:12932-7.
[28] Prasad D, Rothlin CV, Burrola P, Burstyn-Cohen T, Lu Q, Garcia de Frutos P, Lemke G: TAM receptor function in the retinal pigment epithelium. Molecular and Cellular Neuroscience 2006, 33:96-108.
[29] Finnemann SC: Focal adhesion kinase signaling promotes phagocytosis of integrin-bound photoreceptors. The EMBO Journal 2003, 22:4143-54.
[30] Qin S, Rodrigues GA: Roles of alphavbeta5, FAK and MerTK in oxidative stress inhibition of RPE cell phagocytosis. Experimental Eye Research 2012, 94:63-70.
[31] Nandrot EF, Silva KE, Scelfo C, Finnemann SC: Retinal pigment epithelial cells use a MerTK-dependent mechanism to limit the phagocytic particle binding activity of alphavbeta5 integrin. Biology of the cell / under the auspices of the European Cell Biology Organization 2012, 104:326-41.
[32] Hall MO, Obin MS, Heeb MJ, Burgess BL, Abrams TA: Both protein S and Gas6 stimulate outer segment phagocytosis by cultured rat retinal pigment epithelial cells. Experimental Eye Research 2005, 81:581-91.
[33] Burstyn-Cohen T, Lew ED, Través PG, Burrola PG, Hash JC, Lemke G: Genetic Dissection of TAM Receptor-Ligand Interaction in Retinal Pigment Epithelial Cell Phagocytosis. Neuron 2012, 76:1123-32.
[34] Yin J, Brocher J, Fischer U, Winkler C: Mutant Prpf31 causes pre-mRNA splicing defects and rod photoreceptor cell degeneration in a zebrafish model for Retinitis pigmentosa. Molecular Neurodegeneration 2011, 6:1-18.
[35] Masland RH: Cell populations of the retina: the Proctor lecture. Investigative Ophthalmology & Visual Science 2011, 52:4581-91.
[36] Finnemann SC, Nandrot EF: MerTK activation during RPE phagocytosis in vivo requires alphaVbeta5 integrin. Advances in Experimental Medicine and Biology 2006, 572:499-503.
[37] Gal A, Li Y, Thompson DA, Weir J, Orth U, Jacobson SG, Apfelstedt-Sylla E, Vollrath D: Mutations in MERTK, the human orthologue of the RCS rat retinal dystrophy gene, cause retinitis pigmentosa. Nature Genetics 2000, 26:270-1.
[38] Mackay DS, Henderson RH, Sergouniotis PI, Li Z, Moradi P, Holder GE, Waseem N, Bhattacharya SS, Aldahmesh MA, Alkuraya FS: Novel mutations in MERTK associated with childhood onset rod-cone dystrophy. Molecular Vision 2010, 16:369-377.
[39] Tschernutter M, Jenkins S, Waseem N, Saihan Z, Holder G, Bird A, Bhattacharya S, Ali R, Webster A: Clinical characterisation of a family with retinal dystrophy caused by mutation in the Mertk gene. British Journal of Ophthalmology 2006, 90:718-23.
[40] Taniguchi-Ikeda M, Kobayashi K, Kanagawa M, Yu C-c, Mori K, Oda T, Kuga A, Kurahashi H, Akman HO, DiMauro S: Pathogenic exon-trapping by SVA retrotransposon and rescue in Fukuyama muscular dystrophy. Nature 2011, 478:127-31.
[41] Dell'Angelica EC: AP-3-dependent trafficking and disease: the first decade. Current Opinion in Cell Biology 2009, 21:552-9.
[42] Farkas MH, Grant GR, White JA, Sousa ME, Consugar MB, Pierce EA: Transcriptome analyses of the human retina identify unprecedented transcript diversity and 3.5 Mb of novel transcribed sequence via significant alternative splicing and novel genes. BMC Genomics 2013, 14: 486.
[43] Buchholz DE, Pennington BO, Croze RH, Hinman CR, Coffey PJ, Clegg DO: Rapid and Efficient Directed Differentiation of Human Pluripotent Stem Cells Into Retinal Pigmented Epithelium. Stem Cells Translational Medicine 2013, 2:384-93.
[44] Meyer JS, Howden SE, Wallace KA, Verhoeven AD, Wright LS, Capowski EE, Pinilla I, Martin JM, Tian S, Stewart R: Optic Vesicle-like Structures Derived from Human Pluripotent Stem Cells Facilitate a Customized Approach to Retinal Disease Treatment. Stem Cells 2011, 29:1206-18.
[45] Singh R, Phillips MJ, Kuai D, Meyer J, Martin JM, Smith MA, Perez ET, Shen W, Wallace KA, Capowski EE: Functional analysis of serially expanded human iPS cell-derived RPE cultures. Investigative Ophthalmology & Visual Science 2013, 54:6767-78.

### Example 2. Development and functional characterization of PRPF31 knockout ARPE-19 cells using genome editing techniques

As described in Example 1, retinal pigment epithelium (RPE) was identified as the site of pathogenesis in three mutant mouse models of RNA splicing retinitis pigmentosa (RP). However, these results needed to be confirmed in human RPE. With the advent of CRISPR/Cas9 genome editing techniques, human cell line models were developed for these forms of disease. This example presents the use of CRISPR/Cas9 genome editing to knockout *PRPF31* for the first time in human cell lines and characterize the effect on RPE function.

A 20 bp guide RNA (gRNA) to exon 7 of *PRPF31* was designed and cloned into a pCAG vector containing gRNA scaffolding sequence. The gRNA vector was co-transfected with a pCAG-Cas9-GFP vector into ARPE-19 cells.

GFP positive cells were single cell sorted into individual wells of a 96 well plate and grown to confluence. DNA was isolated from each clone and the region around the predicted cut site was Sanger sequenced to identify those that exhibit correct cutting and non-homologous end joining (NHEJ). Five NHEJ lines were selected for further characterization using both qRT-PCR and phagocytosis assays to quantify FITC-labeled photoreceptor outer segment uptake with flow cytometry. These lines were maintained as confluent cultures for 3 weeks to ensure polarization and maximal expression of RPE-specific genes.

Approximately 25% of the individual clones validated following transfection showed NHEJ with deletions between 2 and 11 bases and one clone had a 1 base insertion (Figure 6). Only heterozygous indels were identified, consistent with previous reports that mutations in PRPF31 cause disease via haploinsufficiency. Expression of PRPF31 in 4 of the 5 genome edited clones was significantly (*P* < 0.05) reduced by 50-80%, as compared to the wild-type control. To confirm these changes were a result of genome editing, expression levels of the *PRPF31* modifier *CNOT3* were determined. One line had a 2-fold increase in expression, which may explain reduced levels of *PRPF31* in that line. Flow cytometry analysis of POS uptake demonstrated phagocytosis was reduced by 10-60-fold in the genome edited lines.

Currently, it is difficult to study the disease mechanism of RNA splicing factor RP in human models. We have created a human cell line model for *PRPF31-*associated disease that mimics findings in mouse models. These lines will allow us to study the disease in a more relevant model, affording us the capability to interrogate splicing more deeply. Further, we can study the effect of AAV-mediated gene augmentation of *PRPF31* on disease pathogenesis and rescue of functional deficiencies.

For example, as noted in Example 1, photoreceptor outer segments (POS) are completely renewed every 10 days by continuous growth at their bases regulated by shedding of discs at their distal tips (Young RW. The renewal of photoreceptor cell outer segments. Journal of Cell Biology. 1967;33:61-72; Young RW. Shedding of discs from rod outer segments in the rhesus monkey. JUltrastructRes. 1971;34:190-203). Phagocytosis of the spent POS material by the RPE is essential for proper retinal function, as its absence or delay leads to loss of vision (Dowling JE, Sidman RL. Inherited retinal dystrophy in the rat. Journal Cell Biology. 1962;14:73-109; Nandrot EF, Kim Y, Brodie SE, Huang X, Sheppard D, Finnemann SC. Loss of synchronized retinal phagocytosis and age-related blindness in mice lacking alphavbeta5 integrin. Journal Experimental Medicine. 2004;200:1539-45). Phagocytosis of POS was decreased in primary RPE cell cultures from 10-day old mutant mice, and this was replicated by shRNA-mediated knockdown of PRPF31 in human ARPE-19 cells (Example 1, Figure 1). The diurnal rhythmicity of phagocytosis in vivo was also lost, and the strength of the adhesion between RPE apical microvilli and POS declined at the time of peak adhesion in all 3 mutant models (Nandrot EF, Kim Y, Brodie SE, Huang X, Sheppard D, Finnemann SC. Loss of synchronized retinal phagocytosis and age-related blindness in mice lacking alphavbeta5 integrin. Journal Experimental Medicine. 2004;200:1539-45; Nandrot EF, Finnemann SC. Lack of alphavbeta5 integrin receptor or its ligand MFG-E8: distinct effects on retinal function. Ophthalmic Research. 2008;40:120-3).

### Example 3. Development and functional characterization of PRPF31 knockout human induced pluriopotent stem cells (hiPSCs) using genome editing techniques

CRISPR/Cas9 genome editing was used to knockout PRPF31 in normal hiPSCs (Hou Z, Zhang Y, Propson NE, Howden SE, Chu LF, Sontheimer EJ, Thomson JA. Efficient genome engineering in human pluripotent stem cells using Cas9 from Neisseria meningitidis. Proceedings of the National Academy of Sciences of the United States of America. 2013;110:15644-9; Xue H, Wu J, Li S, Rao MS, Liu Y. Genetic Modification in Human Pluripotent Stem Cells by Homologous Recombination and CRISPR/Cas9 System. Methods Molecular Biology. 2014Peters DT, Cowan CA, Musunuru K. Genome editing in human pluripotent stem cells. StemBook. Cambridge (MA) 2013; Ding Q, Regan SN, Xia Y, Oostrom LA, Cowan CA, Musunuru K. Enhanced efficiency of human pluripotent stem cell genome editing through replacing TALENs with CRISPRs. Cell Stem Cell. 2013;12:393-4. PMCID: 3925309) as described above in Example 2.

The development of hiPSC technology now makes it possible to determine if human RPE cells are similarly affected by mutations in RNA splicing factor genes, since hiPSCs can be readily differentiated into RPE cells (see Example 1, Singh R, Phillips MJ, Kuai D, Meyer JT, Martin J, Smith M, Shen W, Perez ET, Wallace KA, Capowski EE, Wright L, Gamm DM. Functional analysis of serially expanded human iPS cell-derived RPE cultures. Investigative Ophthalmology & Visual Science. 2013;54:6767-78; Buchholz DE, Hikita ST, Rowland TJ, Friedrich AM, Hinman CR, Johnson LV, Clegg DO. Derivation of functional retinal pigmented epithelium from induced pluripotent stem cells. Stem Cells. 2009;27:2427-34; Okamoto S, Takahashi M. Induction of retinal pigment epithelial cells from monkey iPS cells. Investigative Ophthalmology & Visual Science. 2011;52:8785-90; Ukrohne TU, Westenskow PD, Kurihara T, Friedlander DF, Lehmann M, Dorsey AL, Li W, Zhu S, Schultz A, Wang J, Siuzdak G, Ding S, Friedlander M. Generation of retinal pigment epithelial cells from small molecules and OCT4 reprogrammed human induced pluripotent stem cells. Stem cells translational medicine. 2012;1:96-109; Westenskow PD, Moreno SK, Krohne TU, Kurihara T, Zhu S, Zhang ZN, Zhao T, Xu Y, Ding S, Friedlander M. Using flow cytometry to compare the dynamics of photoreceptor outer segment phagocytosis in iPS-derived RPE cells. Investigative Ophthalmology & Visual Science. 2012;53:6282-90; Buchholz DE, Pennington BO, Croze RH, Hinman CR, Coffey PJ, Clegg DO. Rapid and efficient directed differentiation of human pluripotent stem cells into retinal pigmented epithelium. Stem cells translational medicine. 2013;2:384-93). hiPSC-derived RPE cells share many features with native RPE cells, including functional tight junctions, phagocytosis of POS, and polarization (*Ibid*).

To obtain hiPSC-RPE, embryoid bodies (EBs) are generated, adhered to laminin-coated plates and cultured in retinal differentiation medium (RDM) for 60-90 days. Regions of pigmented cells will then be microdissected, dissociated and passed onto transwell inserts according to established protocols (Singh R, Phillips MJ, Kuai D, Meyer JT, Martin J, Smith M, Shen W, Perez ET, Wallace KA, Capowski EE, Wright L, Gamm DM. Functional analysis of serially expanded human iPS cell-derived RPE cultures. Investigative Ophthalmology & Visual Science. 2013;54:6767-78; Phillips MJ, Wallace KA, Dickerson SJ, Miller MJ, Verhoeven AD, Martin JM, Wright LS, Shen W, Capowski EE, Percin EF, Perez ET, Zhong X, Canto-Soler MV, Gamm DM. Blood-derived human iPS cells generate optic vesicle-like structures with the capacity to form retinal laminae and develop synapses. Investigative Ophthalmology & Visual Science. 2012;53:2007-19). Cells will then be cultured for an additional 30-60 days, when pigmented monolayers reform. Prior to use in experiments the transepithelial resistance (TER) of the hiPSC-RPE monolayers grown on Transwell inserts will be measured; only those cultured with TER > 150Ωcm² will be selected for further study. For every experiment, we will include duplicates for each mutation of interest and wild-type control cells, which will be cultured and analyzed in parallel. The structure and function of the hiPSC-derived RPE cells will be characterized using several methods:
*Structure.* Light microscopy and electron microscopy is used to assess polarization, including formation of apical processes and basolateral infoldings (Exaple 1, Garland DL, Fernandez-Godino R, Kaur I, Speicher KD, Harnly JM, Lambris JD, Speicher DW, Pierce EA. Mouse genetics and proteomic analyses demonstrate a critical role for complement in a model of DHRD/ML, an inherited macular degeneration. Human Molecular Genetics. 2013;Sep 4. [Epub ahead of print]; Liu Q, Lyubarsky A, Skalet JH, Pugh EN, Jr., Pierce EA. RP1 is required for the correct stacking of outer segment discs. Investigative Ophthalmology & Visual Science. 2003;44:4171-83).
*Phagocytosis.* As described above, primary cultures of RPE cells from the *Prpf3*^{T494M/T494M}, *Prpf8*^{H2309P/H2309P}, and *Prpf31*^{+/-} mice have significantly decreased ability to phagocytose POS (Figure 1). We will assess the phagocytic function of hiPSC-derived RPE cells using established techniques (see Example 1; Finnemann SC, Bonilha VL, Marmorstein AD, Rodriguez-Boulan E. Phagocytosis of rod outer segments by retinal pigment epithelial cells requires alpha(v)beta5 integrin for binding but not for internalization. ProcNatlAcadSciUSA. 1997;94:12932-7; Singh R, Shen W, Kuai D, Martin JM, Guo X, Smith MA, Perez ET, Phillips MJ, Simonett JM, Wallace KA, Verhoeven AD, Capowski EE, Zhang X, Yin Y, Halbach PJ, Fishman GA, Wright LS, Pattnaik BR, Gamm DM. iPS cell modeling of Best disease: insights into the pathophysiology of an inherited macular degeneration. Human Molecular Genetics. 2013;22:593-60.)

To assess the polarity of the hiPSC-derived RPE cells, vibratome sections of stably transfected cells grown on Transwells are immunostained with antibodies against established RPE cell markers using established techniques (Nandrot EF, Kim Y, Brodie SE, Huang X, Sheppard D, Finnemann SC. Loss of synchronized retinal phagocytosis and age-related blindness in mice lacking alphavbeta5 integrin. Journal Experimental Medicine. 2004;200:1539-45; Nandrot EF, Finnemann SC. Lack of alphavbeta5 integrin receptor or its ligand MFG-E8: distinct effects on retinal function. Ophthalmic Research. 2008;40:120-3; Finnemann SC, Nandrot EF. MerTK activation during RPE phagocytosis in vivo requires alphaVbeta5 integrin. Advances Experimental Medicine Biology. 2006;572:499-503). The stained cells will be evaluated by confocal microscopy, and the distribution and relative amounts of the marker proteins will be compared in mutant and control hiPSC-derived RPE cells. The levels of these RPE cell markers will also be evaluated in differentiated cells via western blotting (Nandrot EF, Kim Y, Brodie SE, Huang X, Sheppard D, Finnemann SC. Loss of synchronized retinal phagocytosis and age-related blindness in mice lacking alphavbeta5 integrin. Journal Experimental Medicine. 2004;200:1539-45).

Changes in RPE phenotype observed in the genome edited hiPSCs are confirmed using hiPSCs from patients with RNA splicing factor RP. Patients and families with RP due to mutations in the *PRPF31* gene have been identified, and hiPSCs are generated using fibroblasts from one affected and one unaffected family member from each of 3 families. Briefly, fibroblasts are reprogrammed using non-integrating, oriP-containing plasmid vectors encoding seven reprogramming factors (OCT4, SOX2, NANOG, LIN28, c-Myc, KLF4, and SV40 large T-antigen), as described ( Yu J, Hu K, Smuga-Otto K, Tian S, Stewart R, Slukvin II, Thomson JA. Human induced pluripotent stem cells free of vector and transgene sequences. Science. 2009;324:797-801). hiPSC lines with normal karyotypes and that are confirmed to be pluripotent by teratoma studies and expression of the pluripotency markers OCT4, SSEA4, NANOG and TRA-1-81 would be selected for further study (Singh R, Shen W, Kuai D, Martin JM, Guo X, Smith MA, Perez ET, Phillips MJ, Simonett JM, Wallace KA, Verhoeven AD, Capowski EE, Zhang X, Yin Y, Halbach PJ, Fishman GA, Wright LS, Pattnaik BR, Gamm DM. iPS cell modeling of Best disease: insights into the pathophysiology of an inherited macular degeneration. Human Molecular Genetics. 2013;22:593-607; Singh R, Phillips MJ, Kuai D, Meyer JT, Martin J, Smith M, Shen W, Perez ET, Wallace KA, Capowski EE, Wright L, Gamm DM. Functional analysis of serially expanded human iPS cell-derived RPE cultures. Investigative Ophthalmology & Visual Science. 2013;54:6767-78; Meyer JS, Howden SE, Wallace KA, Verhoeven AD, Wright LS, Capowski EE, Pinilla I, Martin JM, Tian S, Stewart R, Pattnaik B, Thomson JA, Gamm DM. Optic vesicle-like structures derived from human pluripotent stem cells facilitate a customized approach to retinal disease treatment. Stem Cells. 2011;29:1206-18). After confirming that each hiPSC line carries the expected mutation, hiPSC-derived RPE function is characterized in cells from patients and compared to unaffected family members using the techniques described above.

### Example 4. AAV Vectors for Gene Augmentation Therapy

The identification of RPE cells as likely to be the primary cells affected in RNA splicing factor RP (see Example 1) creates an opportunity to use gene augmentation therapy for diseases caused by mutations in *PRPF31.* To achieve this goal, we have developed AAV vectors for expressing human *PRPF31* in RPE cells, and tested the ability of the AAV-delivered *PRPF31* to ameliorate the phenotype in cultured RPE cells, and then in *Prpf31*^{+/-} mice *in vivo.* AAV is the preferred gene delivery vector for retinal disorders based on the success of clinical trials of gene therapy for *RPE65* LCA and choroideremia, as well as other clinical and pre-clinical studies (Maguire AM, Simonelli F, Pierce EA, Pugh EN, Jr., Mingozzi F, Bennicelli J, Banfi S, Marshall KA, Testa F, Surace EM, Rossi S, Lyubarsky A, Arruda VR, Konkle B, Stone E, Sun J, Jacobs J, Dell'Osso L, Hertle R, Ma JX, Redmond TM, Zhu X, Hauck B, Zelenaia O, Shindler KS, Maguire MG, Wright JF, Volpe NJ, McDonnell JW, Auricchio A, High KA, Bennett J. Safety and efficacy of gene transfer for Leber's congenital amaurosis. New England Journal of Medicine. 2008;358:2240-8. PMCID: 2829748; Bainbridge JW, Smith AJ, Barker SS, Robbie S, Henderson R, Balaggan K, Viswanathan A, Holder GE, Stockman A, Tyler N, Petersen-Jones S, Bhattacharya SS, Thrasher AJ, Fitzke FW, Carter BJ, Rubin GS, Moore AT, Ali RR. Effect of gene therapy on visual function in Leber's congenital amaurosis. New England Journal of Medicine. 2008;358:2231-9; Cideciyan AV, Aleman TS, Boye SL, Schwartz SB, Kaushal S, Roman AJ, Pang JJ, Sumaroka A, Windsor EA, Wilson JM, Flotte TR, Fishman GA, Heon E, Stone EM, Byrne BJ, Jacobson SG, Hauswirth WW. Human gene therapy for RPE65 isomerase deficiency activates the retinoid cycle of vision but with slow rod kinetics. Proceedings National Academy Sciences USA. 2008;105:15112-7. PMCID: 2567501; Maguire AM, High KA, Auricchio A, Wright JF, Pierce EA, Testa F, Mingozzi F, Bennicelli JL, Ying GS, Rossi S, Fulton A, Marshall KA, Banfi S, Chung DC, Morgan JI, Hauck B, Zelenaia O, Zhu X, Raffini L, Coppieters F, De Baere E, Shindler KS, Volpe NJ, Surace EM, Acerra C, Lyubarsky A, Redmond TM, Stone E, Sun J, McDonnell JW, Leroy BP, Simonelli F, Bennett J. Age-dependent effects of RPE65 gene therapy for Leber's congenital amaurosis: a phase 1 dose-escalation trial. Lancet. 2009;374:1597-605; Jacobson SG, Cideciyan AV, Ratnakaram R, Heon E, Schwartz SB, Roman AJ, Peden MC, Aleman TS, Boye SL, Sumaroka A, Conlon TJ, Calcedo R, Pang JJ, Erger KE, Olivares MB, Mullins CL, Swider M, Kaushal S, Feuer WJ, Iannaccone A, Fishman GA, Stone EM, Byrne BJ, Hauswirth WW. Gene therapy for leber congenital amaurosis caused by RPE65 mutations: safety and efficacy in 15 children and adults followed up to 3 years. Archives Ophthalmology. 2012;130:9-24; Bennett J, Ashtari M, Wellman J, Marshall KA, Cyckowski LL, Chung DC, McCague S, Pierce EA, Chen Y, Bennicelli JL, Zhu X, Ying GS, Sun J, Wright JF, Auricchio A, Simonelli F, Shindler KS, Mingozzi F, High KA, Maguire AM. AAV2 gene therapy readministration in three adults with congenital blindness. Science translational medicine. 2012;4:120ra15; Bowles DE, McPhee SW, Li C, Gray SJ, Samulski JJ, Camp AS, Li J, Wang B, Monahan PE, Rabinowitz JE, Grieger JC, Govindasamy L, Agbandje-McKenna M, Xiao X, Samulski RJ. Phase 1 gene therapy for Duchenne muscular dystrophy using a translational optimized AAV vector. Molecular therapy : the journal of the American Society of Gene Therapy. 2012;20:443-55. PMCID: 3277234; Maclachlan TK, Lukason M, Collins M, Munger R, Isenberger E, Rogers C, Malatos S, Dufresne E, Morris J, Calcedo R, Veres G, Scaria A, Andrews L, Wadsworth S. Preclinical safety evaluation of AAV2-sFLT01- a gene therapy for age-related macular degeneration. Molecular Therapy. 2011;19:326-34. PMCID: 3034852; Nathwani AC, Tuddenham EG, Rangarajan S, Rosales C, McIntosh J, Linch DC, Chowdary P, Riddell A, Pie AJ, Harrington C, O'Beirne J, Smith K, Pasi J, Glader B, Rustagi P, Ng CY, Kay MA, Zhou J, Spence Y, Morton CL, Allay J, Coleman J, Sleep S, Cunningham JM, Srivastava D, Basner-Tschakarjan E, Mingozzi F, High KA, Gray JT, Reiss UM, Nienhuis AW, Davidoff AM. Adenovirus-associated virus vector-mediated gene transfer in hemophilia B. New England Journal of Medicine. 2011;365:2357-65. PMCID: 3265081).

AAV has an exceptional safety record in early phase clinical studies and also poses less risk of genotoxicity compared to other vector systems since AAV genomes are stable in an episomal form in terminally differentiated cells such as photoreceptor and RPE cells (Yang GS, Schmidt M, Yan Z, Lindbloom JD, Harding TC, Donahue BA, Engelhardt JF, Kotin R, Davidson BL. Virus-mediated transduction of murine retina with adeno-associated virus: effects of viral capsid and genome size. JVirol. 2002;76:7651-60; Acland GM, Aguirre GD, Bennett J, Aleman TS, Cideciyan AV, Bennicelli J, Dejneka NS, Pearce-Kelling SE, Maguire AM, Palczewski K, Hauswirth WW, Jacobson SG. Long-term restoration of rod and cone vision by single dose rAAV-mediated gene transfer to the retina in a canine model of childhood blindness. Molecular Therapy. 2005;12:1072-82).

Several AAV vector systems for *PFPF31* are developed, with different promoter choices and capsid serotypes. With regard to promoters, vectors can include promoters that drive expression in many cell types (e.g., CAG or CASI), RPE cells (e.g., promotors for RPE-specific proteins such as VMD2, RPE65, RLBP1, RGR, or TIMP3) and photoreceptor cells (RHO) (Esumi N, Oshima Y, Li Y, Campochiaro PA, Zack DJ. Analysis of the VMD2 promoter and implication of E-box binding factors in its regulation. Journal Biological Chemistry. 2004;279:19064-73; Guziewicz KE, Zangerl B, Komaromy AM, Iwabe S, Chiodo VA, Boye SL, Hauswirth WW, Beltran WA, Aguirre GD. Recombinant AAV-Mediated BEST1 Transfer to the Retinal Pigment Epithelium: Analysis of Serotype-Dependent Retinal Effects. PLoS One. 2013;8:e75666; Allocca M, Mussolino C, Garcia-Hoyos M, Sanges D, Iodice C, Petrillo M, Vandenberghe LH, Wilson JM, Marigo V, Surace EM, Auricchio A. Novel adeno-associated virus serotypes efficiently transduce murine photoreceptors. J Virol. 2007;81:11372-80). The components of the AAV vectors are synthesized using codon-optimized *PRPF31* sequences to improve the level and duration of gene expression (I11 CR, Chiou HC. Gene therapy progress and prospects: recent progress in transgene and RNAi expression cassettes. Gene Therapy. 2005;12:795-802; Foster H, Sharp PS, Athanasopoulos T, Trollet C, Graham IR, Foster K, Wells DJ, Dickson G. Codon and mRNA sequence optimization of microdystrophin transgenes improves expression and physiological outcome in dystrophic mdx mice following AAV2/8 gene transfer. Molecular Therapy. 2008;16:1825-32; Sack BK, Merchant S, Markusic DM, Nathwani AC, Davidoff AM, Byrne BJ, Herzog RW. Transient B cell depletion or improved transgene expression by codon optimization promote tolerance to factor VIII in gene therapy. PLoS One. 2012;7:e37671). In preliminary studies, codon optimized PRPF31 produced full-length PRPF31 protein in ARPE-19 cells. The vectors prepared encode minimal vector genome necessary to achieve optimal expression. Since we are interested primarily in transducing RPE cells, we will use AAV2 as a control serotype, as this vector is known to transduce cultured monolayer cells and transduced the RPE well *in vivo* (Pang JJ, Lauramore A, Deng WT, Li Q, Doyle TJ, Chiodo V, Li J, Hauswirth WW. Comparative analysis of in vivo and in vitro AAV vector transduction in the neonatal mouse retina: effects of serotype and site of administration. Vision Research. 2008;48:377-85; Vandenberghe LH, Bell P, Maguire AM, Cearley CN, Xiao R, Calcedo R, Wang L, Castle MJ, Maguire AC, Grant R, Wolfe JH, Wilson JM, Bennett J. Dosage thresholds for AAV2 and AAV8 photoreceptor gene therapy in monkey. Science translational medicine. 2011;3:88ra54; Tolmachova T, Tolmachov OE, Barnard AR, de Silva SR, Lipinski DM, Walker NJ, Maclaren RE, Seabra MC. Functional expression of Rab escort protein 1 following AAV2-mediated gene delivery in the retina of choroideremia mice and human cells ex vivo. Journal of Molecular Medicine. 2013;91:825-37. PMCID: 3695676). Vector preparations are generated and purified using established techniques (Vandenberghe LH, Bell P, Maguire AM, Cearley CN, Xiao R, Calcedo R, Wang L, Castle MJ, Maguire AC, Grant R, Wolfe JH, Wilson JM, Bennett J. Dosage thresholds for AAV2 and AAV8 photoreceptor gene therapy in monkey. Science translational medicine. 2011;3:88ra54, Lock M, Alvira M, Vandenberghe LH, Samanta A, Toelen J, Debyser Z, Wilson JM. Rapid, simple, and versatile manufacturing of recombinant adeno-associated viral vectors at scale. Human Gene Therapy. 2010;21:1259-71. PMCID: 2957274). Titration is performed by Taqman qPCR with primer-probe sets directed toward the poly-adenylation signal in the vector genome.

To study PRPF31 expression in cultured cells, the PRPF31 mutant and control ARPE-19 cells are cultured on Transwell filters, as described in Example 1. Cells are treated with the desired amount of AAV-*PRPF31* vectors, and cultured for an additional 11-14 days. Wild-type RPE cells treated with AAV-*PRPF31,* and *PRFP31*^{+/-} cells treated with *AAV-EGFP* are used as controls. The effects of the AAV-*PRPF31* treatment are evaluated using several approaches. The production of full-length PRPF31 protein is evaluated by immunofluorescence microscopy and western blotting experiments 2-4 days following transduction (Liu Q, Zhou J, Daiger SP, Farber DB, Heckenlively JR, Smith JE, Sullivan LS, Zuo J, Milam AH, Pierce EA. Identification and subcellular localization of the RP1 protein in human and mouse photoreceptors. Investigative Ophthalmology & Visual Science. 2002;43:22-32; Liu Q, Zuo J, Pierce EA. The retinitis pigmentosa 1 protein is a photoreceptor microtubule-associated protein. Journal Neuroscience. 2004;24:6427-36; Falk MJ, Zhang Q, Nakamaru-Ogiso E, Kannabiran C, Fonseca-Kelly Z, Chakarova C, Audo I, Mackay DS, Zeitz C, Borman AD, Staniszewska M, Shukla R, Palavalli L, Mohand-Said S, Waseem NH, Jalali S, Perin JC, Place E, Ostrovsky J, Xiao R, Bhattacharya SS, Consugar M, Webster AR, Sahel JA, Moore AT, Berson EL, Liu Q, Gai X, Pierce EA. NMNAT1 mutations cause Leber congenital amaurosis. Nature Genetics. 2012;44:1040-5). Gene transfer is assayed by qPCR for vector genomes. Restoration of the normal phagocytic activity of the mutant cells is measured by treatment with FITC-labeled POS, using established techniques (Example 1, Finnemann SC, Bonilha VL, Marmorstein AD, Rodriguez-Boulan E. Phagocytosis of rod outer segments by retinal pigment epithelial cells requires alpha(v)beta5 integrin for binding but not for internalization. ProcNatlAcadSciUSA. 1997;94:12932-7; Singh R, Shen W, Kuai D, Martin JM, Guo X, Smith MA, Perez ET, Phillips MJ, Simonett JM, Wallace KA, Verhoeven AD, Capowski EE, Zhang X, Yin Y, Halbach PJ, Fishman GA, Wright LS, Pattnaik BR, Gamm DM. iPS cell modeling of Best disease: insights into the pathophysiology of an inherited macular degeneration. Human Molecular Genetics. 2013;22:593-607).

To study *PRPF31* expression and function in Prpf31^{+/-} mutant mice, AAV-mediated delivery of *PRPF31* is used to treat the defective phagocytosis in *Prpf31*^{+/-} mice *in vivo.* For these studies, the optimal doses of the AAV-*PRPF31* vectors identified in cell culture studies is injected sub-retinally into one eye of *Prpf31*^{+/-} mice. Eyes are harvested 1 month after injection and evaluated for expression and localization of the full-length PRPF31 protein using immunofluorescence and western blotting assays (Liu Q, Lyubarsky A, Skalet JH, Pugh EN, Jr., Pierce EA. RP1 is required for the correct stacking of outer segment discs. Investigative Ophthalmology & Visual Science. 2003;44:4171-83; Liu Q, Saveliev A, Pierce EA. The severity of retinal degeneration in Rplh gene-targeted mice is dependent on genetic background. Investigative Ophthalmology & Visual Science. 2009;50:1566-74; Liu Q, Collin RW, Cremers FP, den Hollander AI, van den Born LI, Pierce EA. Expression of Wild-Type Rp1 Protein in Rp1 Knock-in Mice Rescues the Retinal Degeneration Phenotype. PLoS One. 2012;7:e43251).

The ability of AAV-delivered *PRPF31* to prevent and/or rescue the loss of rhythmicity of RPE phagocytosis is assessed at 2 hours before light onset (-2), at light onset (0), and 2, 4, and 6 (+2, +4, +6) hours after light onset using established techniques for immunofluorescent staining for rhodopsin and detection of phagosomes located in the RPE cell layer (Nandrot EF, Kim Y, Brodie SE, Huang X, Sheppard D, Finnemann SC. Loss of synchronized retinal phagocytosis and age-related blindness in mice lacking alphavbeta5 integrin. Journal Experimental Medicine. 2004;200:1539-45; Nandrot EF, Finnemann SC. Lack of alphavbeta5 integrin receptor or its ligand MFG-E8: distinct effects on retinal function. Ophthalmic Research. 2008;40:120-3). We evaluate the treated retinas for evidence of phenotype rescue initially at 1 month and 2 months following AAV-*PRPF31* injection in these animals. To evaluate for evidence of prevention of the RPE degeneration, mice are treated at 1 month of age, and the ultrastructure of the RPE is evaluated for phenotypic rescue at 5, 8 and 11 months following AAV-*PRPF31* injection (Graziotto JJ, Farkas MH, Bujakowska K, Deramaudt BM, Zhang Q, Nandrot EF, Inglehearn CF, Bhattacharya SS, Pierce EA. Three gene-targeted mouse models of RNA splicing factor RP show late-onset RPE and retinal degeneration. Investigative Ophthalmology & Visual Science. 2011;52:190-8). Based on data from asymptomatic carriers of PRPF31 mutations, we anticipate that even a modest increase in PRPF31 level in the treated RPE cells will be therapeutic (Rio FT, Wade NM, Ransijn A, Berson EL, Beckmann JS, Rivolta C. Premature termination codons in PRPF31 cause retinitis pigmentosa via haploinsufficiency due to nonsense-mediated mRNA decay. Journal Clinical Investigation. 2008;118:1519-31; Vithana EN, Abu-Safieh L, Pelosini L, Winchester E, Hornan D, Bird AC, Hunt DM, Bustin SA, Bhattacharya SS. Expression of PRPF31 mRNA in patients with autosomal dominant retinitis pigmentosa: a molecular clue for incomplete penetrance? Investigative Ophthalmology & Visual Science. 2003;44:4204-9).

### Example 5. AAV-mediated gene augmentation therapy to ameliorates the defective phagocytosis phenotype in cultured RPE cells

As described above, there is good evidence that mutations in *PRPF31* cause disease via haploinsuffiency, and thus that this form of dominant RP is amenable to treatment with gene augmentation therapy (Wang et al., American Journal Medical Genetics A. 2003;121A:235-9; Xia et al., Molecular Vision. 2004;10:361-5; Abu-Safieh et al., MolVis. 2006;12:384-8; Rivolta et al., Human Mutation. 2006;27:644-53; Sullivan et al., Investigative Ophthalmology & Visual Science. 2006;47:4579-88; Rio et al., Human Mutation. 2009;30:1340-7). Consistent with this hypothesis, the level of *PRPF31* expression from the wild-type allele correlates with the severity of disease in patients with mutations in *PRPF31* (Rio et al., Journal Clinical Investigation. 2008;118:1519-31; Venturini et al., PLoS genetics. 2012;8:e1003040; Rose et al., Scientific reports. 2016;6:19450). To test this hypothesis, we used AAV-mediated gene augmentation therapy to ameliorate the phenotype in cultured RPE cells.

For these studies, we generated an AAV.CASI.PRPF31 viral vector, and showed that this can produce full length PRPF31 protein in cultured cells. The sequence of this vector is as follows:

| | |
|---|---|
| ITR - pZac2.1 inverted terminal repeat - | nts 1-130 and 3291-3420 |
| Promoter - CASI | nts 197-1252 |
| Tag - V5 | nts 1259-1309 |
| Insert - PRPF31 | nts 1319-2818 |
| polyA sequence - rabbit β-globin | nts 2825-3211 |

We next tested the ability of the AAV.CASI.PRPF31 to correct the defective phagocytosis phenotype in genome-edited *PRFP31*-deficient ARPE-19 cells. For these experiments, genome-edited *PRPF31* mutant (GE31) ARPE-19 cells were transduced with AAV.CASI.*PRPF31* at a multiplicity of infection (MOI) of 0, 10,000, and 15,000. Following transduction, each replicate was incubated with 1x10⁶ FITC-labeled photoreceptor outer segments (FITC-POS) for 1 hour at 37°C. FITC-POS uptake was determined by counting FITC positive cells using flow cytometry. Treatment of the GE31 mutant cell line resulted in increased FITC-POS uptake, in a dose-dependent fashion (Figure 7). This result confirms the potential of gene augmentation therapy to be used for treating *PRPF31*-associated retinal degeneration.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. An Adeno-associated virus type 2 (AAV2) vector comprising a sequence encoding human PRPF31, operably linked to a promotor that drives expression in retinal pigment epithelial (RPE) cells, wherein the PRPF31 sequence is or comprises, or encodes the same protein as nts 1319-2818 of SEQ ID NO:34.

2. The vector of claim 1, wherein the promotor is a CAG, CASI, RPE65 or VMD2 promotor.

3. The vector of claim 1, wherein the PRPF31 sequence is codon optimized.

4. The vector of any one of the preceding claims, further comprising an AAV capsid polypeptide having an amino acid sequence selected from the group consisting of SEQ ID NOs: 1, 3, 5, 7, 9, 11, 13, 15, 19, 20, 21, 22, 23, 24, 25 and 26.

5. A pharmaceutical composition comprising the vector of any one of claims 1-4, formulated for delivery via sub-retinal injection.

6. A pharmaceutical composition consisting essentially of a gene delivery system comprising the vector of any one of claims 1 to 4 in a diluent.

7. A pharmaceutical composition consisting essentially of a gene delivery system comprising the vector of any one of claims 1 to 4 embedded in a slow release matrix.

8. The pharmaceutical composition of any one of claims 6 and 7, wherein the gene delivery system further comprises helper viruses, proteins, and/or lipids.

9. The pharmaceutical composition comprising one or more recombinant cells comprising the vector of claims of any one of claims 1 to 4.

10. The vector of claims 1-4, for use in treating retinitis pigmentosa caused by mutations in PRPF31 in the eye of a human subject.

11. The vector of claims 1-4, for use in increasing expression of PRPF31 in the eye of a human subject.

12. The pharmaceutical composition of any one of claims 5 to 9, for use in treating retinitis pigmentosa caused by mutations in PRPF31 in the eye of a human subject.

13. The pharmaceutical composition of any one of claims 5 to 9, for use in increasing expression of PRPF31 in the eye of a human subject.

## Patentansprüche

1. Adenoassoziierter Virustyp 2 (AAV2) Vektor, der eine Sequenz zur Codierung des menschlichen PRPF31 umfasst, wirksam mit einem Promotor verbunden, der Expression in retinalen Pigmentepithelzellen (RPE-Zellen) antreibt, wobei die PRPF31-Sequenz dasselbe Protein wie nts 1319-2818 von SEQ ID NO:34 ist oder umfasst oder codiert.

2. Vektor nach Anspruch 1, wobei der Promotor ein CAG-, CASI-, RPE65- oder VMD2-Promotor ist.

3. Vektor nach Anspruch 1, wobei die PRPF31 -Sequenz ist codon-optimiert ist.

4. Vektor nach einem der vorhergehenden Ansprüche, der ferner ein AAV-Kapsidpolypeptid umfasst, das eine Aminosäuresequenz aufweist, die aus der Gruppe selektiert ist, die aus folgenden SEQ ID NOs besteht: 1, 3, 5, 7, 9, 11, 13, 15, 19, 20, 21, 22, 23, 24, 25 und 26.

5. Pharmazeutische Zusammensetzung, die den Vektor irgendeines der Ansprüche 1-4 umfasst, die zur Abgabe über subretinale Injektion formuliert ist.

6. Pharmazeutische Zusammensetzung, die im Wesentlichen aus einem Genabgabesystem besteht, das den Vektor irgendeines der Ansprüche 1 bis 4 in einem Verdünnungsmittel umfasst.

7. Pharmazeutische Zusammensetzung, die im Wesentlichen aus einem Genabgabesystem besteht, das den Vektor irgendeines der Ansprüche 1 bis 4 eingebettet in einer Matrix für langsame Freisetzung umfasst.

8. Pharmazeutische Zusammensetzung nach irgendeinem der Ansprüche 6 und 7, wobei das Genabgabesystem ferner Helfervirusse, Proteine und/oder Lipide umfasst.

9. Pharmazeutische Zusammensetzung, die eine oder mehrere rekombinante Zellen umfasst, die den Vektor der Ansprüche irgendeines der Ansprüche 1 bis 4 umfasst.

10. Vektor von Ansprüchen 1-4 zur Verwendung bei der Behandlung von Retinitis pigmentosa, die durch Mutationen in PRPF31 im Auge einer menschlichen Patientenperson verursacht wurde.

11. Vektor von Ansprüchen 1-4 zur Verwendung beim Steigern der Expression von of PRPF31 im Auge einer menschlichen Patientenperson.

12. Pharmazeutische Zusammensetzung nach irgendeinem der Ansprüche 5 bis 9 zur Verwendung bei der Behandlung von Retinitis pigmentosa, die durch Mutationen in PRPF31 im Auge einer menschlichen Patientenperson verursacht wurde.

13. Pharmazeutische Zusammensetzung nach irgendeinem von Ansprüchen 5 bis 9 zur Verwendung beim Steigern der Expression von of PRPF31 im Auge einer menschlichen Patientenperson.

## Revendications

1. Vecteur du virus adéno-associé de type 2 (AAV2) comprenant une séquence codant le PRPF31 humain, lié de manière fonctionnelle à un promoteur qui entraîne l'expression dans des cellules d'épithélium pigmentaire rétinien (EPR), dans lequel la séquence du PRPF31 est ou comprend, ou code la même protéine que nts 1319-2818 de SEQ ID NO:34.

2. Vecteur selon la revendication 1, dans lequel le promoteur est un promoteur CAG, CASI, RPE65 ou VMD2.

3. Vecteur selon la revendication 1, dans lequel la séquence du PRPF31 est à codon optimisé.

4. Vecteur selon l'une quelconque des revendications précédentes, comprenant en outre un polypeptide de capside d'AAV ayant une séquence d'acides aminés sélectionnée dans le groupe constitué par SEQ ID NO: 1, 3, 5, 7, 9, 11, 13, 15, 19, 20, 21, 22, 23, 24, 25 et 26.

5. Composition pharmaceutique comprenant le vecteur selon l'une quelconque des revendications 1-4, formulée pour administration via injection sous-rétinienne.

6. Composition pharmaceutique constituée essentiellement d'un système d'administration de gène comprenant le vecteur selon l'une quelconque des revendications 1 à 4 dans un diluant.

7. Composition pharmaceutique constituée essentiellement d'un système d'administration de gène comprenant le vecteur selon l'une quelconque des revendications 1 à 4, intégrée dans une matrice à libération progressive.

8. Composition pharmaceutique selon l'une quelconque des revendications 6 et 7, dans laquelle le système d'administration de gènes comprend en outre des virus, des protéines, et/ou des lipides auxiliaires.

9. Composition pharmaceutique comprenant une ou plusieurs cellules recombinantes comprenant le vecteur selon l'une quelconque des revendications 1 à 4.

10. Vecteur selon les revendications 1-4, destiné à être utilisé dans le traitement de la rétinite pigmentaire causée par des mutations du PRPF31 dans l'œil d'un sujet humain.

11. Vecteur selon les revendications 1-4, destiné à être utilisé dans l'augmentation de l'expression de PRPF31 dans l'œil d'un sujet humain.

12. Composition pharmaceutique selon l'une quelconque des revendications 5 à 9, destinée à être utilisée dans le traitement de la rétinite pigmentaire causée par des mutations du PRPF31 dans l'œil d'un sujet humain.

13. Composition pharmaceutique selon l'une quelconque des revendications 5 à 9, destinée à être utilisée dans l'augmentation de l'expression du PRPF31 dans l'œil d'un sujet humain.
